# EUROPEAN PATENT SPECIFICATION

(11) **EP 0 050 334 B1**
(45) Date of publication and mention of the grant of the patent: **10.06.1987**
(21) Application number: 81108420.1
(22) Date of filing: 16.10.1981
(51) Int. Cl.: C07D 487/04, C07D 519/00, A61K 31/40, C07C 123/00, C07C 149/233

(54) **2-Carbamimidoyl-6-substituted-1-carbadethiapen-2-em-3-carboxylic acids, a process for preparing and an antibiotic composition comprising the same**
2-Carbamimidoyl-6-substituierte-1-carbadethiapen-2-em-3-carbonsäure, Verfahren zur Herstellung und sie enthaltendes antibiotisches Arzneipräparat
Acides 2-carbamimidoyl-6-substitués-1-carbadethiapen-2-ème-3-carboxyliques, procédé de préparation et une composition antibiotique les contenant

(30) Priority: 17.10.1980 US 197856
(43) Date of publication of application: 28.04.1982
(73) Proprietor: Merck & Co., Inc., Rahway New Jersey 07065-0900 (US)
(72) Inventor: Christensen, Burton G., Scotch Plains, NJ 07076 (US); Johnston, David B.R., Warren, NJ 07060 (US); Schmitt, Susan M., Scotch Plains, NJ 07076 (US)
(74) Representative: Abitz, Walter, Dr.-Ing.

## Description

### BACKGROUND OF THE INVENTION

This invention relates to 2- and 6-substituted-1-carbadethiapen-2-em-3-carboxylic acids (I) and the pharmaceutically acceptable salt, ester and amide derivatives thereof which are useful as antibiotics: 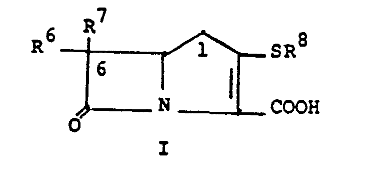 and the pharmaceutically acceptable salt, ester and amide derivatives thereof known in the β-lactam antibiotic art; wherein:
R⁶ and R⁷, are independently selected from the group consisting of hydrogen; substituted and unsubstituted: alkyl, alkenyl, and alkynyl having 1-10 carbon atoms; cycloalkyl, cycloalkylalkyl, and alkylcycloalkyl having 3-6 carbon atoms in the cycloalkyl ring and 1-6 carbon atoms in the alkyl moiety; aryl, aralkyl, aralkenyl, and aralkynyl wherein the aryl moiety is phenyl and aliphatic portion has 1-6 carbon atoms; R⁶ and R⁷ may be joined to form a cyclicalkyl having, together with the carbon atom to which they are attached, 3-6 carbon atoms; wherein the substituent or substituents on R⁶, and R⁷ are independently selected from chloro, fluoro, hydroxy, bromo, carboxyl, cyano, azido, amino, mono- and dialkylamino (each alkyl having 1-6 carbon atoms), ureido, alkylthio having 1-6 carbon atoms, and alkoxyl having 1-6 carbon atoms; and R⁸ is a carbamimidoyl selected from the group consisting of: 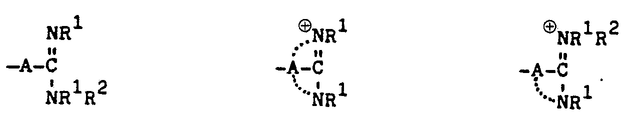 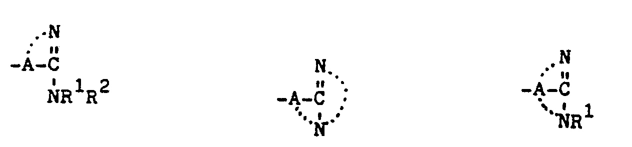 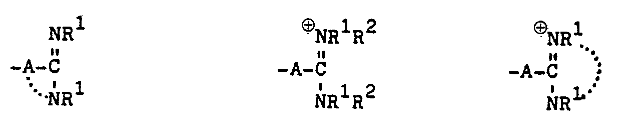 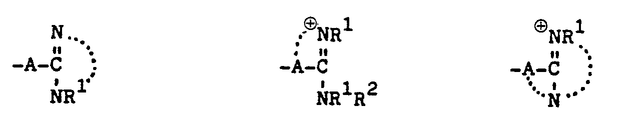 wherein:
A is a single, direct bond with the exception that A is not a single, direct bond with such compounds wherein the group -C=N(NR^{I}R²) is not quaternary, or is a divalent cyclic or acyclic connector selected from the group consisting of alkylene, alkenylene, and alkynylene having 1-10 carbon atoms which may be interrupted by a hetero atom selected from 0, S or N, or by phenylene, cycloalkylene, cycloalkenylene having 3―6 carbon atoms, heterocyclylene or heteroarylene wherein such cyclic interruptions comprise 3―6 ring atoms selected from C, O, S and N; cycloalkylene, cycloalkenylene having 3-6 carbon atoms; heterocyclylene or hetereoarylene each having 5-10 ring atoms and one or more hetero atoms selected from 0, N and S; and phenylene; R' and R are independently selected from hydrogen and the previously defined, but monovalent, values for the group A; and wherein the dotted lines indicate provision for cyclic structures formed by the joinder of the indicated nitrogen atom and the connector group A and by the joinder of the indicated nitrogen atoms, with the exception of compounds wherein 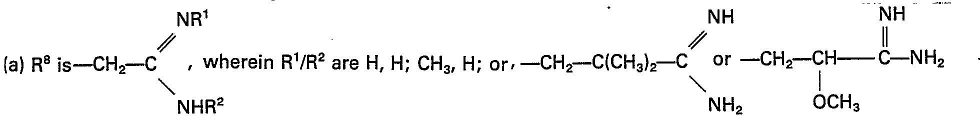 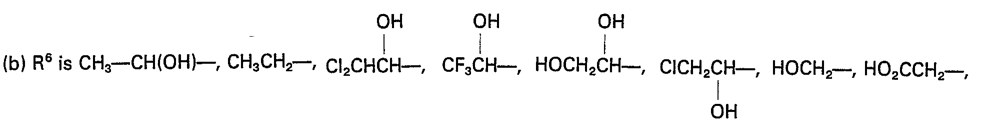 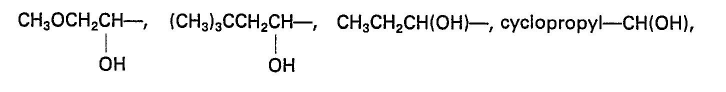 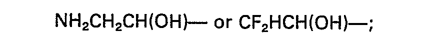
R⁷ is H and 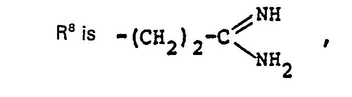 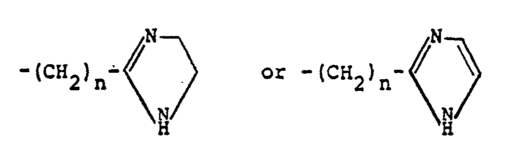 wherein n is 1 or 2 and
(c) R⁶ is (CH₃)₂CH―CH(OH)―
R⁷ is Hand

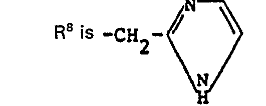

It should be noted that the final products of this inventin (I) can exist in either neutral or zwitterionic (internal salt) forms. In the zwitterionic form, the basic function is protonated and positively charged and the carboxyl group is deprotonated and negatively charged. The zwitterionic form is the predominant species under most conditions and is in equilibrium with a minor amount of the uncharged, neutral species. The equilibrium process is conveniently visualized as an internal acid-base neutralization. The neutral and zwitterionic forms are shown below. 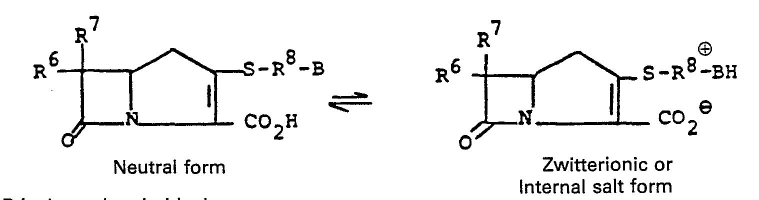 wherein B is the carbamimidoyl group.

Further, the final products of this invention I wherein R⁸ contains a positively charged quaternary nitrogen function such as the "carbamimidinium" can exist as zwitterionic (internal salt) forms or as external salt forms. The preferred form of this product group is the zwitterionic or internal salt form. These forms are shown below: 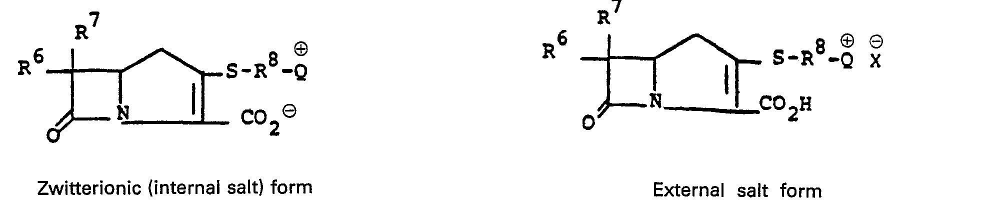 wherein Q represents the quaternized nitrogen group, and wherein X is a pharmaceutically acceptable anion such as those listed in U.S. Patent 4,194,047, issued 3/18/80, which is incorporated herein by reference. This invention also relates to the carboxyl derivatives of I which are antibiotics and which may be represented by the following generic structure (I): 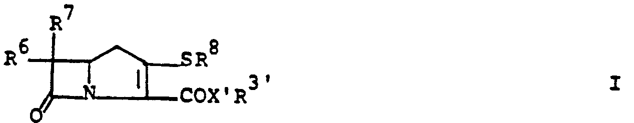 wherein X' is oxygen, NR' (R' = H or lower alkyl having 1-6 carbon atoms); and R³' is hydrogen, or, inter alia is representatively selected to provide the pharmaceutically acceptable salt, ester, and amide moieties known in bicyclic β-lactam antibiotic art; R^{3'} may also be a readily removable blocking group. The definition of R^{3'} is given in greater detail below.

This invention also relates to processes for the preparation of such compounds I and pharmaceutical compositions comprising such compounds.

There is a continuing need for new antibiotics. For unfortunately, there is no static effectiveness of any given antibiotic because continued wide scale usage selectively gives rise to resistant strains of pathogens. In addition, the known antibiotics suffer from the disadvantage of being effective only against certain types of microorganisms. Accordingly, the search for new antibiotics continues.

Thus, it is an object of the present invention to provide a novel class of antibiotics which are useful in animal and human therapy and in inaminate systems. These antibiotics are active against a broad range of pathogens which representatively include both Gram positive bacteria such as S. aureus, Strep. pyogenes, and B. subtilis, and Gram negative bacteria such as E. coli, Pseudomonas, Proteus morganii, Serratia, and Klebsiella. Further objects of this invention are to provide chemical processes for the preparation of such antibiotics and their nontoxic, pharmaceutically acceptable salts; and pharmaceutical compositions comprising such antibiotics.

### DETAILED DESCRIPTION OF THE INVENTION

The compounds of the present invention (I, above) are conveniently prepared by the following scheme: 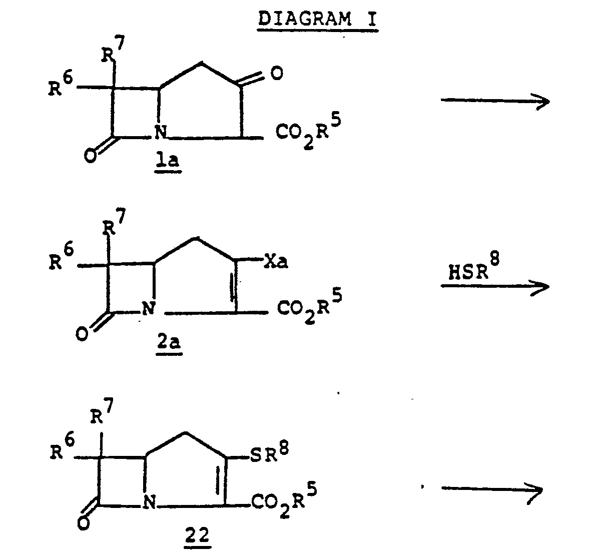 (wherein X" is a leaving group and R⁵ a protecting group)

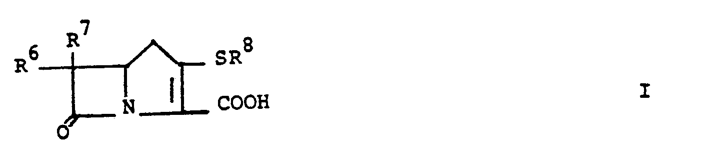

In words relative to the above reaction scheme, Diagram I, the step la to 2a to establish leaving group X^{a} is accomplished by acylating the bicyclic keto ester 1a with an acylating agent RX^{a} such as p-toluenesulfonic acid anhydride, p-nitrophenylsulfonic acid anhydride, 2,4,6-triisopropylphenylsulfonic acid anhydride, methanesulfonic acid anhydride, trifluoromethane sulfonic acid anhydride, diphenyl chlorophosphate, toluenesulfonyl chloride, p-bromophenylsulfonyl chloride, or the like; wherein X^{a} is the corresponding leaving group such as toluene sulfonyloxy, p-nitrophenylsulfonyloxy, benzenesulfonyloxy, diphenylphosphoryl, and other leaving groups which are established by conventional procedures and are well known in the art. Typically, the above acylation to establish leaving group X^{a} is conducted in a solvent such as methylene chloride, acetonitrile or dimethylformamide, in the presence of a base such as diisopropylethylamine, triethylamine, 4-dimethylaminopyridine or the like at a temperature of from -20 to 40° for from 0.1 to 5 hours. The leaving group X^{a} of intermediate 2a can also be halogen. The halogen leaving group is established by treating 1a with a halogenating agent such as φ₃PCl₂, φ₃PBr₂, (φO)₃PBr₂, oxalyl chloride or the like in a solvent such as CH₂CI₂, CH₃CN, THF, or the like in the presence of a base such as diisopropylethylamine, triethylamine, or 4-dimethylaminopyridine or the like. [φ = phenyl.]

The reaction 2a to 22 is accomplished by treating 2a in a solvent such as dioxane, dimethylformamide, dimethylsulfoxide, acetonitrile, hexamethylphosphoramide, or the like, in the presence of an approximately equivalent to excess of the mercaptan reagent HSR^{B}, wherein R^{B} is defined above, in the presence of a base such as sodium hydrogen carbonate, potassium carbonate, triethylamine, diisopropylethylamine, or the like at a temperature of from -40 to 25°C for from 3 sec. to 1 hour.

The final deblocking step 22 to / is accomplished by conventional procedures such as solvolysis or hydrogenation. The conditions of deblocking 22 to / are thus: typically 22 in a solvent such as tetrahydrofuran-water, tetrahydrofuran-ethanol-water, dioxane-water, dioxane-ethanol-water, n-butanol- water, or the like containing pH 7 morpholinopropanesulfonic acid-sodium hydroxide buffer, pH 7 phosphate buffer, dipotassium hydrogen phosphate, sodium bicarbonate, or the like, is treated under a hydrogen pressure of from 1 to 4 atmospheres in the presence of a catalyst such as platinum oxide, palladium on charcoal, or palladium hydroxide on charcoal, or the like, at a temperature of from 0° to 50°C for from 0.25 to 4 hours to provide I. Photolysis, when R⁵ is a group such as o-nitrobenzyl, for example, may also be used for deblocking.

Relative to Diagram I, the bicyclic keto ester la may be obtained by a variety of schemes of total synthesis. One of these schemes wherein R⁷ is hydrogen and R⁶ is hydroxyethyl (1a): 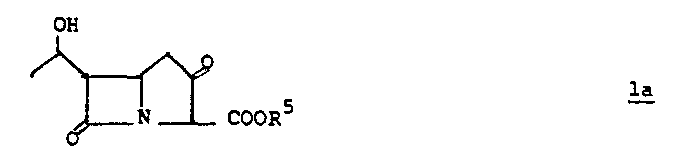 is disclosed in European Patent Application Number 79101307.1 filed May 1, 1979, publication number 0007973 (February 20,1980). This application is incorporated herein by reference, particularly to the extent that it describes the preparation of this embodiment of starting material la and its activation to 2a, in the context of the reactive scheme: 1a→2a→22→I.

The bicyclic keto ester la, in the general case, may be prepared by the processes disclosed and claimed in the three following, co-pending, commonly assigned U.S. Patent Applications of Christensen, Ratcliffe and Salzmann. To the extent that these applications disclosed processes for the preparation of la, in its general expression, 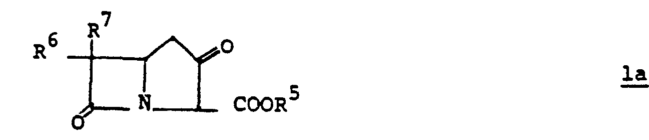 and the process scheme for the preparation of antibiotics I of the present invention according to the reactive scheme 1a→2a→22→l, they are hereby incorporated by reference. The three applications are:
1) Process for the Preparation of 1-Carbapenems and Intermediates via 4-Allylazetidinone; European Patent Application 81102268.0 filed March 26, 1981 (EP-A-37080).
2) Process for the Preparation of 1-Carbapenems and Intermediates via Trithioorthoacetates; European Patent Application 81102269.8 filed March 26, 1981 (EP-A-37081).
3) Process for the Preparation of 1-Carbapenems and Intermediates via Silyl-Substituted Dithioacetals; 1980; European Patent Application 81102270.6 filed March 26, 1981 (EP-A-37082).

### HSR^{B} Reagents

Relative to the foregoing description of the invention, suitable carbamimidolyl and carbamimidinium mercaptans HSR^{B} which are utilized in the transformation 2a to 22 are listed below.

For example R¹ and R² may be derived from thiophene, imidazole, tetrazolyl, furyl and pyridine. When located on the same nitrogen atom, the substituents R¹ and R² can be joined to form a cyclic group comprising 3-8 atoms. The resulting ring can contain additional 0-, S- or N-atoms. For example:

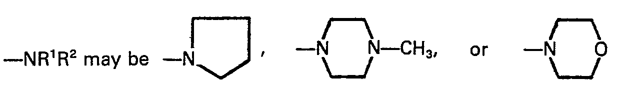

For example A may be derived from thiophene, imidazole, pyridine, furyl and the like;
Representative examples of preferred ―SR⁸ groups (represented as HSR⁸) are:

### EXAMPLES

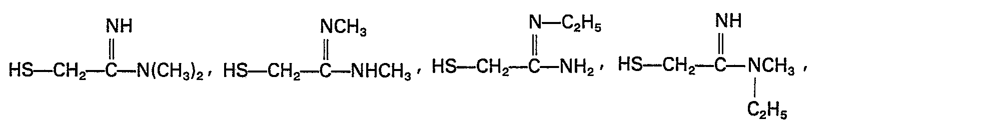

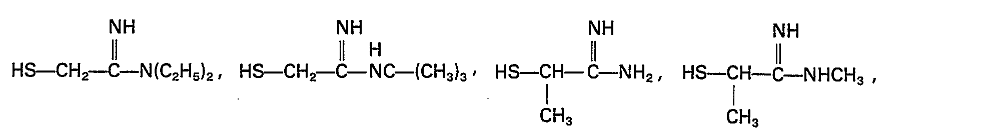

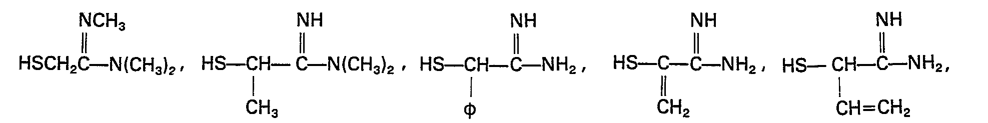

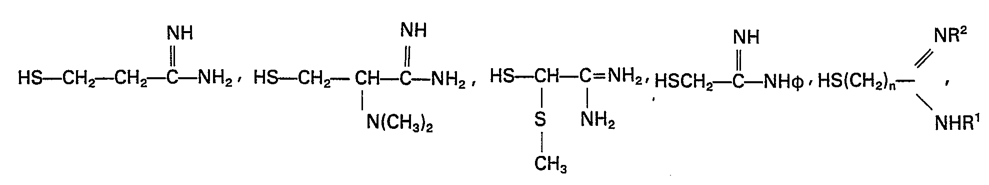

n = 2-5, R² = H, CH₃, R¹ = H, CH₃ 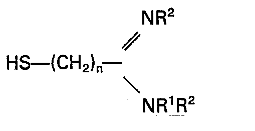 n = 2―5, R¹, R² = H, CH₃

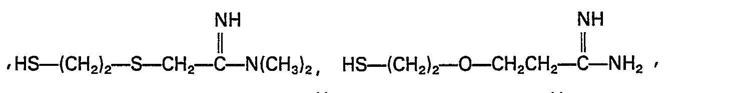

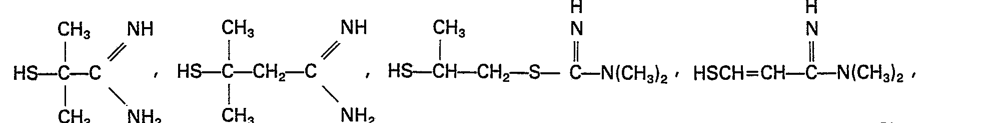

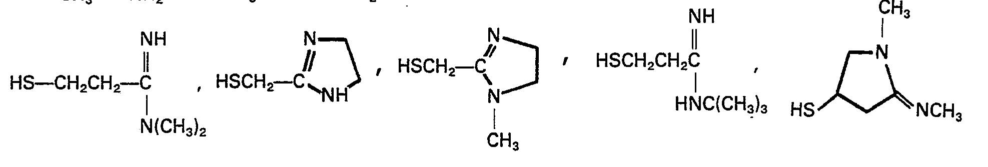

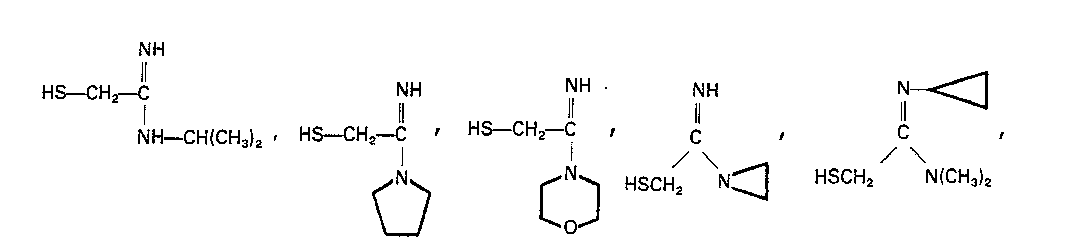

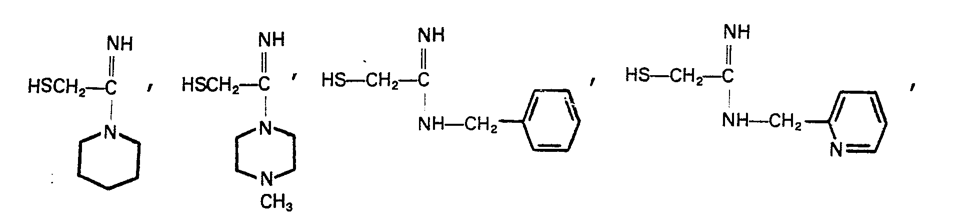

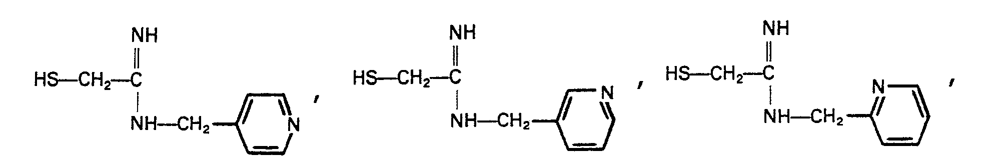

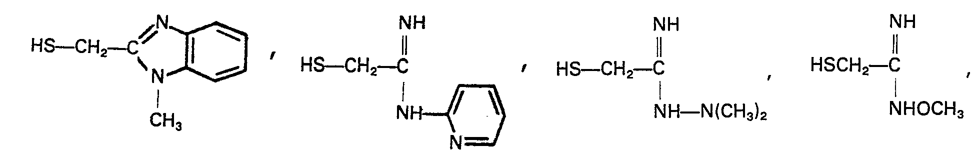

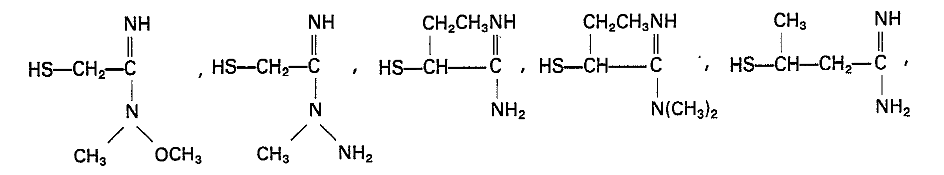

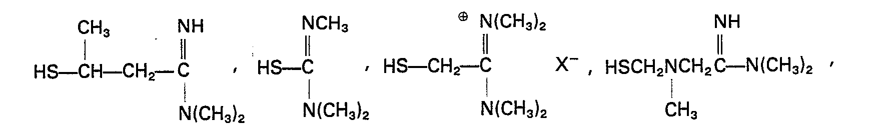

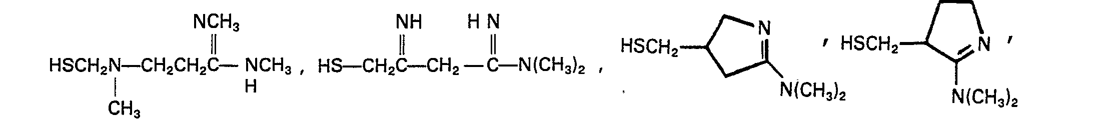

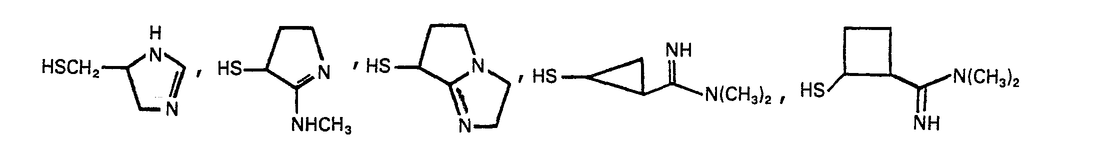

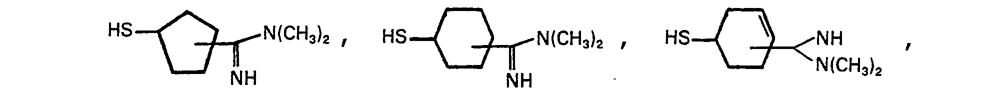

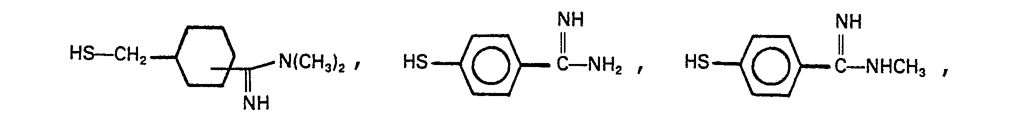

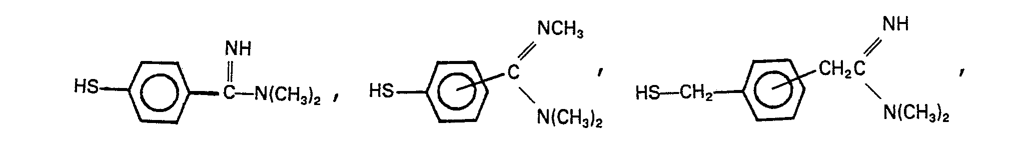

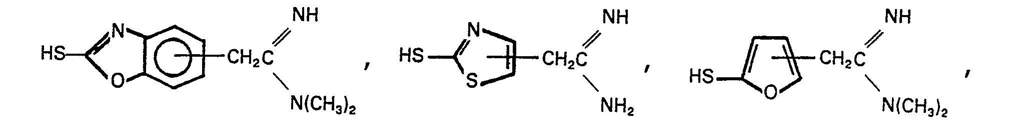

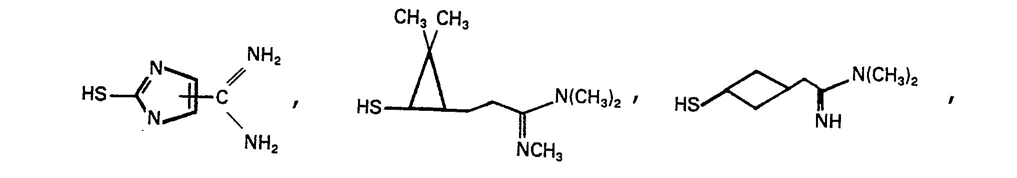

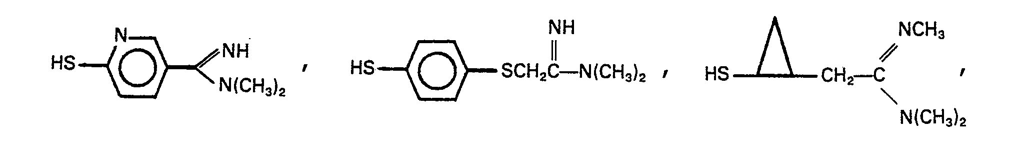

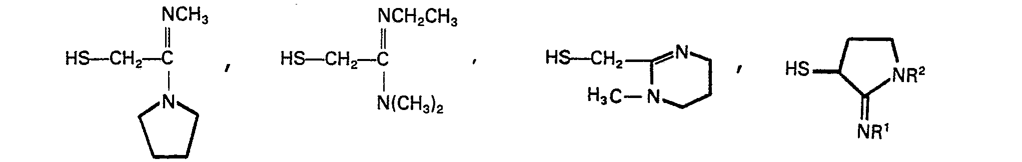

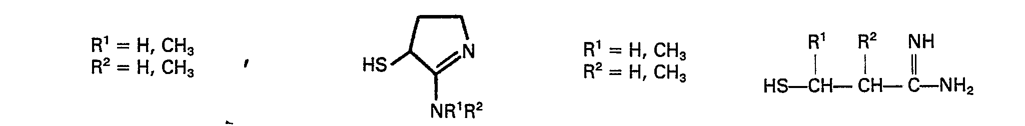

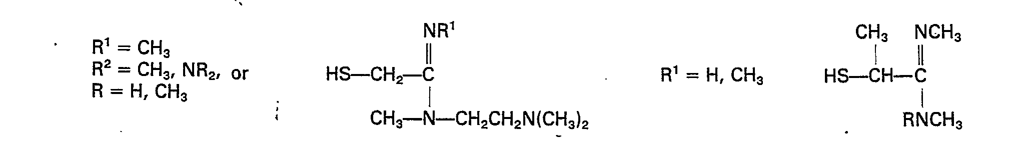

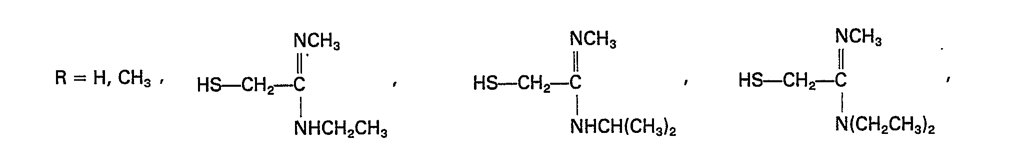

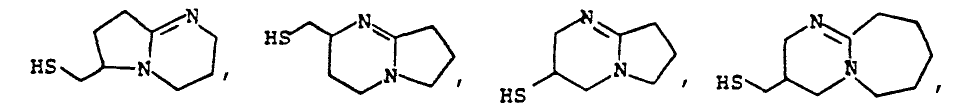

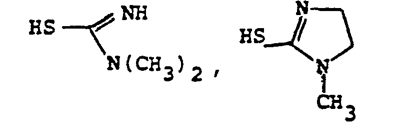

Relative to the foregoing, representative list of suitable reagents HSR^{B}, it should be noted that when the mercaptan contains a functional group which might interfere with the intended course of reaction, the offending group is covered. For example, when a basic nitrogen group is encountered (-NHR or-NH₂, for example) it is usually protected by acylation (e.g., -C0₂PNB) and when a carboxyl group (-C0₂H) is present, it is usually protected by esterification (e.g., PNB ester). Such protection also facilitates in the purification of products by chromatographic means. (PNB is p-nitrobenzyl). Such protection is, however, not a necessary requirement for introduction of the ―SR⁸ side chain. The transformation 2a→22 (Diagram I) is conveniently carried out using both protected and unprotected HSR⁸ forms. It is recognized that SR^{B} side chains in which the R⁸ group contains one or more chiral centers can be added as racemic or diastereomeric mixtures to provide mixtures of diastereomeric products or can be added as resolved, isomerically pure reagents to provide diastereomerically pure products. Since antibacterial activity and other pharmacological properties vary among isomers, it is frequently advantageous to prepare isomerically pure products by the introduction of resolved ―SR⁸ side chains.

### Alkylating and acylating reagents for establishing R⁶ and R⁷ and p'referred values for R⁶ and R⁷

As shown above, the compounds of the present invention (I) are conveniently prepared from starting material 1a, Diagram I. The following section describes the establishment of R⁶ and R⁷ at the azetidinone level in the preparation of 1a. Also described is the establishment of R⁶ and R⁷ upon other azetidinones which are also useful in the synthesis of compounds of the present invention. In this regard, see European Patent Application Serial Number 80102076.9 filed April 18, 1980, (EP-A-17992) which is fully incorporated herein by reference, particularly to the extent that it describes the azetidinone starting materials which are summarized below. 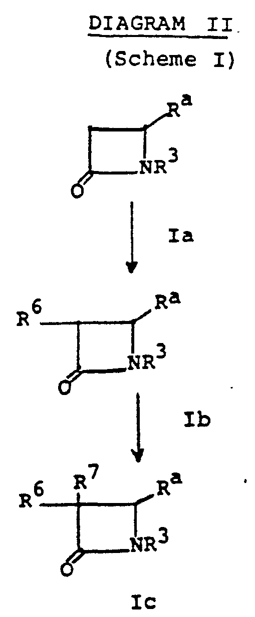 wherein:
1) R^{a} is CH₂CH₂0R²; R³ and R² are as defined in European Patent Application Serial Number 80102076.9 (EP-A-17992);
2) R^{a} is CH=CH₂; see European Patent Application Serial Number 80102076.9 (EP-A-17992);
3) R^{a} is CH₂CH=CH₂, see previously incorporated by reference European Patent Application 81102268.0 filed March 26, 1981 (EP-A-37080);
4) R^{a} is CH₂C(SR°)₃, R° values are selected from alkyl, aryl, and aralkyl; see previously incorporated by reference European Patent Application 81102269.8 filed March 26, 1981 (EP-A-37081); and
5) R^{a} is CH₂C(SR°)₂SiR°₃, R° values are selected from alkyl, aryl and aralkyl; see previously incorporated by reference European Patent Application 81102270.6 filed March 26, 1981 (EP-A-37082).

In words relative to the above reaction diagram, and as described above, starting material la can be mono- or dialkylated at ring position 3. Alkylation of la provides Ic. Typically, la is treated with a strong base such as lithium diisopropylamide, lithium, 2,2,6,6-tetramethylpiperidide, potassium hydride, lithium hexamethyldisilazane, phenyllithium or the like in a solvent such as tetrahydrofuran (THF), hexamethylphosphoramide, ether, dimethoxyethane, and the like at a temperature of from -80°C to 0°C whereupon the alkylating agent of choice, R⁶X° is added (X° is chloro, iodo or bromo); alternatively the alkylating agent may be R⁶⁻tosylate, R⁶⁻mesylate or an aldehyde or ketone such as acetaldehyde to provide monoalkylated species lb. When desired, dialkylated species Ic may be obtained from Ib by repeating the alkylating procedures la→lb.

The eventual 6-substituents (nomenclature relative to final, bicyclic structure) can also be established by direct acylation using an acylating agent such as N-acyl imidazole or the like. Such N-acyl imidazole acylating reagents are listed below. Also given below is a detailed description of this second approach for establishing, R⁶ and R⁷.

The following list is representative of useful alkylating agents for establishing R⁶ and R⁷, according to the above scheme la→lb→lc (this will be referred to as Scheme I, to the distinguished from Scheme II, below, which involves acylation):

### Alkylating Agents

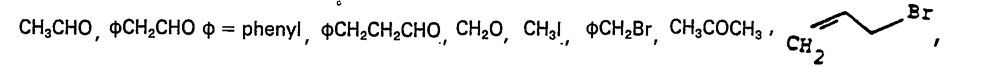

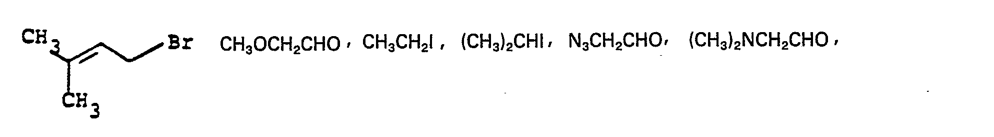

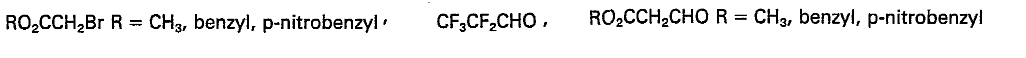

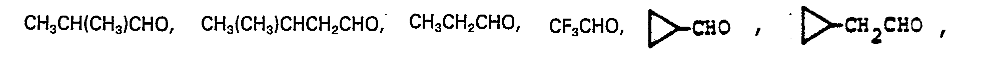

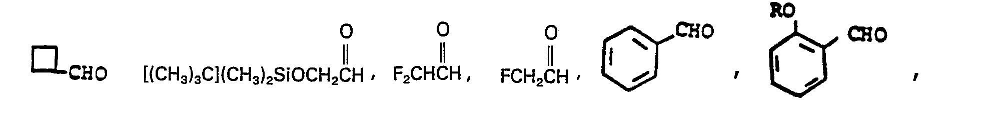

R = protecting group 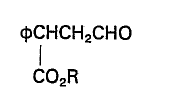
R is removable carboxyl protecting group, such as benzyl.

As mentioned above, the 6-substituents may also be established by acylation. Utilization of such acylating agents may be demonstrated in the following manner with regard to a preferred starting material Ib or lc: 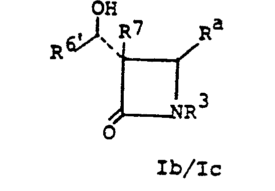 wherein R⁷, R^{a}, and R³ are as defined above. R⁶' is defined relative to the definition of R⁶ and in that sense is the balance of the previously identified group R⁶. In other words, for purposes of this definition R⁶'CH(OH)―=R⁶. An especially preferred material Ib is when R⁷ is hydrogen and R⁶' is methyl. Basically, such 1'-hydroxy R⁶' species Ib are prepared according to the following scheme:

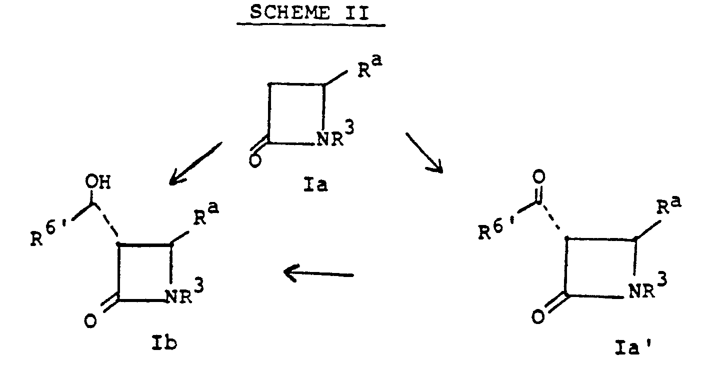

The alkylation la→lb, Scheme II, is accomplished as previously described, by treating la in a solvent such as tetrahydrofuran, dimethoxyethane, diethylether, hexamethylphosphoramide, at a temperature of from -100° to -20°C with a strong base such as lithium diisopropylamide, lithium hexamethyldisilazide, lithium 2,2,6,6-tetramethylpiperidide, potassium hydride or the like followed by the addition of an equivalent to 10 fold excess of an aldehyde. This reaction gives a mixture of isomers from which the desired trans-R form Ib can be conveniently separated by chromatography or crystallization.

Intermediate la may proceed directly to Ib as indicated above, or it may take the circuitous path via la'. The direct acylation, to la' is accomplished by treating la with two or more equivalents of a base such as lithium diisopropylamide, lithium hexamethyldisilazide, lithium 2,2,6,6-tetramethylpiperidide, in a solvent such as tetrahydrofuran, diethylether, or dimethoxyethane, for example, at a temperature of from -100 to -20°C with an acylating agent such as N-acyl imidazole or the like. Addition of the la plus base mixture to the acylating agent is preferred.

Representative acylating agents for this scheme la→la'→lb are listed below.

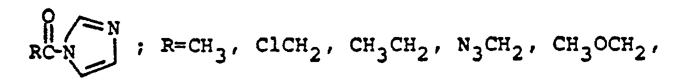

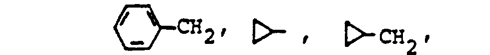

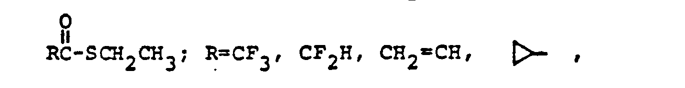

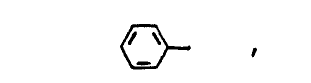

Further with respect to Scheme II, the reduction, la'→lb is accomplished by contacting the ketone with a reducing agent such as potassium tri(sec-butyl)borohydride, lithium tri(sec-butyl)borohydride, sodium borohydride, sodium tris(methoxyethoxy)aluminum hydride, lithium aluminum hydride or the like in a solvent such as diethylether, tetrahydrofuran, toluene, i-propanol or the like at a temperature of from -78° to 25°C. The reaction can conveniently be conducted in the presence of an added complexing salt such as potassium iodide, magnesium bromide or the like.

In a similar manner, unresolved Ib (cis and trans) may be oxidized to la' for reduction to Ib as indicated above:

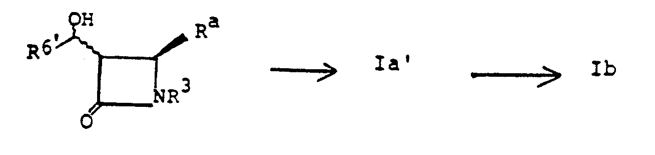

The oxidation is accomplished with an oxidizing agent such as dipyridine chromium (VI) oxide, trifluoroacetic anhydride-dimethylsulfoxide-triethylamine, pyridinium dichromate, acetic anhydride-dimethylsulfoxide in a solvent such as methylene chloride, acetonitrile, or the like at a temperature of from -78 to 25°C for from 5 minutes to 5 hours.

Finally, relative to 6-substituents R⁶ and R⁷, it is to be emphasized that the most preferred class of compounds I is that wherein R⁷ is hydrogen and R⁶ is―CH(OH)CH₃, and wherein the absolute configuration is 5R, 6S, 6(R 1-hydroxyethyl) about carbon atoms 5 and 6:

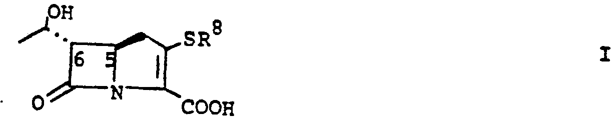

### Other preferred values for R⁶ (R⁷ = H) are: FCH₂CH(OH)-, CH₃CH₂CH(OH)-, CH₃CH₂-, and (CH₃)₂C(OH)―.

As noted above, the compounds of the present invention may also generally be represented by the following structural formula: 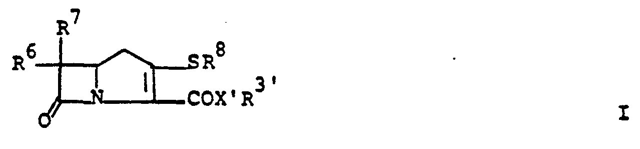 wherein X' is oxygen, NR' (R' is hydrogen or loweralkyl having from 1 to 6 carbon atoms); and R^{3'} is hydrogen, or, inter alia, is representatively selected to provide the pharmaceutically acceptable salt, ester, and amide moieties known in the bicyclic β-lactam antibiotic art; R^{3'} may also be a readily removable blocking group.

### Identification of the Radical -COX'R³'

In the generic representation of the compounds of the present invention (I, above), the radical represented by―COX'R^{3'} is, interalia,-COOH (X' is oxygen and R³' is hydrogen) and all radicals known to be effective as pharmaceutically acceptable ester, and amide radicals in the bicyclic β-lactam antibiotic art, such as the cephalosporins and penicillins and nuclear analogues thereof.

Suitable blocking esters (R^{3'}, X = O) include those selected from the following list which is representative:
(i) R^{3'} = CR^{a}R^{b}R^{c} wherein at least one of R^{a}R^{b} and R° is an electron-donor, e.g., p-methoxyphenyl. The remaining R^{a}, R^{b} and R^{c} groups may be hydrogen or organic substituting groups. Suitable ester groups of this type include p-methoxybenzyloxycarbonyl.
(ii) R³' = CR^{a}R^{b}R^{c} wherein at least one of R^{a}, R^{b} and R° is an electron-attracting group, e.g., p-nitrophenyl, trichloromethyl, and o-nitrophenyl. Suitable esters of this type include p-nitrobenzyloxycarbonyl, and 2,2,2-trichloroethoxycarbonyl.
(iii) R^{3'} = CR^{a}R^{b}R^{c} wherein at least two of R^{a}, R^{b} and R° are hydrocarbon such as alkyl, e.g., methyl or ethyl, or aryl, e.g., phenyl and the remaining R^{a}, R^{b} and R° group, if there is one, is hydrogen. Suitable esters of this type include t-butyloxycarbonyl, diphenylmethoxycarbonyl and triphenylmethoxycarbonyl.

Silyl esters, under this category of blocking groups, may conveniently be prepared from a halosilane of the formula: R⁴₃SiX' wherein X' is a halogen such as chloro or bromo and R⁴ is alkyl, e.g., methyl, ethyl, t-butyl.

Pharmaceutically acceptable carboxyl derivatives of the present invention are those derived by reacting I with alcohols and the like. For example, esters and amides of interest are the above-listed starting materials and final products having the -COX'R3' group at the 3-position; wherein X' is oxygen or NR' (R' is H or R³'), and R^{3'} is alkyl having 1-6 carbon atoms, straight or branched, such as methyl, ethyl, t-butyl, and the like; carbonylmethyl, including phenacyl; aminoalkyl including 2-methylaminoethyl, 2-diethylaminoethyl; alkanoyloxyalkyl wherein the alkanoyloxy portion is straight or branched and has 1-6 carbon atoms and the alkylportion has 1-6 carbon atoms, such as piveloyloxymethyl; haloalkyl wherein halo is chloro, and the alkyl portion is straight or branched having 1-6 carbon atoms, e.g., 2,2,2-trichloroethyl; alkenyl having 1-4 carbon atoms such, as 2-propenyl, 3-butenyl, and 4-butenyl; aralkyl and lower alkoxyl-and nitro-substituted aralkyl such as benzyl, benzhydryl, o-nitrobenzyl, p-methoxybenzyl, and p-nitrobenzyl; phthalidyl; benzyloxyalkyl having 8-10 carbon atoms such as benzyloxymethyl, and (4-nitro) benzyloxymethyl.

In addition to the esters listed above, amides are also embraced by the present invention, i.e., wherein X' is the 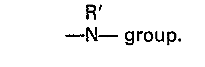 Representative of such amides are those wherein R' is selected from the group consisting of hydrogen and lower alkyl such as methyl and ethyl.

The most preferred -COX'R3' radicals of the present invention are those wherein (relative to Structure I above), X' is oxygen and R³' is hydrogen; loweralkyl having 1-4 carbon atoms; lower alkenyl such as 3-methylbutenyl, 4-butenyl and the like; benzyl and substituted benzyl such as p-nitrobenzyl; pivaloyloxymethyl, 3-phthalidyl; and phenacyl.

The compounds of the present invention (I) are valuable antibiotics active against various gram- positive and gram-negative bacteria and accordingly find utility in human and veterinary medicine. Representative pathogens which are sensitive to antibiotics I include: Staphylococcus aureus, Escherichia coli, Klebsiella pneumoniae, Bacillus subtilis, Salmonella typhosa Psuedomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy and inhibit the growth of harmful bacteria on medical and dental equipment and as bactericides in industrial applications, for example in waterbased paints and in the white water of paper mills to inhibit the growth of harmful bacteria.

The products of this invention may be used in any of a variety of pharmaceutical preparations. They may be employed in capsule, powder form, in liquid solution, or in suspension. They may be administered by a variety of means; those of principal interest include: orally, topically or parenterally by injection (intravenously or intramuscularly).

Such tablets and capsules, designed for oral administration, may be in unit dosage form, and may contain conventional excipients, such as binding agents, for example, syrup, acacia, gelatin, sorbitol, tragacanth, or polyvinylpyrrolidone; fillers, for example, lactose, sugar, cornstarch, calcium phosphate, sorbitol, or glycerine; lubricants, for example, magnesium stearate, talc, polyethylene glycol, silica; disintegrants, for example, potato starch, acceptable wetting agents such as sodium lauryl sulphate. The tablets may be coated according to methods well known in the art. Oral liquid preparations may be in the form of aqueous or oily suspensions, or solutions, or they may be presented as a dry product for reconstitution with water or other suitable vehicle before use. Such liquid preparations may contain conventional additive such as suspending agents, for example, sorbitol, methyl cellulose, glucose/sugar syrup, gelatin, hydroxyethylcellulose, or carboxymethyl cellulose. Suppositories will contain conventional suppository bases, such as cocoa butter or other glycerides.

Compositions for injection, the preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water.

The compositions may also be prepared in suitable forms for absorption through the mucous membranes of the nose and throat or bronchial tissues and may conveniently take the form of liquid sprays or inhalants, lozenges, or throat paints. For medication of the eyes or ears, the preparation may be presented in liquid or semi-solid form. Topical applications may be formulated in hydrophobic or hydrophilic bases as ointments, creams, lotions, paints, or powders.

The dosage to be administered depends to a large extent upon the condition and size of the subject being treated as well as the route and frequency of administration - the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg. of body weight of the subject in one or more treatments per day. A preferred daily dosage for adult humans lies in the range of from about 10 to 240 mg. of active ingredient per kg. of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1 % to 99% of active material, the preferred range being from about 10-60%. The composition will generally contain from about 15 mg. to about 1500 mg. of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution. For zwitterionic species described under Structure I, the pH of such solutions typically will correspond to the zwitterionic point; however, consideration of individual properties of solubility and stability may require such aqueous solutions to have a pH other than that of the zwitterionic point, for example in the range of 5.5 to 8.2.

### Incorporation by Reference

The following paragraphs summarize the previous incorporations.

The compounds of the present invention may also be prepared by the processes disclosed and claimed in the three (3) following, EPO Applications. To the extent that these applications define R⁶, R⁷, and R^{B} of Structure I and to the extent that they describe processes for the synthesis of I, they are hereby incorporated by reference. 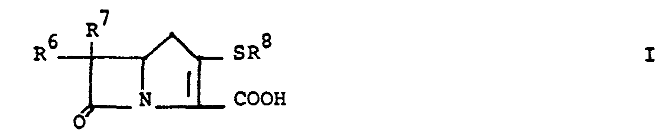
1) Process for the Preparation of 1-Carbapenems and Intermediates via 4-Allylazetidinone; EPO Application 81102268.0 filed 3-26-81 (EP-A-37 080).
2) Process for the Preparation of 1-Carbapenems and Intermediates via Trithioorthoacetates; EPO Application 81102269.8 filed 3-26-81 (EP-A-37 081).
3) Process for the Preparation of 1-Carbapenems and Intermediates via Silyl-Substituted Dithioacetals; EPO Application 81102270.6 filed 3-26-81 (EP-A-37 082).
4) European Patent Application Serial Number 80102076.9 filed April 18, 1980 (EP-A-17 992).

Also incorporated by reference is published European Patent Application 0007614 (Application Number 79102615.6, filed 24 July 1979). This application discloses certain dipeptidase inhibitors which, on co-administration to mammalian subjects, enhance the efficacy of certain 1-carbadethiapenem antibiotics. Thus, to the extent that the cited application: 1.) defines the manner by which susceptible carbadethiapenems substrates of the present invention may be identified; and 2.) discloses suitable inhibitors, compositions, and methods of treatment, it is incorporated herein by reference. A particularly preferred inhibitor is 6-(L-2-Amino-2-carboxyethylthio)-2-(2,2-DCC)-2-hexenoic acid.

The following examples recite a precise scheme of total synthesis. It is to be understood that the purpose of this recitation is to further illustrate the total synthesis and not to impose any limitation. Temperature is in °C.

### Example 1

This example and those depending from it demonstrate the so called "carbamimidoyl" embodiments of I. As defined above, such carbamimidoyl embodiments are characterized by the following generic representation: 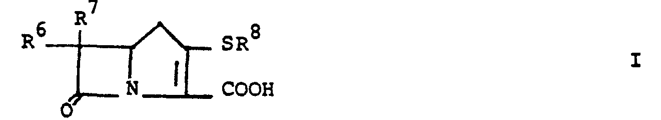 wherein:
R⁸, characterized by the carbamimidoyl group, is defined above.

The following diagram summarizes the foregoing text, and is illustrative of a preferred procedure for making the carbamimidoyl embodiments. In the following scheme, the synthesis shows a subgeneric objective, since it is representative of the entire carbamimidoyl genus. Other subgeneric expressions and species members of the carbamimidoyls are obtained by analogy: 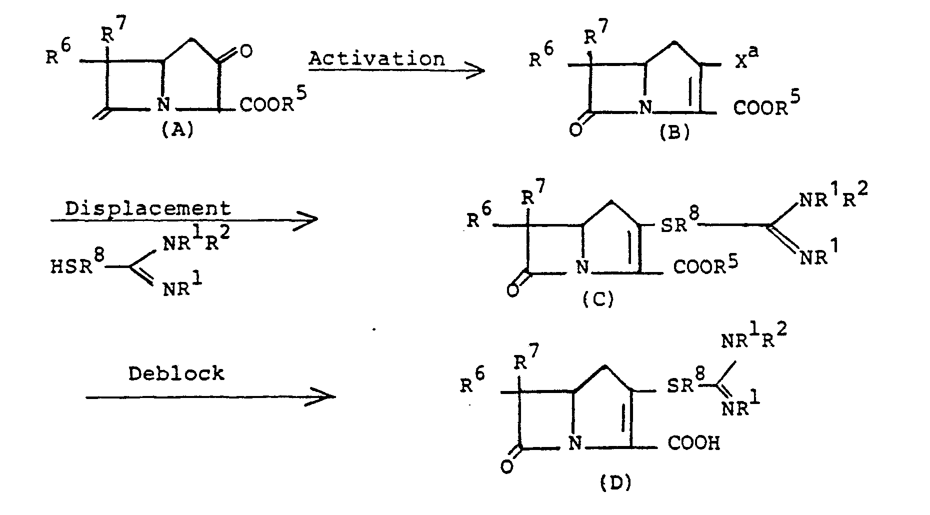 wherein: all symbols are as previously defined.

The activation step, A→B, which establishes the leaving group X" is accomplished by acylating the keto ester A with an acylating agent such as, for example, p-toluenesulfonic acid anhydride, trifluorometane- sulfonic acid anhydride, p-nitrophenylsulfonic acid anhydride, 2,4,6-triisopropylphenylsulfonic acid anhydride, methanesulfonic acid anhydride, diphenylchlorophosphate, toluenesulfonyl chloride, p-bromophenylsulfonyl chloride, or the like. The leaving group X^{a} thus is established as the corresponding p-toluenesulfonyloxy, p-nitrophenylsulfonyloxy, methanesulfonyloxy, p-bromophenylsulfonyloxy, diphenylphosphoryl, for example. Typically, the activation is carried out in a suitable organic solvent such as methylene chloride, chloroform, acetonitrile, dimethylformamide, dichloromethane, tetrahydrofuran, and the like, in the presence of a base such as diisopropylethylamine, triethylamine, 4-dimethylaminopyridine, pyridine, and the like at a temperature of from -20 to 40°C. The reaction usually is complete in from 0.5 to 5 hours.

The carboxyl protecting group R⁵ in the keto ester, A may be any of the well-known, readily removable carboxyl protecting groups such as, benzyl, p-nitrobenzyl, o-nitrobenzyl, methoxymethyl, allyl and the like.

The carbamimidoylthio side chain, B→C, is established by treating the activated keto ester, B, in a solvent such as dioxane, dimethylformamide, dimethylsulfoxide, acetonitrite, dichloromethane, tetrahydrofuran, hexamethylphosphoramide, and the like, in the presence of an approximately equivalent to excess quantity of the mercaptan reagent of choice in the presence of a base, such as diisopropylamine, triethylamine, pyridine, sodium hydrogen carbonate, potassium carbonate, and the like. The reaction is run at -40 to 50°C and usually is complete in 5 minutes to 10 hours. Such mercaptan reagents are either known or readily prepared by known techniques.

The mercaptan reagent may be employed in the form of the free base (as described above), a salt such as the hydrochloride, the hydrobromide, the sulfate and the toluenesulfonate, or a nitrogen atom may be protected with a conventional N-blocking group such as p-nitrobenzyloxycarbonyl. When the mercaptan reagent is in the free base form, the displacement reaction does not require the presence of additional base.

The final deblocking step, C-jD, preferably is carried out by catalytic hydrogenation. Catalysts suitable for the reaction include platinum metals, for example, platinum oxide, Pd/C, Pd(OH)₂, Pt/C, and the like which are employed with a hydrogen pressure of from 1 to 10 atmospheres at a temperature of from 0 to 25°C in the presence of a solvent such as tetrahydrofuran, dioxane, water, and the like. The reductive deblocking step is usually carried out in the presence of a buffer such as pH 7 phosphate buffer or pH 7 MOPS-NaOH buffer. The reaction usually is complete in from 0.5 to 12 hours.

The following Examples specifically demonstrate the process of Example 1.

### Example 2

(5R,6S)-2-(N,N-Dimethylcarbamimidoylmethyl-1-thio)-6-(1-R-hydroxyethyl)carbapen-2-em-3-carboxylic acid

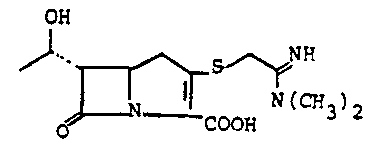

To a stirred solution of (5R,6S)-p-nitrobenzyl-6-[(R)-1-hydroxyethyl]-1-azabicyclo[3.2.0]heptan-3,7-dione-2-carboxylate (1.45 g) in acetonitrile (20 ml) in an ice-bath under nitrogen is added diphenylchlorophosphate (0.88 ml) and diisopropylethylamine (0.84 ml) dropwise during 5 minutes. After 30 minutes, diisopropylethylamine (0.7 ml) and a solution of N,N-dimethyl-mercaptoacetamidine hydrochloride (0.82 g) in dimethylsulfoxide (12 ml) are added dropwise during 15 minutes. The solution is poured into ether (0.6 L) and the resulting gum is immediately taken up in a mixture of tetrahydrofuran 120 ml, water 40 ml, and 0.1 M phosphate (pH 7, 140 ml) and hydrogenated for 2 hours at 50PSIG in the presence of 1.4 g. of 10% Pd/C catalyst. The catalyst is filtered and the filtrate is extracted with ether (250 ml). The aqueous layer is concentrated to 125 ml under vacuum and chromatographed on 600 ml of Dowex 50 x 2 (K⁺ cycle) resin, eluted with water. The fractions obtained between 1.1 L and 1.6 L of eluate are combined, concentrated and freeze dried. Yield 0.58 g (33%) U.V. Xmax.295 p, E% 205, 94% NH₂0H ext.

Following the above procedure, the following compound is obtained when the corresponding 9-fluoro starting material is substituted in equivalent amount:

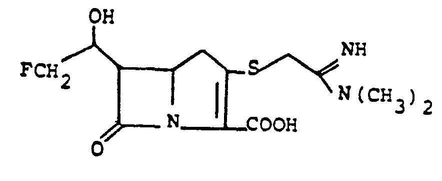

### Example 3

### N,N-Dimethyl-2-chloroacetamidine Hydrochloride

Sodium (23 mg) is dissolved in methanol (40 ml) under an atmosphere of nitrogen. To the solution is added chloroacetonitrile (6.32 ml) followed after 30 minutes by dimethylamine hydrochloride (8.16 g). After stirring an additional hour the solution is concentrated to a small volume. Ether is added and the product which crystallizes is filtered and washed with ether, yielding 14.5 g of N,N-dimethylchloroacetamidine hydrochloride. NMR, 60 MHZ, D₂0, 3.23 and 3.37N (CH₃)₂; 4.06 (S) CH₂Cl. This process is found in J. Med. Chem, 1979, Vol. 22, p 295; William A. Bolhofer, Charles W. Habeckes, Adolph M. Pretruszkiewicz.

### Example 4

### N,N-Dimethyl-2-mercaptoacetamidine Hydrochloride

A solution of N,N-dimethyl 2-chloroacetamidine hydrochloride (1.8 g), and trisodium phosphoro- thioate (2.2 g)⁻in 15 ml of water is stirred at room temperature for one hour. Hydrochloric acid (12 ml, 1N) is added and the solution is heated at 90° for 30 minutes under an atmosphere of nitrogen. The solution is concentrated to a slurry of crystals and isopropanol is added. The mixture is filtered and the filtrate is evaporated to an oil. The residue is triturated several times with ether and finally residual solvents are removed on a vacuum pump leaving a gummy residue. This is recrystallized from ethanol ether. m.p. 157―159°, 60 MHz, NMR, D₂O, δ 3.15 and 3.28 (CH₃)₂; 3.65 CH₂SH. See Bolhofer, et al., J. Med Chem.,1979, Vol. 22, p. 295.

### Example 5

Following the procedure in Examples 3 and 4, but using ethylamine hydrochloride there is obtained N-ethyl-2-mercaptoacetamidinium chloride 60 MHZ, NMR, D₂0, δ 1.3 (t) CH₂CH₃; 3.42 CH₂CH₃; 3.62, (S), CH₂SH. Substituting 2-chloropropionitrile in gives R,S-2-mercapto propionamidine hydrochloride, NMR, δ, 1.64, (d), CH₃; 3.93, (q) CH, and R,S-N-methyl-2-mercaptopropionamidine hydrochloride, NMR, 6, 1.61, d, CH₃; 3.0, NCH₃; 3.97, (q), CHCH₃.

### Example 6

Following the procedure of the foregoing Examples p-nitrobenzyl (5R, 6S)-3-diphenylphosphoryl-6-[(R)-1-hydroxyethyl]-1-azabicyclo[3.2.0]hept-2-en-7-one-2-carboxylate is reacted with the following mercapto carbamimidoyls to yield the corresponding carbamimidoyls I:

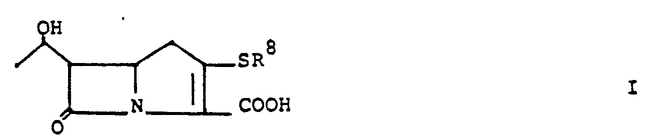

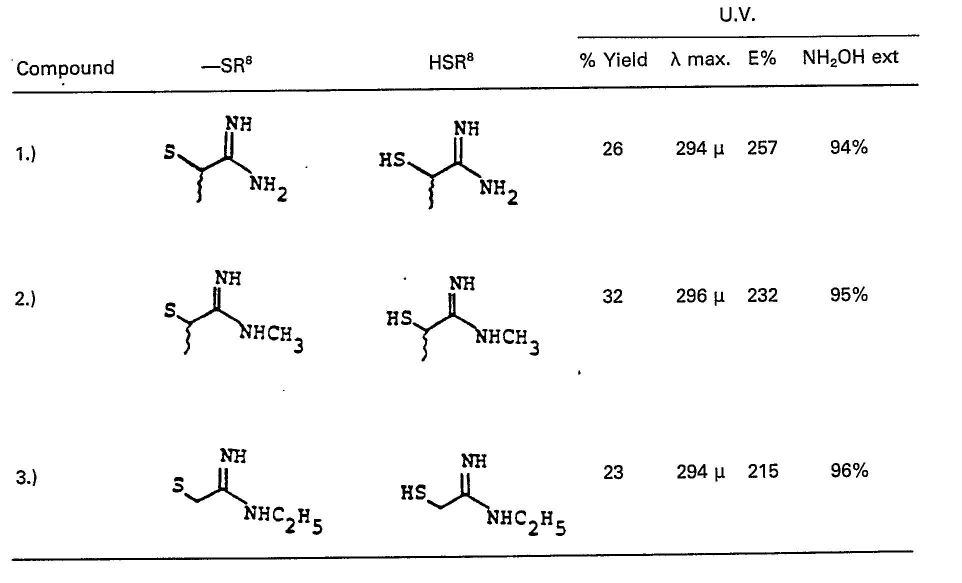

### Example 7

Following the procedure of Examples 1-6, the following compounds I are obtained when an equivalent amount of the appropriately substituted bicyclic keto ester (A) is taken:

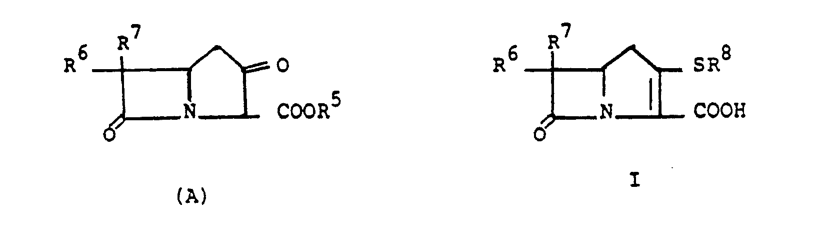

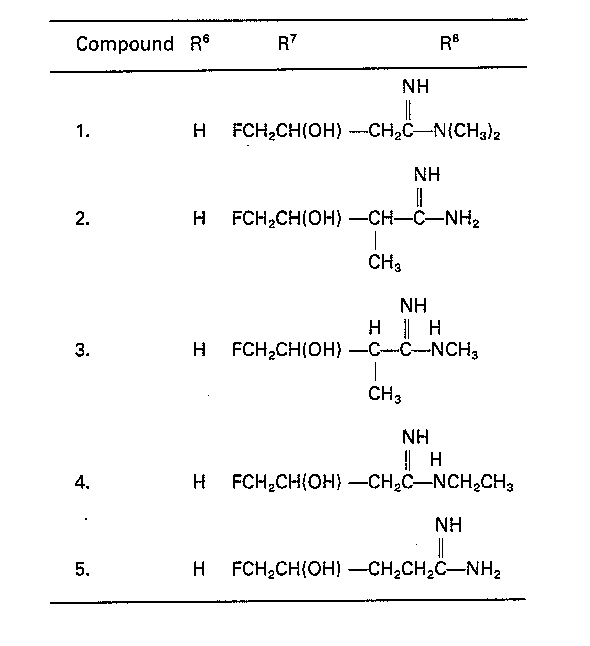

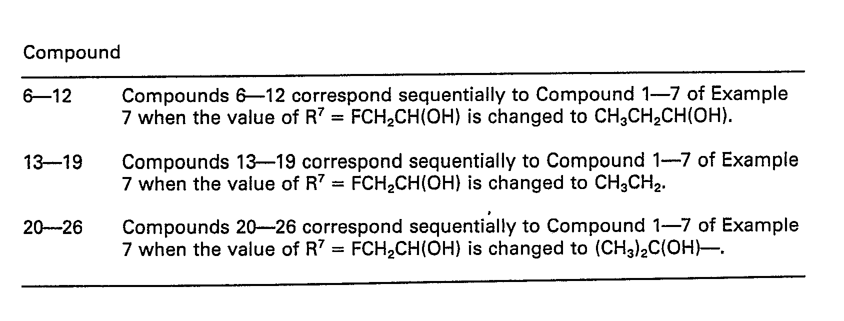

### Example 8

Following the procedure of the foregoing text and examples, the following "carbamimidoyl" embodiments of the present invention are obtained.

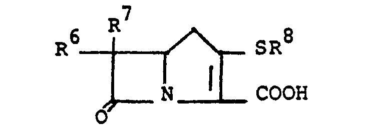

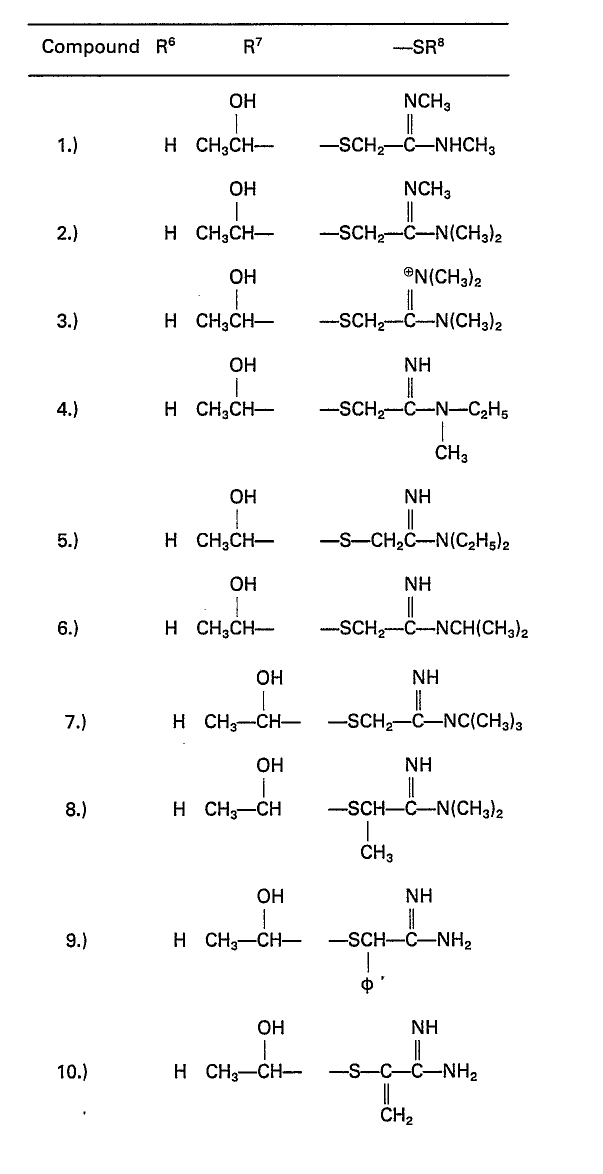

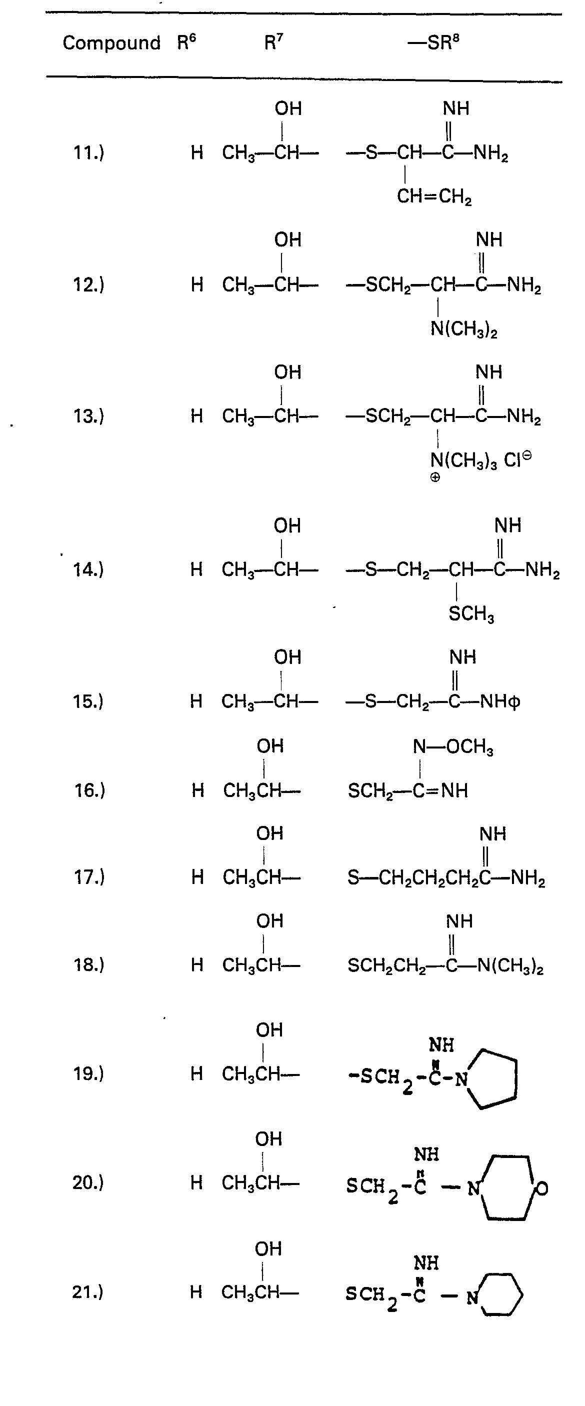

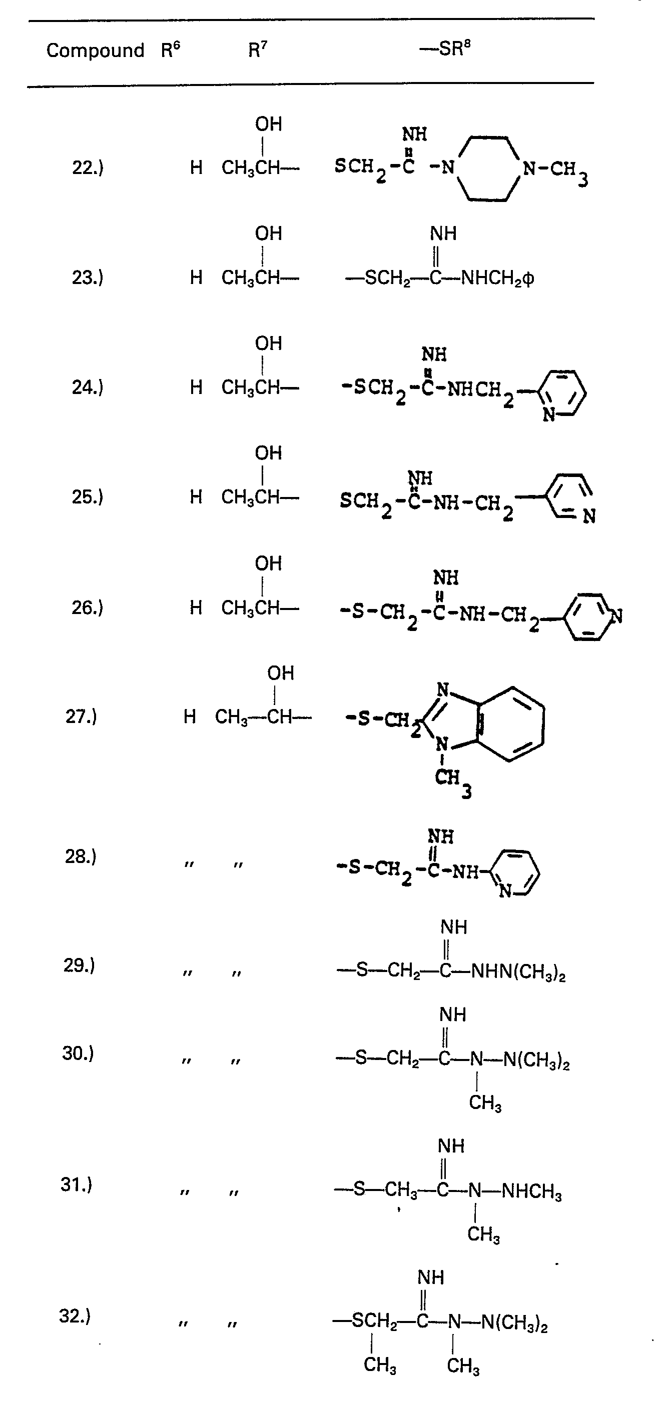

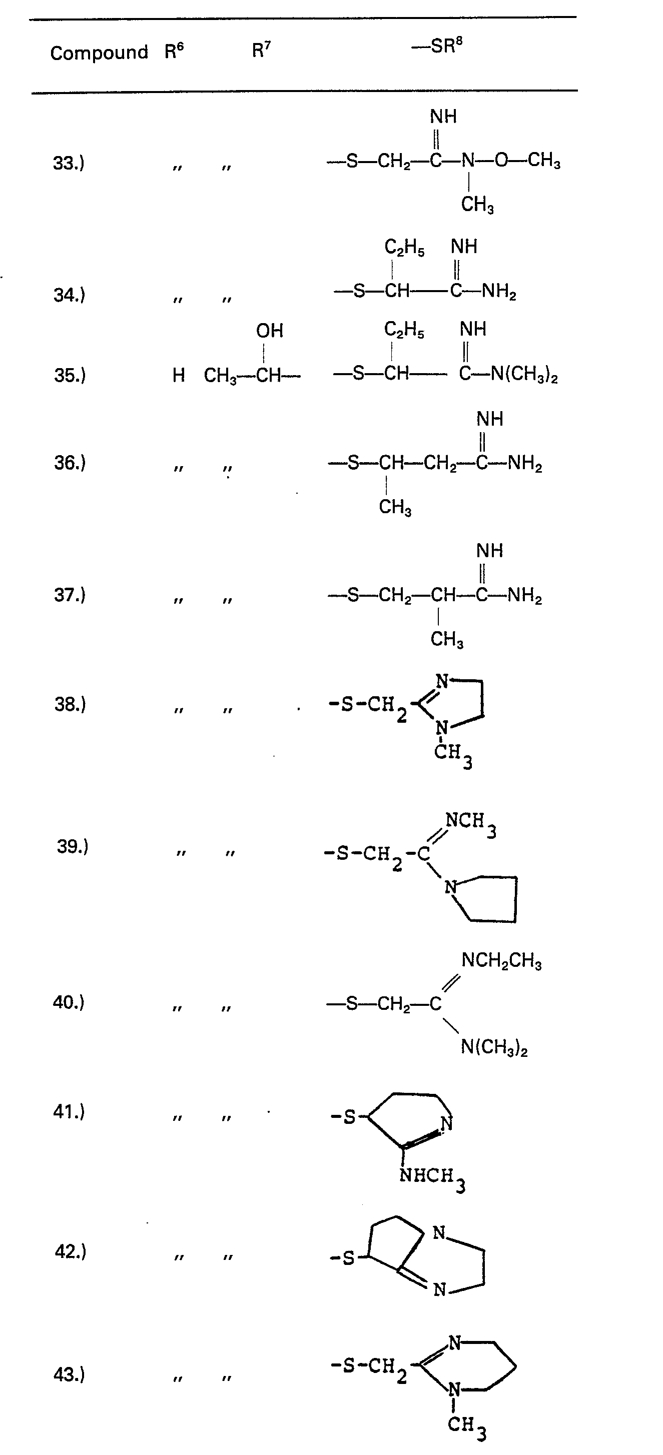

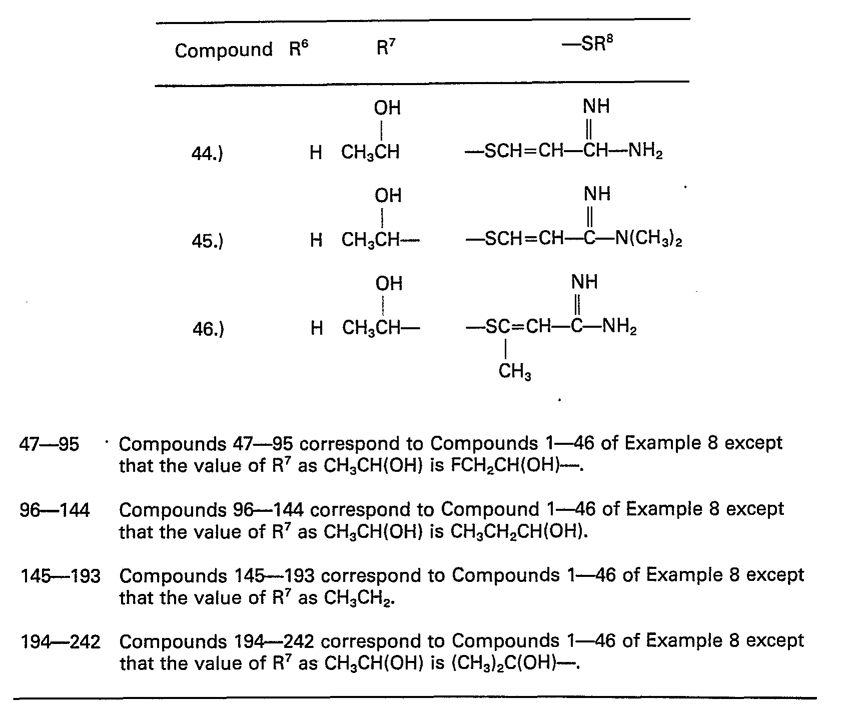

### Example 9

### Preparation of Pharmaceutical Compositions

One such unit dosage form is prepared by mixing 120 mg of the compound of Example 2 (Compound A) with 20 mg of lactose and 5 mg of magnesium stearate and placing the 145 mg mixture into a No. 3 gelatin capsule. Similarly, by employing more of the active ingredient and less lactose, other dosage forms can be put up in No. 3 gelatin capsules, and, should it be necessary to mire more than 145 mg of ingredients together, larger capsules such as compressed tablets and pills can be prepared. The following examples are illustrative of the preparation of pharmaceutical formulations:

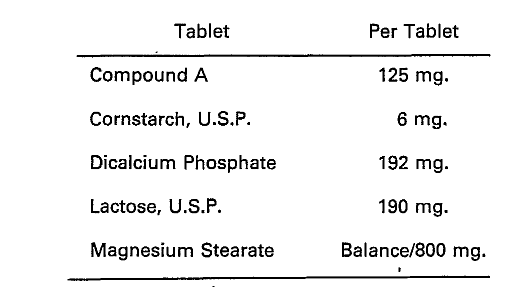

The active ingredient is blended with the dicalcium phosphate, lactose and about half of the cornstarch. The mixture is then granulated with 15% cornstarch paste (16 mg) and rough-screened. It is dried at 45°C and screened again through No. 16 screens. The balance of the cornstarch and magnesium stearate is added and the mixture is compressed into tablets, approximately 0.5 inch in diameter each weighing 800 mg.

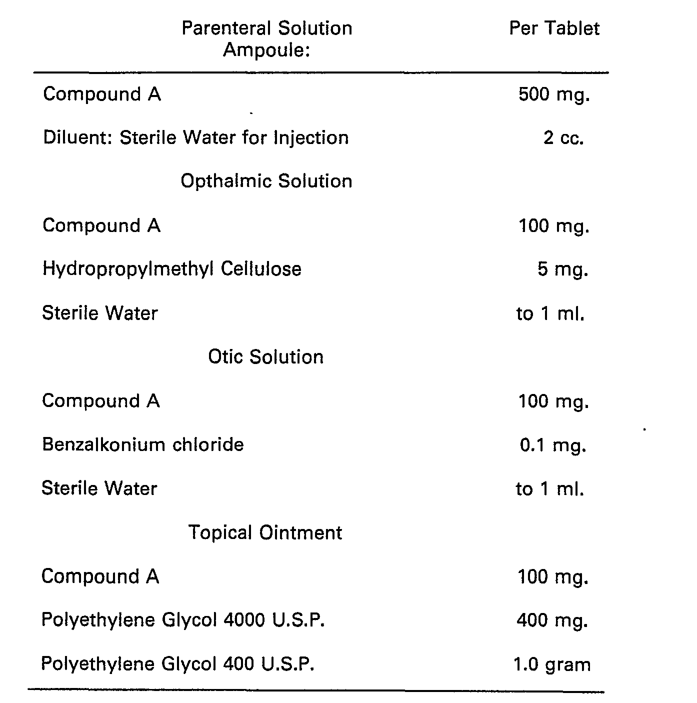

## Claims (Claims for the following Contracting State(s) : BE CH DE FR GB IT LI LU NL SE)

1. A compound having the structural formula: 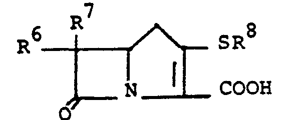 and the pharmaceutically acceptable salt, ester and amide derivatives thereof known in the (3-lactam antibiotic art; wherein:
R⁶ and R⁷, are independently selected from the group consisting of hydrogen; substituted and unsubstituted: alkyl, alkenyl, and alkynyl having 1-10 carbon atoms; cycloalkyl, cycloalkyalkyl, and alkylcycloalkyl having 3-6 carbon atoms in the cycloalkyl ring and 1―6 carbon atoms in the alkyl moiety; aryl, aralkyl, aralkenyl, and aralkynyl wherein the aryl moiety is phenyl and aliphatic portion has 1-6 carbon atoms;
R⁶ and R⁷ may be joined to form a cyclicalkyl having, together with the carbon atom to which they are attached, 3―6 carbon atoms; wherein the substituent or substituents on R⁶, and R⁷ are independently selected from chloro, fluoro, hydroxy, bromo, carboxyl, cyano, azido, amino, mono- and dialkylamino (each alkyl having 1-6 carbon atoms), ureido, alkylthio having 1-6 carbon atoms, and alkoxyl having 1-6 carbon atoms; and
R⁸ is a carbamimidoyl selected from the group consisting of: 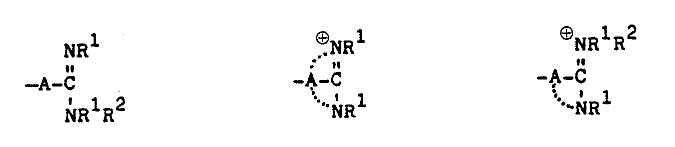 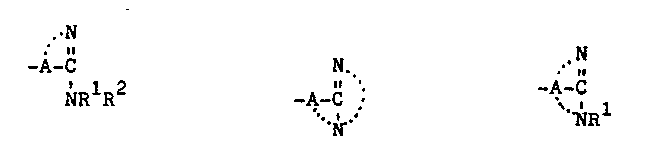 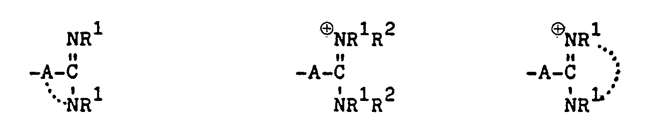 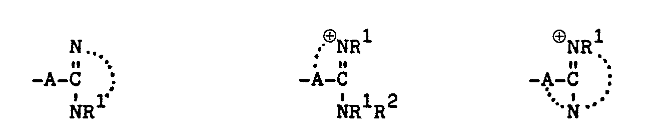 wherein:
A is a single, direct bond with the exception that A is not a single, direct bond with such compounds wherein the group ―C=N(NR¹R²) is not quarternary, or is a divalent cyclic or acyclic connector selected from the group consisting of alkylene, alkenylene, and alkynylene having 1-10 carbon atoms which may be interrupted by a hetero atom selected from O, S or N, or by phenylene cycloalkylene, cycloalkenylene having 3-6 carbon atoms, heterocyclylene or heteroarylene wherein such cyclic interruptions comprise 3-6 ring atoms selected from C, O, S and N; cycloalkylene, cycloalkenylene having 3-6 carbon atoms; heterocyclylene or heteroarylene each having 5-10 ring atoms and one or more hereo atoms selected from O, N and S; and phenylene;
R' and R² are independently selected from hydrogen and the previously defined, but monovalent, values for the group A; and wherein the dotted lines indicate provision for cyclic structures formed by the joinder of the indicated nitrogen atoms and the connector group A and by the joinder of the indicated nitrogen atoms, with the exception of compounds wherein a) R⁸ 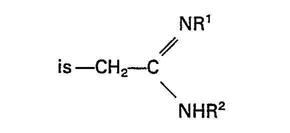 wherein R¹/R² are H,H; CH₃, H; or 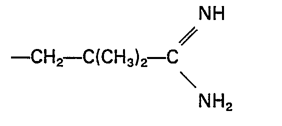 or 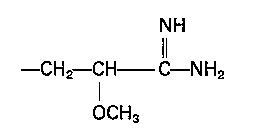 (b) R⁶ is 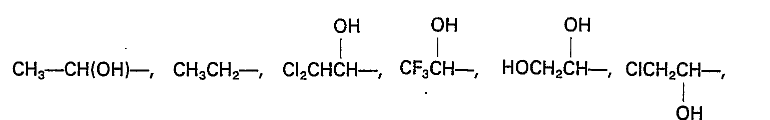 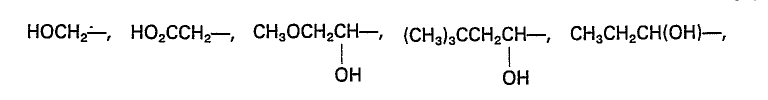 cyclopropyl-CH(OH)-, NH₂CH₂CH(OH)- or CF₂HCH(OH)-; R⁷ is H and 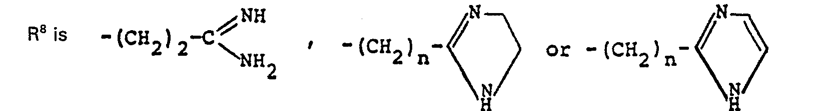 wherein n is 1 or 2 and (c) R⁶ is (CH₃)₂CH-CH(OH)-R⁷ is Hand

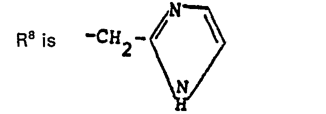

2. A compound according to Claim 1 wherein ―SR⁸ is selected from the group consisting of: 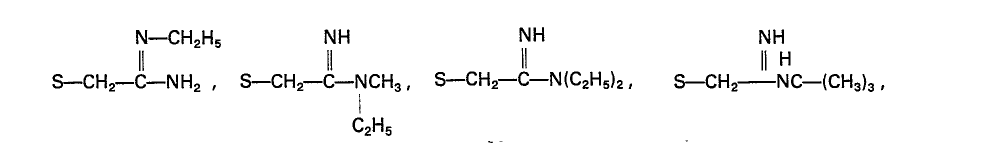 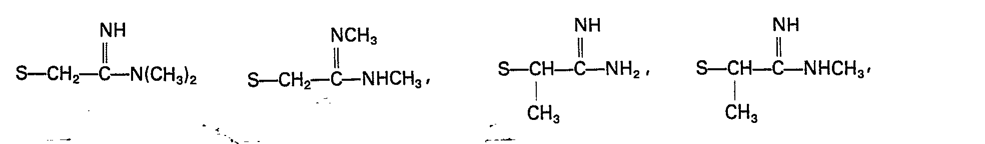 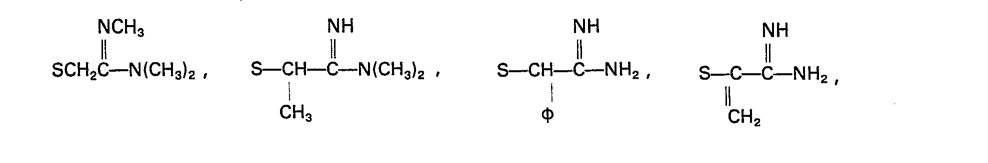 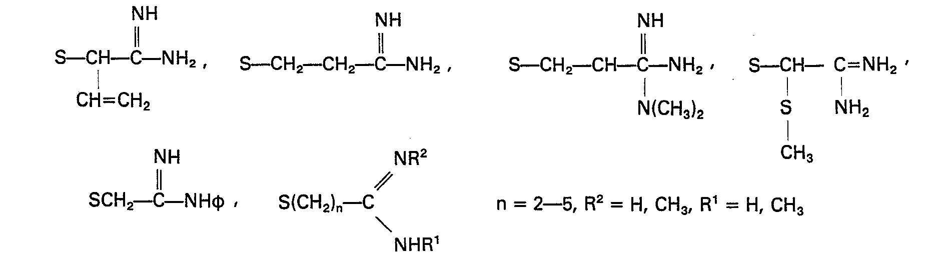 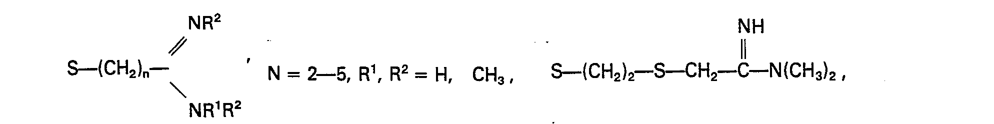 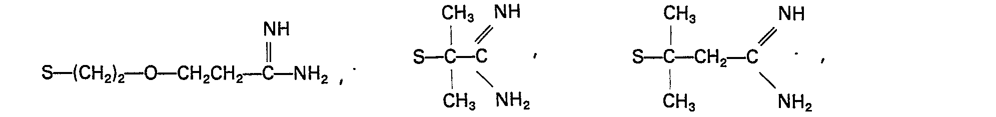 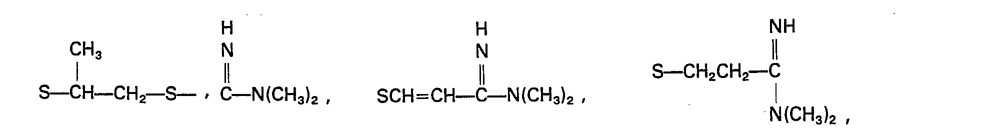 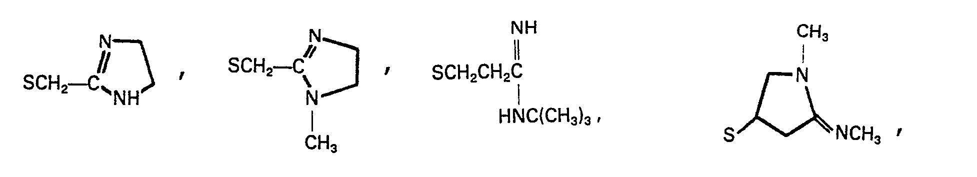 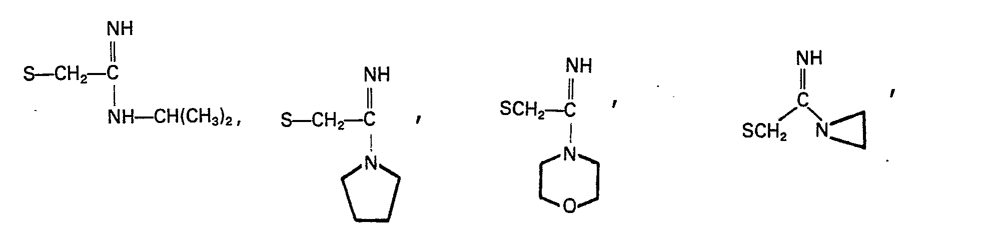 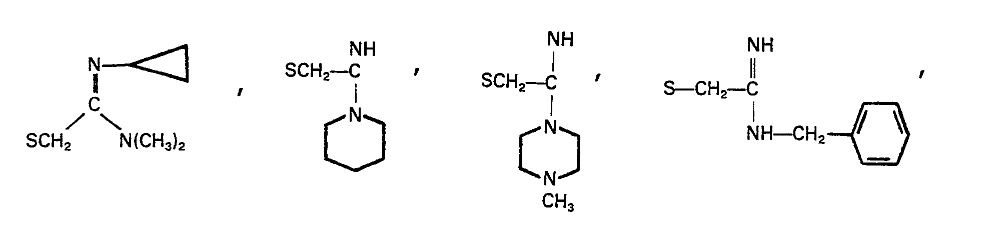 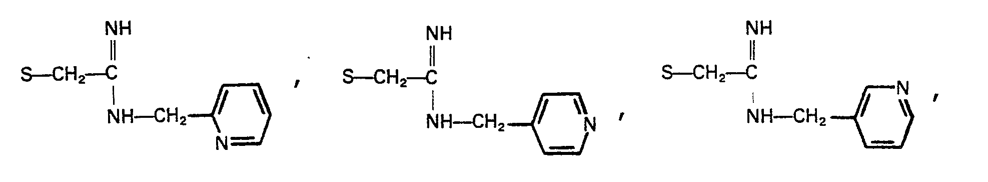 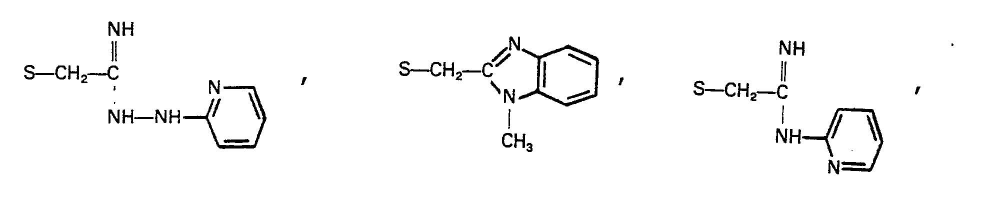 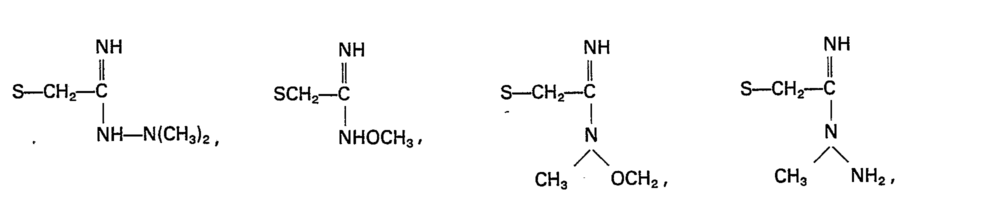 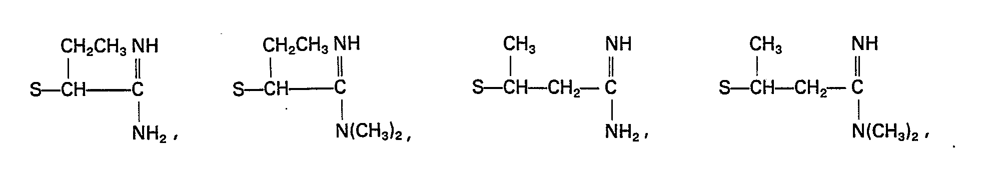 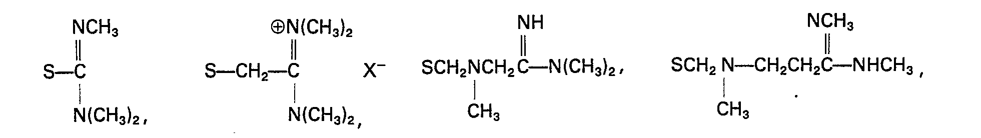 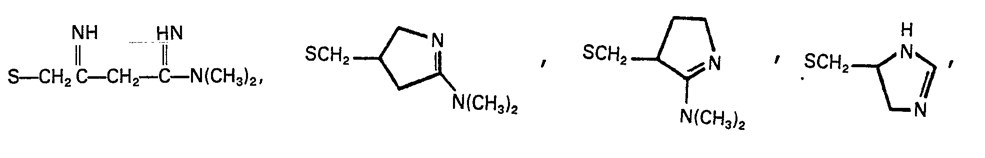 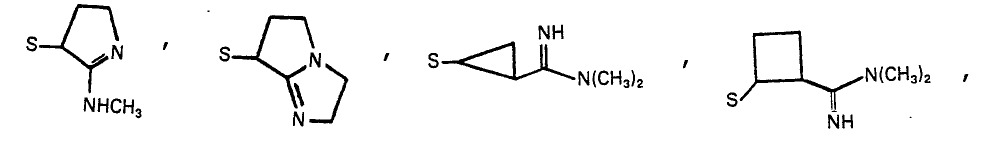 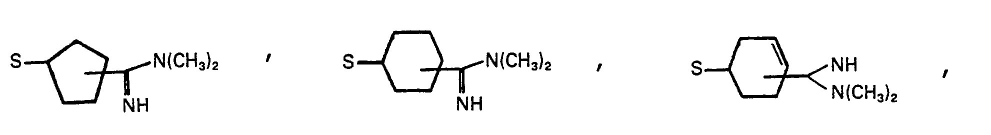 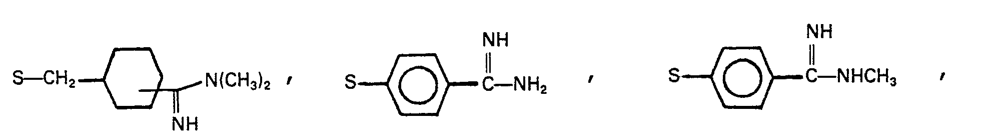 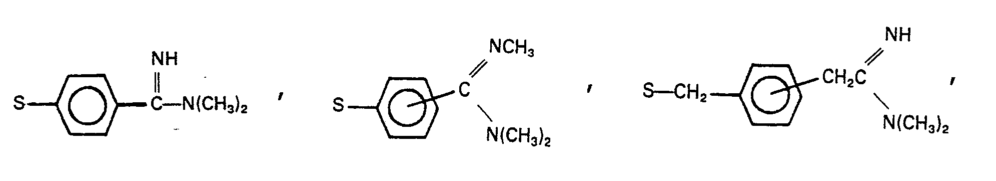 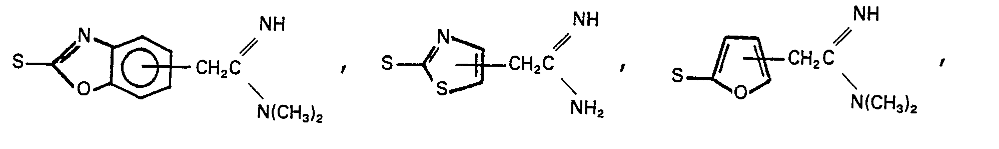 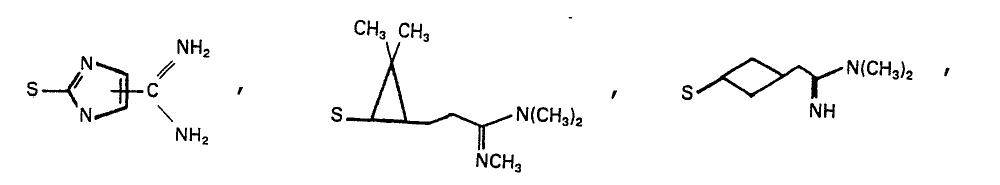 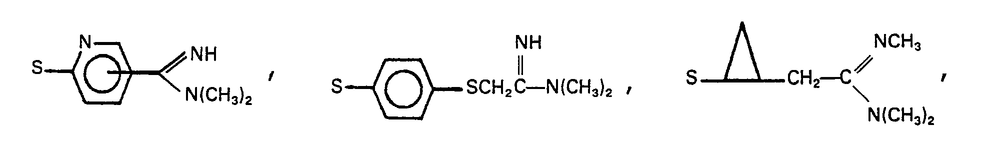 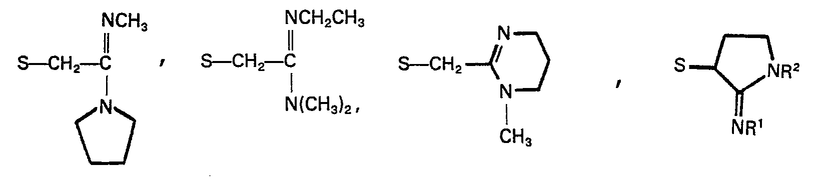 R¹ = H, CH₃ R² = H, CH₃ 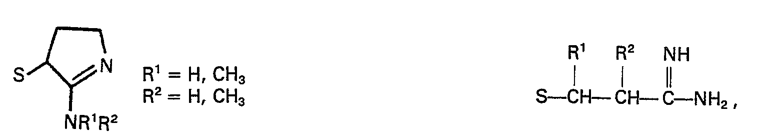 R¹ = CH₃ R² = CH₃, NR₂, OR R = H, CH₃

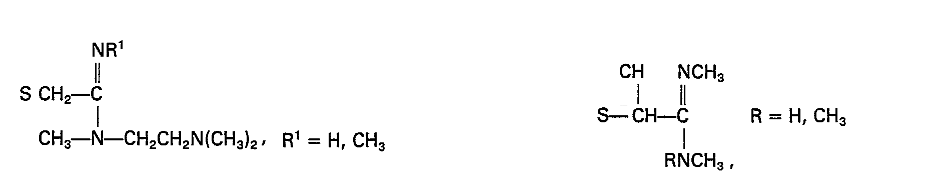

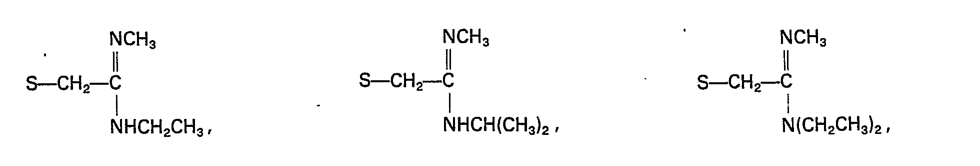

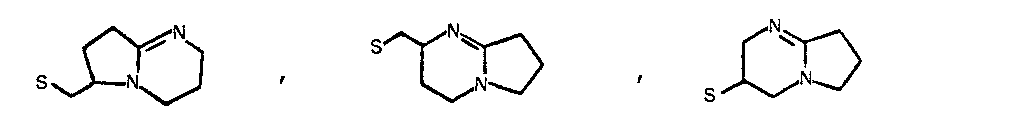

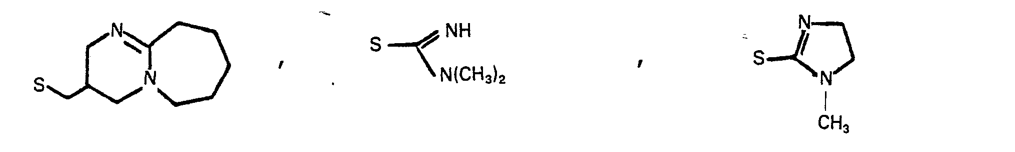

3. A compound according to Claims 1 or 2, wherein R⁷ is selected from H and CH₃.

4. A compound according to Claims 1-3, wherein R⁶ is selected from the group consisting of: substituted and·unsubstituted: alkyl, alkenyl and cycloalkyalkyl wherein the substituent or substituents are selected from hydroxyl, alkoxyl having from 1-6 carbon atoms, amino, and carboxy.

5. A compound according to Claims 1-4 wherein R⁶ is selected from the group consisting of alkyl, cycloalkylalkyl, alkyl substituted by one or more hydroxyl groups, or cycloalkylalkyl substituted by one ore more hydroxyl groups.

6. A compound according to Claims 1-5 wherein R⁷ is hydrogen.

7. A compound according to Claims 1-6 wherein R⁶ is selected from: hydrogen,

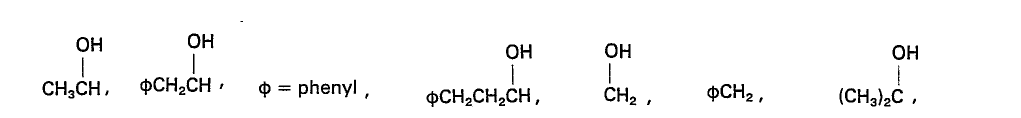

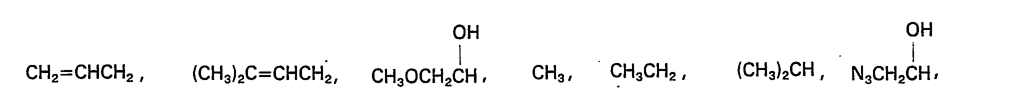

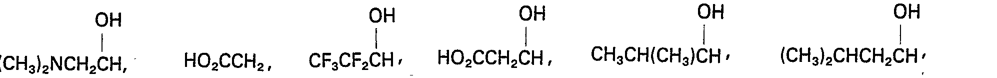

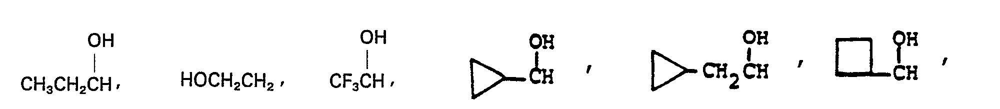

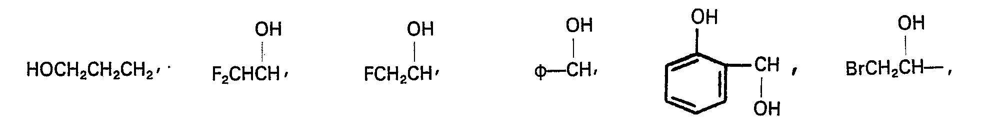

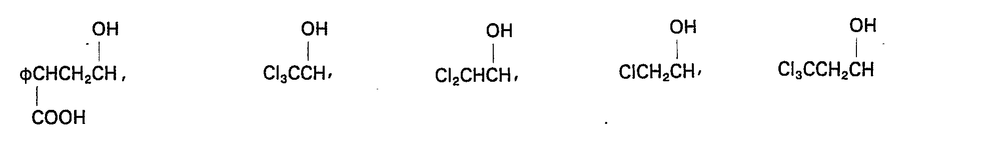

8. A compound according to Claims 1-7, wherein R⁶ is hydrogen,

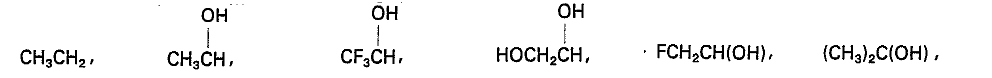

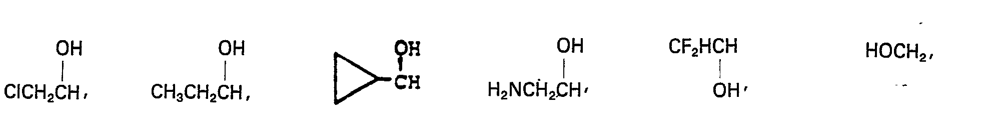

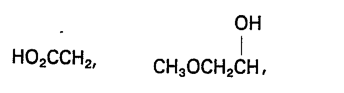

9. A compound according to Claims 1-8 wherein R⁶ is: hydrogen; substituted and unsubstituted: alkyl, cycloalkyl, cycloalkylalkyl, phenylalkyl; wherein the substituent or substituents are selected from: hydroxyl, chloro, fluoro, bromo, carboxyl, ureido, amino, alkoxyl, or alkylthio.

10. A compound according to Claims 1-9 wherein R⁶ is selected from:

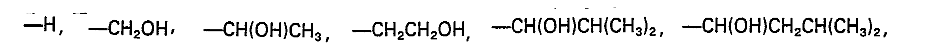

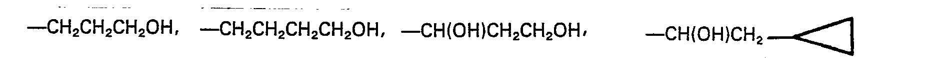

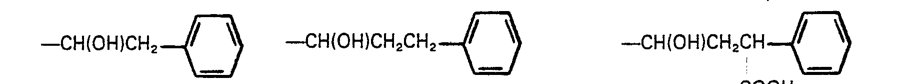

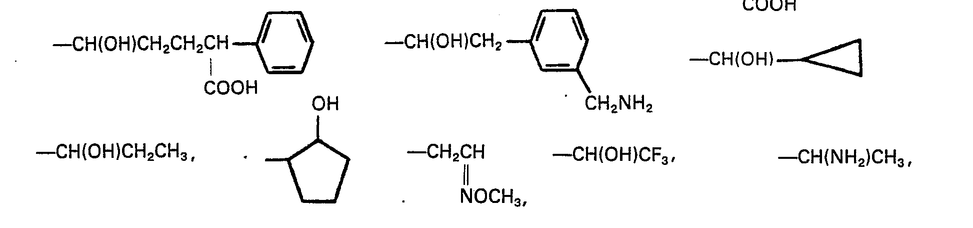

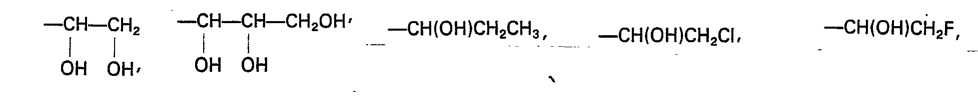

11. A process for preparing a compound according to claim 1 comprising the steps of treating: 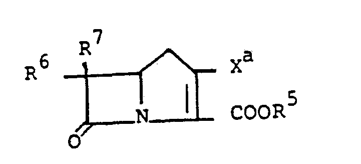 with HSR⁸; wherein X^{a} is a leaving group and R⁵ is a protecting group.

12. An antibiotic composition comprising a therapeutically effective amount of a compound according to Claim 1 and a pharmaceutically effect carrier therefor.

13. A compound according to Claim 2 wherein R⁷ is hydrogen and R⁶ is

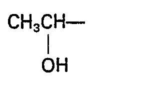

## Claims (Claims for the following Contracting State(s) : AT)

1. A process for preparing a compound of the structural formula: 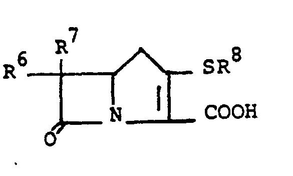 and the pharmaceutically acceptable salt, ester and amide derivatives thereof known in the β-lactam antibiotic art; wherein:
R⁶ and R⁷, are independently selected from the group consisting of hydrogen; substituted and unsubstituted: alkyl, alkenyl, and alkynyl having 1-10 carbon atoms; cycloalkyl, cycloalkylalkyl, and alkylcycloalkyl having 3―6 carbon atoms in the cycloalkyl ring and 1-6 carbon atoms in the alkyl moiety; aryl, aralkyl, aralkenyl, and aralkynyl wherein the aryl moiety is phenyl and aliphatic portion has 1-6 carbon atoms; R⁶ and R⁷ may be joined to form a cyclicalkyl having, together with the carbon atom to which they are attached, 3―6 carbon atoms; wherein the substituent or substituents on R⁶, and R⁷ are independently selected from chloro, fluoro, hydroxy, bromo, carboxyl, cyano, azido, amino, mono- and dialkylamino (each alkyl having 1-6 carbon atoms), ureido, alkylthio having 1-6 carbon atoms, and alkoxyl having 1-6 carbon atoms; and R^{B} is a carbamimidoyl selected from the group consisting of: 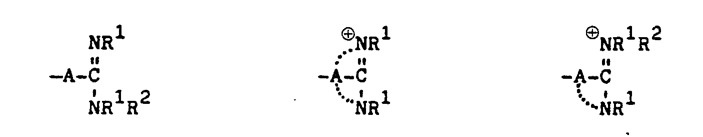 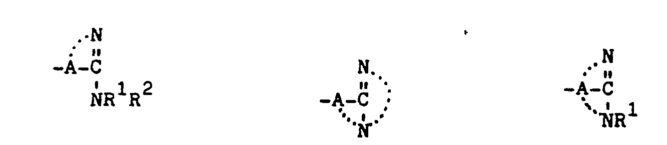 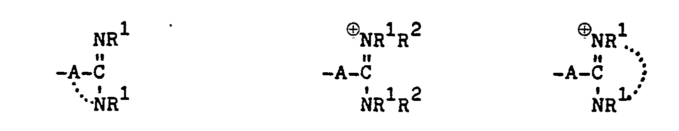 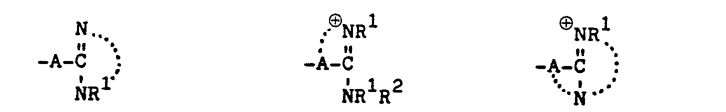 wherein: A is a single, direct bond with the exception that A is not a single, direct bond with such compounds wherein the group ―C=N(NR¹R²) is not quarternary, or is a divalent cyclic or acyclic connector selected from the group consisting of alkylene, alkenylene, and alkynylene having 1-10 carbon atoms which may be interrupted by a hetero atom selected from 0, S or N, or by phenylene cycloalkylene, cycloalkenylene having 3-6 carbon atoms; heterocyclylene or heteroarylene wherein such cyclic interruptions comprise 3―6 ring atoms selected from C, 0, S an N; cycloalkylene, cycloalkenylene having 3―6 carbon atoms; heterocyclylene or hetereoarylene each having 5-10 ring atoms and one or more hetero atoms selected from 0, N and S; an phenylene; R' and R² are independently selected from hydrogen and the previously defined, but monovalent, values for the group A; and wherein the dotted lines indicate provision for cyclic structures formed by the joinder of the indicated nitrogen atom and the connector group A and by the joinder of the indicated nitrogen atoms, with the exception of compounds wherein 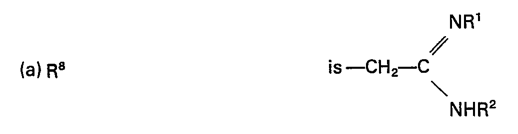 wherein R'/R² are H, H; CH₃, H; or 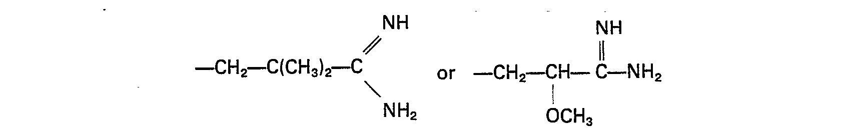 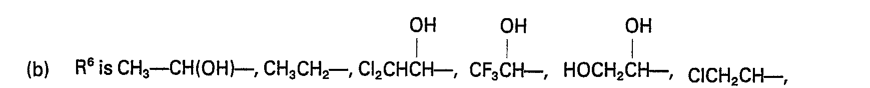 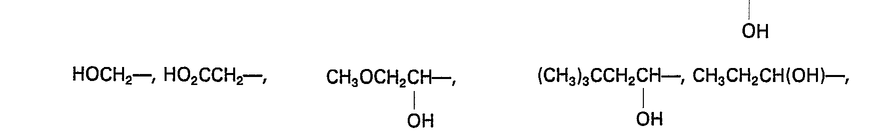 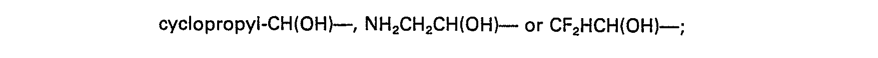 R⁷ is H and 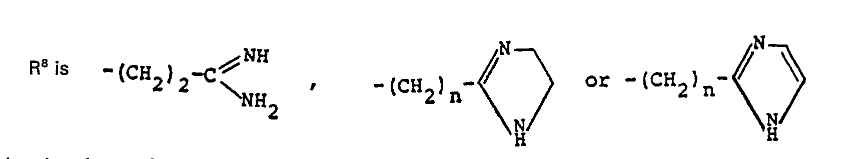 wherein n is 1 or 2 and (c) R⁶ is (CH₃)₂CH-CH(OH)-R⁷ is H and 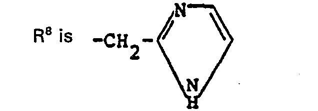 comprising the step of treating: 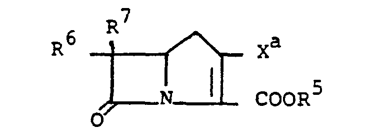 with HSR⁸; wherein X^{a} is a leaving group and R⁵ is a protecting group.

2. The process according to Claim 1 wherein ―SR⁸ is selected from the group consisting of: 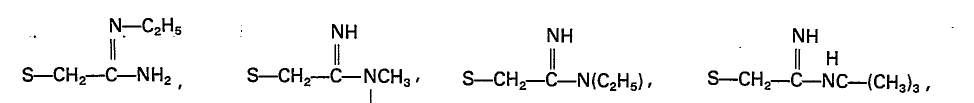 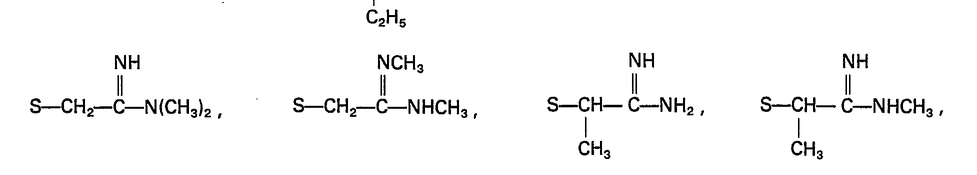 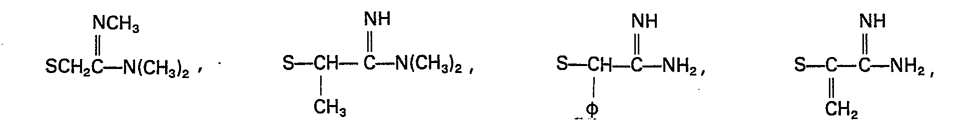 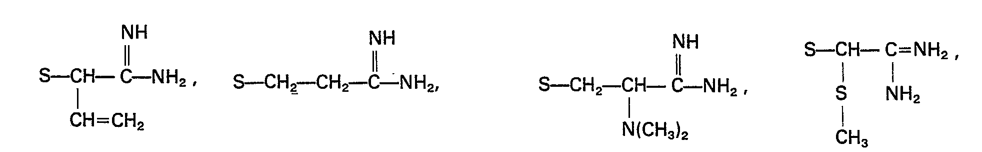 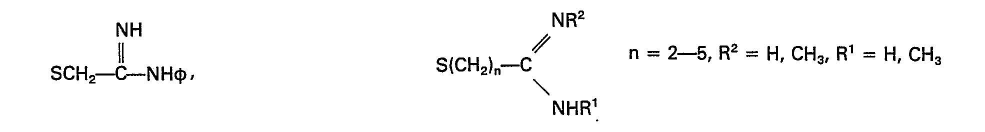 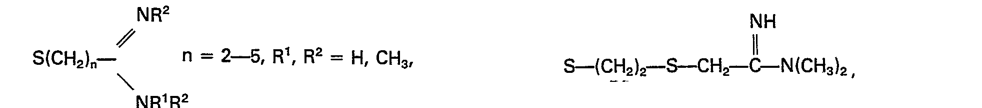 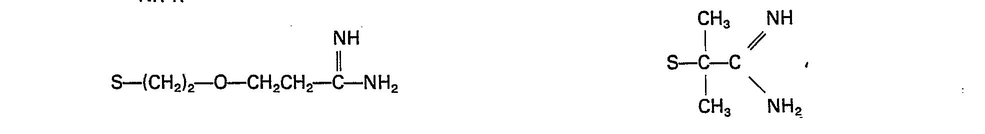 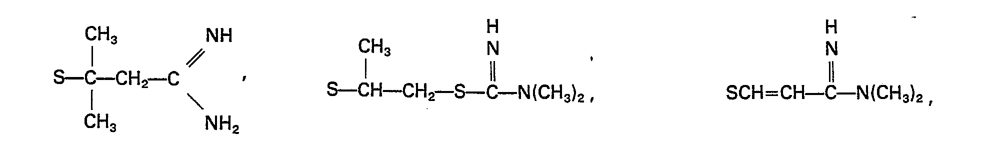 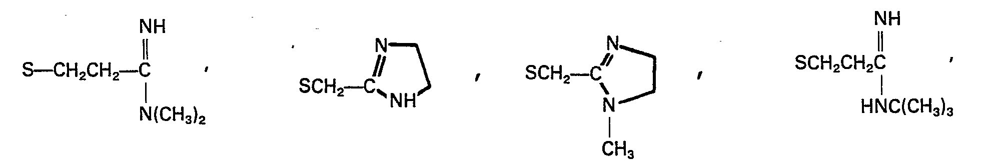 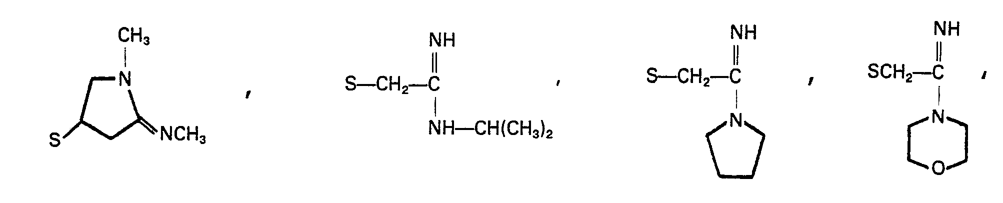 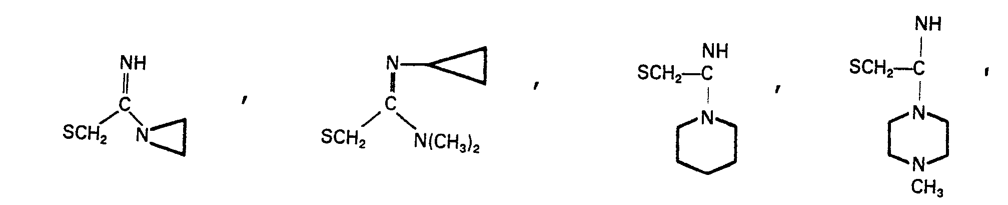 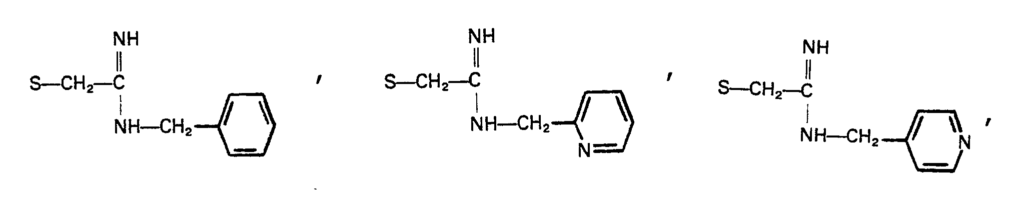 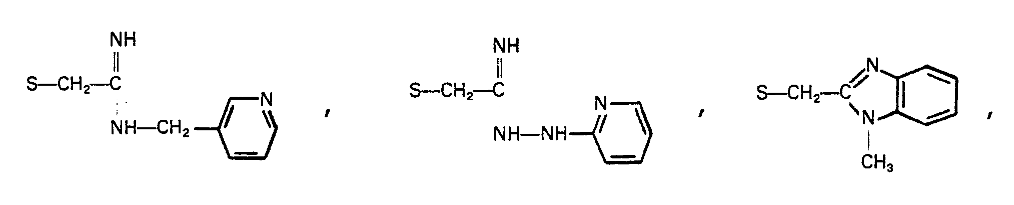 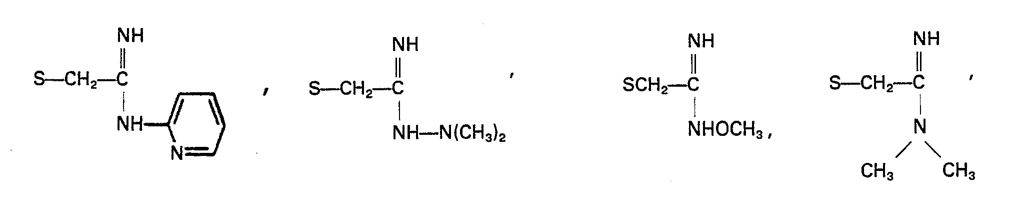 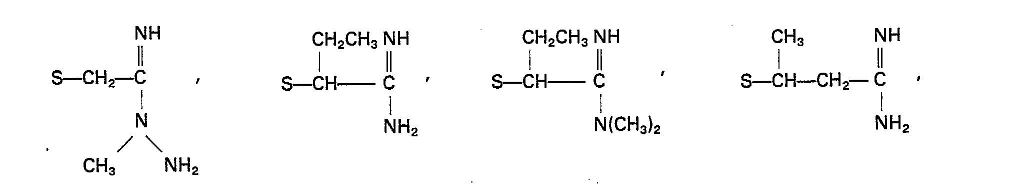 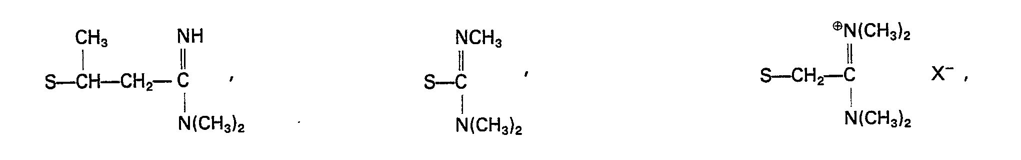 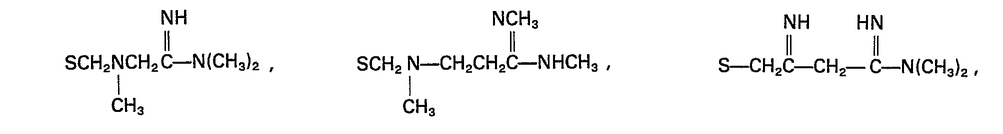 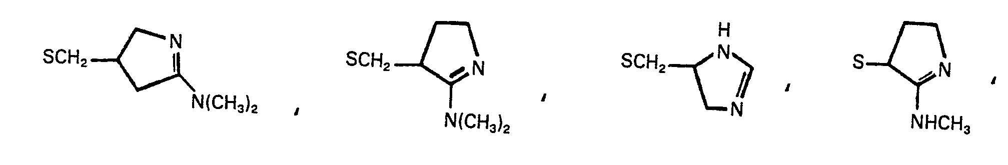 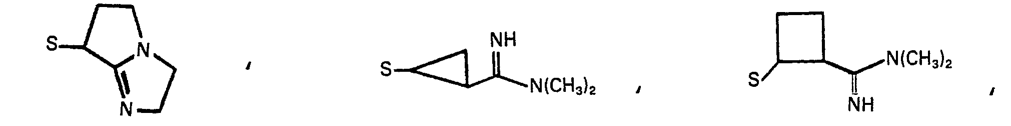 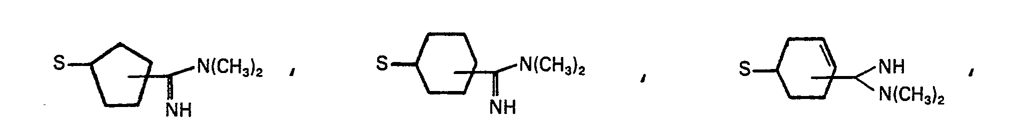 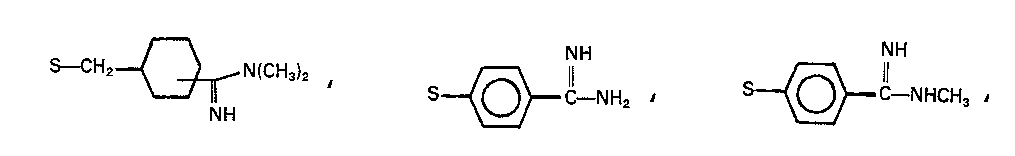 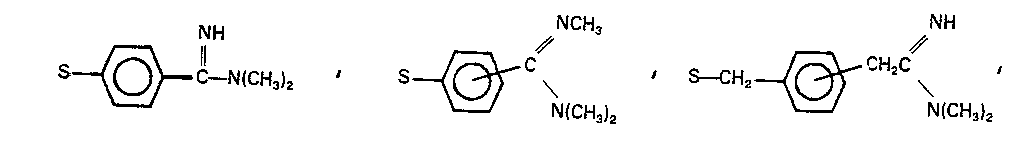 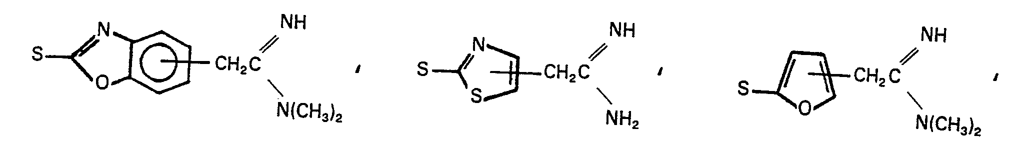 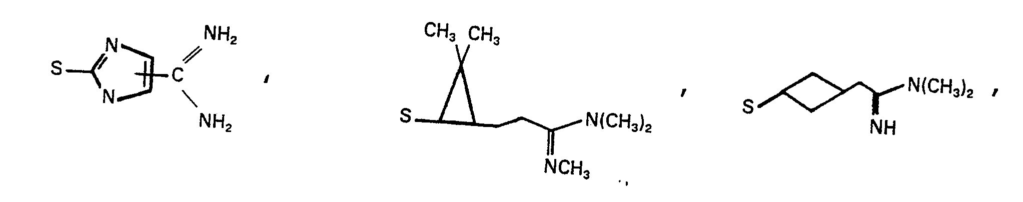 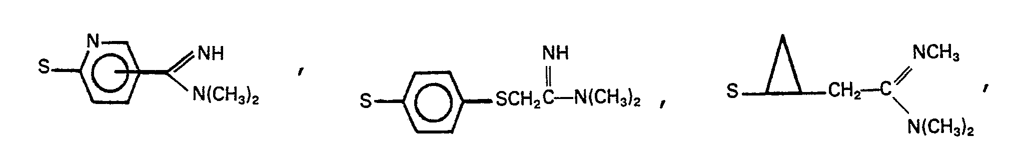 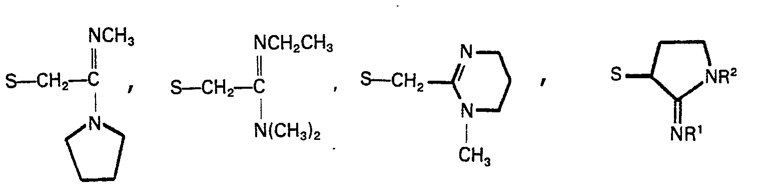 R¹ = H, CH₃ R² = H, CH₃ 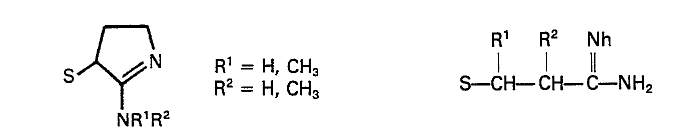 R¹ = CH₃ R² = CH₃, NR₂, OR R = H, CH₃

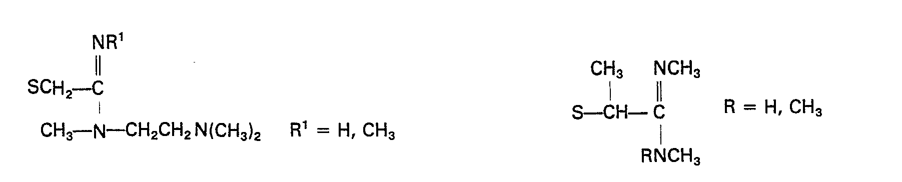

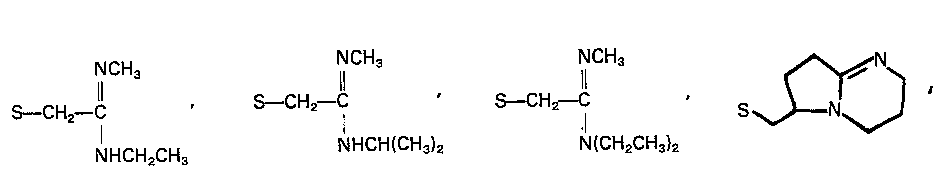

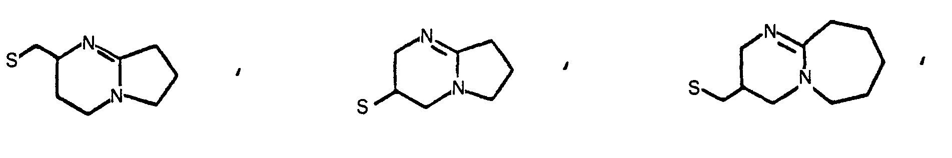

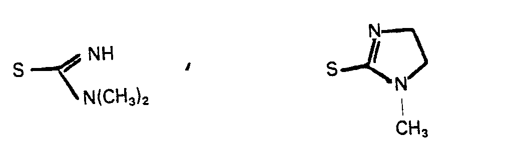

3. The process according to Claims 1 or 2, wherein R⁷ is selected from H and CH₃.

4. The process according to Claims 1-3, wherein R⁶ is selected from the group consisting of: substituted and unsubstituted: alkyl, alkenyl and cycloalkylalkyl wherein the substituent or substituents are selected from hydroxyl, alkoxyl having from 1-6 carbon atoms, amino, and carboxy.

5. The process according to Claims 1-4 wherein R⁶ is selected from the group consisting of alkyl, cycloalkylalkyl, alkyl substituted by one or more hydroxyl groups, or cycloalkylalkyl substituted by one or more hydroxyl groups.

6. The process according to Claims 1-5 wherein R⁷ is hydrogen.

7. The process according to Claims 1-6 wherein R⁶ is selected from: hydrogen,

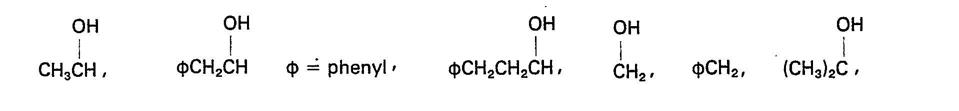

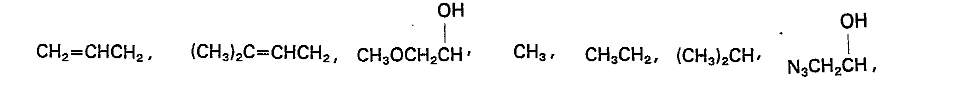

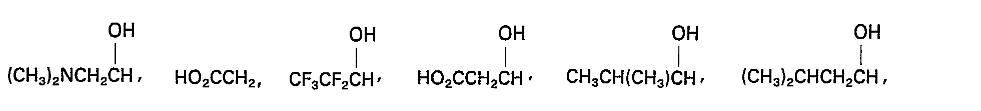

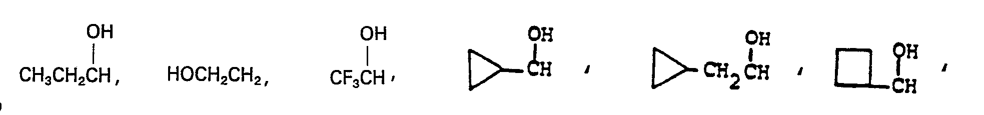

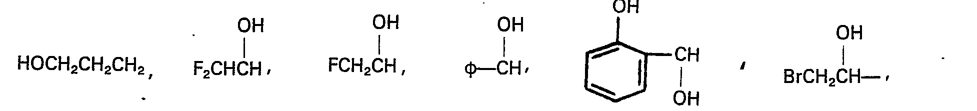

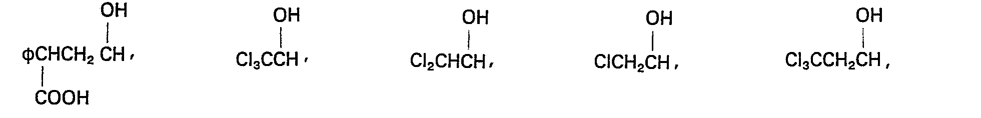

8. The process according to Claims 1-7, wherein R⁶ is hydrogen,

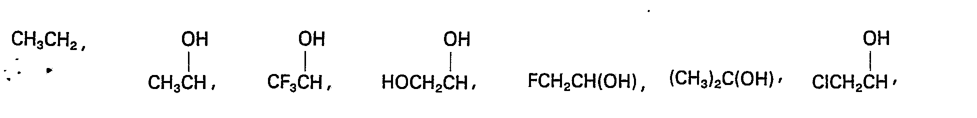

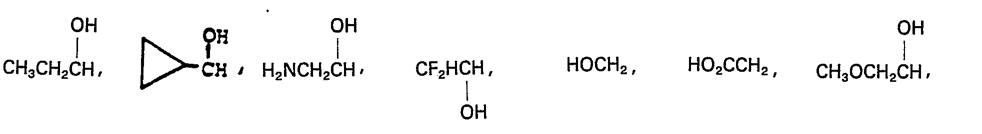

9. The process according to Claims 1-8 wherein R⁶ is: hydrogen; substituted and unsubstituted: alkyl, cycloalkyl, cycloalkylalkyl, phenylalkyl; wherein the substituent or substituents are selected from: hydroxyl, chloro, fluoro, bromo, carboxyl, ureido, amino, alkoxyl, or alkylthio.

10. The process according to Claims 1-9 wherein R⁶ is selected from:

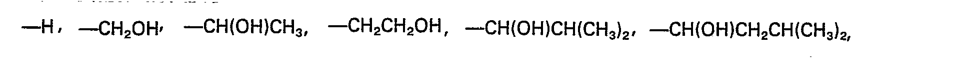

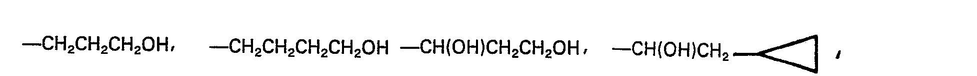

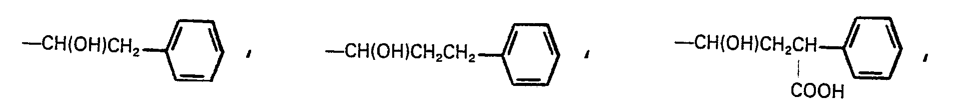

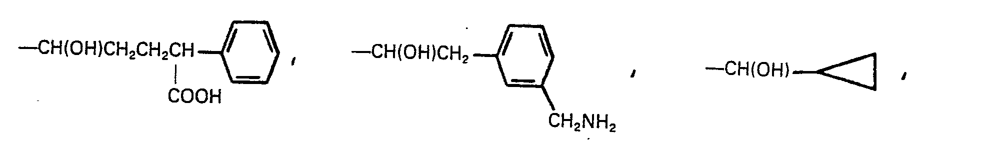

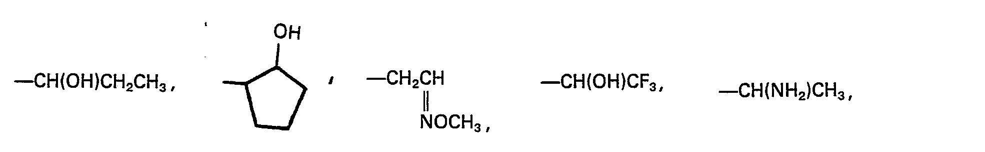

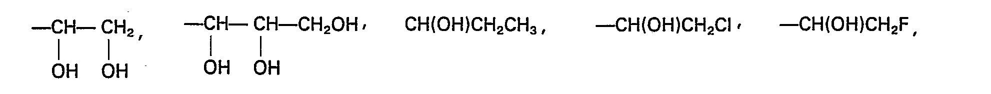

## Patentansprüche (Patentansprüche für folgende(n) Vertragsstaat(en) : BE CH DE FR GB IT LI LU NL SE)

1. Eine Verbindung der Strukturformel: 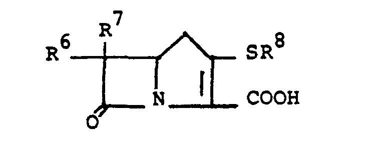 und die pharmazeutisch annehmbaren Salz-, Eter- und Amidderivate davon, wie sie auf dem Gebiet der β-Lactamantibiotika bekannt sind, worin:
R⁶ und R⁷ unabhängig voneinander aus der Gruppe ausgewählt sind, die aus Wasserstoff; substituiertem und unsubstituiertem: Alkyl, Alkenyl und Alkinyl mit 1-10 Kohlenstoffatomen; Cycloalkyl, Cycloalkylalkyl und Alkylcycloalkyl mit 3-6 Kohlenstoffatomen in dem Cycloalkylring und 1―6 Kohlenstoffatomen in dem Alkylrest; Aryl, Aralkyl, Aralkenyl und Aralkinyl, worin der Arylrest Phenyl ist und der aliphatische Abschnitt 1―6 Kohlenstoffatome hat, besteht; wobei R⁶ und R⁷ unter Bildung eines Cycloalkyls verbunden sein können, welches zusammen mit dem Kohlenstoffatom, an das sie gebunden sind, 3―6 Kohlenstoffatome aufweist; und wobei der Substituent oder die Substituenten an R und R⁷ unabhängig voneinander aus Chlor, Fluor, Hydroxy, Brom, Carboxyl, Cyano, Azido, Amino, Mono- und Dialkylamino (jedes Alkyl hat 1-6 Kohlenstoffatome), Ureido, Alkylthio mit 1-6 Kohlenstoffatomen und Alkoxyl mit 1-6 Kohlenstoffatomen ausgewählt sind; und R⁸ ein Carbamimidoyl ist, das aus der Gruppe ausgewählt ist, welche aus: 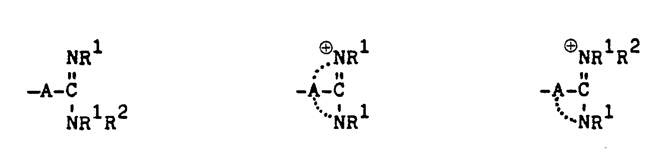 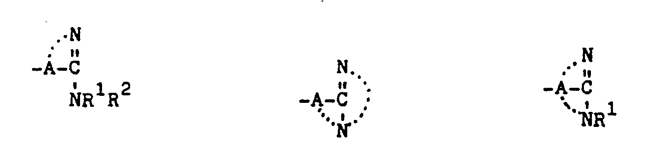 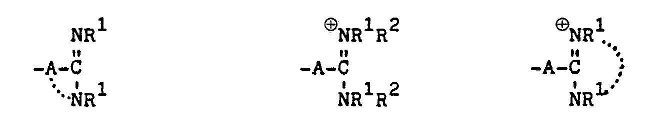 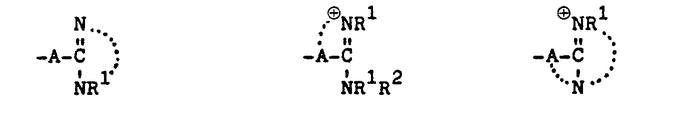 besteht, worin: A eine einfache, direkte Bindung mit der Ausnahme ist, daß A nicht eine einfache, direkte Bindung bei solchen Verbindungen darstellt, worin die Gruppe―C=N(NR¹R²) nicht quaternär ist; oder ein zweiwertiges, cyclisches oder acyclisches Verbindungsglied ist, das aus der Gruppe ausgewählt ist, welche aus Alkylen, Alkenylen und Alkinylen mit 1-10 Kohlenstoffatomen, die durch ein Heteroatom, ausgewählt aus O, S und N, oder durch Phenylen, Cycloalkylen, Cycloalkenylen mit 3―6 Kohlenstoffatomen, Heterocyclylen oder Heteroarylen unterbrochen sein können, wobei solche cyclischen Unterbrechungen 3-6 Ringatome, ausgewählt aus C, 0, S und N, umfassen; Cycloalkylen, Cycloalkenylen mit 3-6 Kohlenstoffatomen; Heterocyclylen oder Heteroarylen, von denen jedes 5-10 Ringatome und eine oder mehrere, aus 0, N und S ausgewählte Heteroatome enthält; und Phenylen besteht; R' und R² unabhängig voneinander aus Wasserstoff und den vorher definierten, jedoch einwertigen, Bedeutungen für die Gruppe A ausgewählt sind; und wobei die punktierten Linien Möglichkeiten für cyclische Strukturen angeben, die durch das Verbinden des angegebenen Stickstoffatoms und der Verbindungsgruppe A und durch das Verbinden der angegebenen Stickstoffatome gebildet werden, mit der Ausnahme von Verbindungen, worin (a) R⁸ 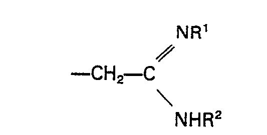 wobei R¹/R² H, H; CH₃, H sind; oder 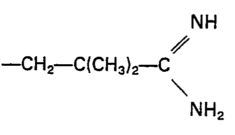 oder 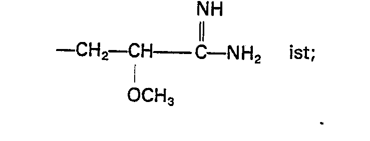 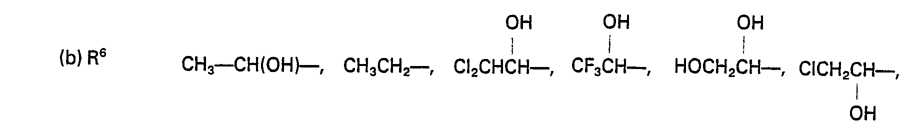 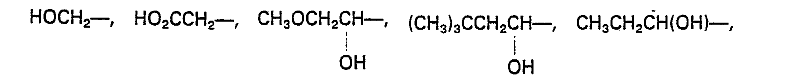 cyclopropyl―CH(OH)―, NH₂CH₂CH(OH)― or CF₂HCH(OH)-; R⁷ H ist und 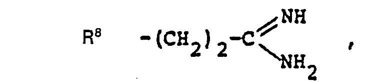 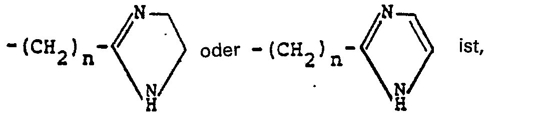 wobei n 1 oder 2 ist; und (c) R⁶ (CH₃)₂CH―CH(OH)― ist, R⁷ H ist und _{R}8 _{R}⁸ 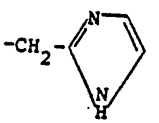 ist.

2. Eine Verbindung nach Anspruch 1, wobei ―SR⁸ aus der Gruppe ausgewählt ist, die aus: 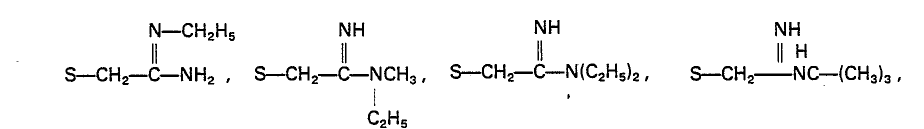 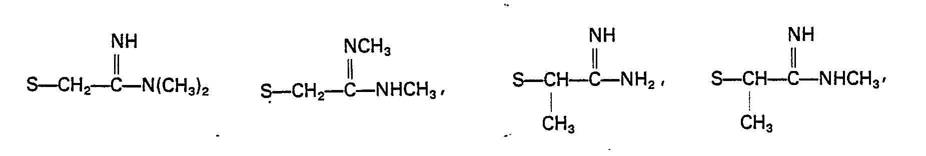 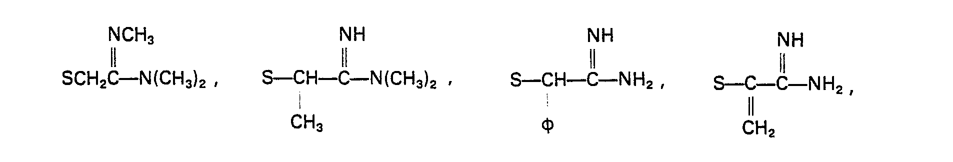 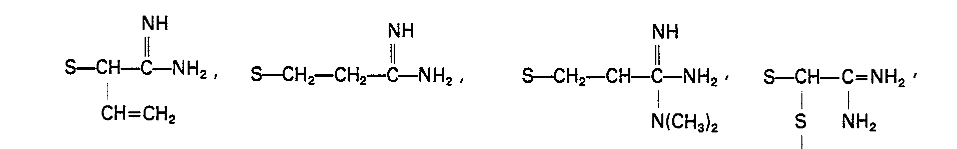 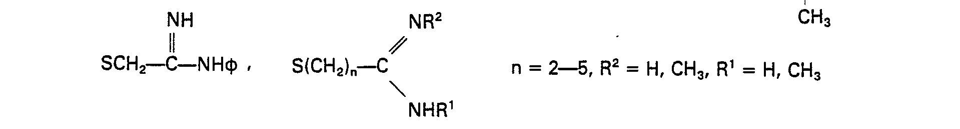 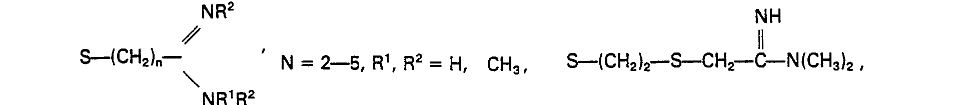 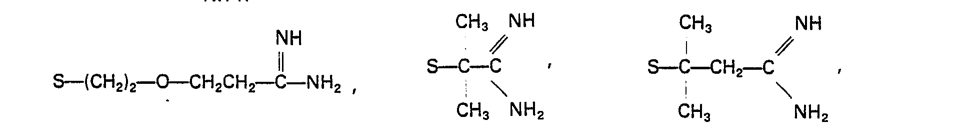 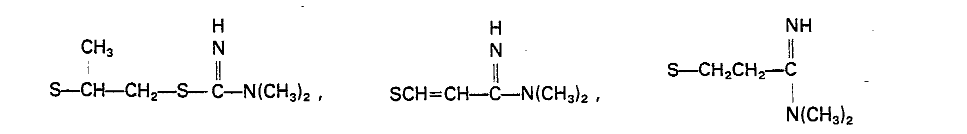 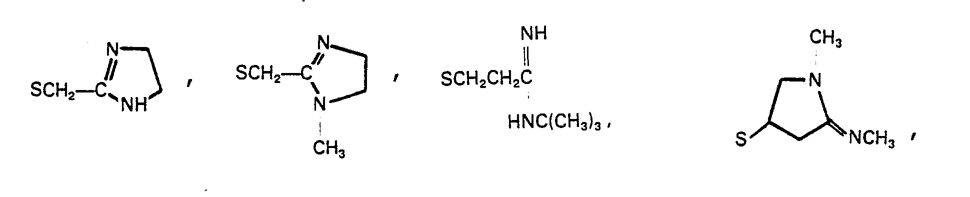 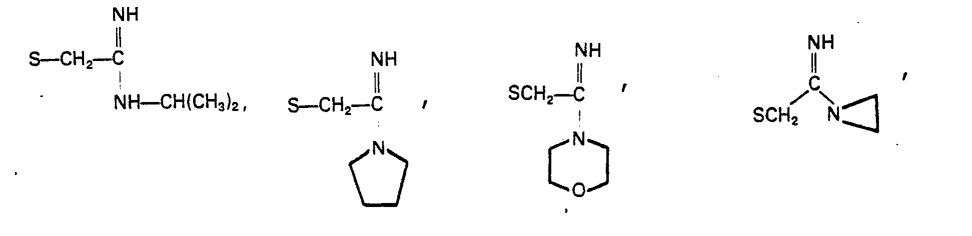 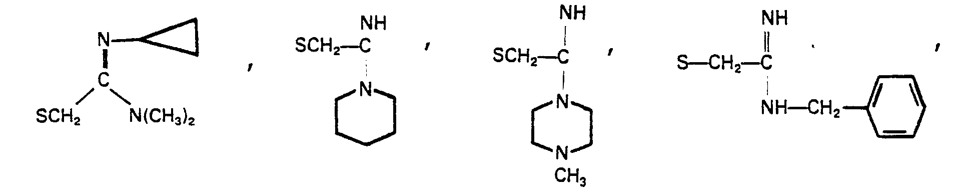 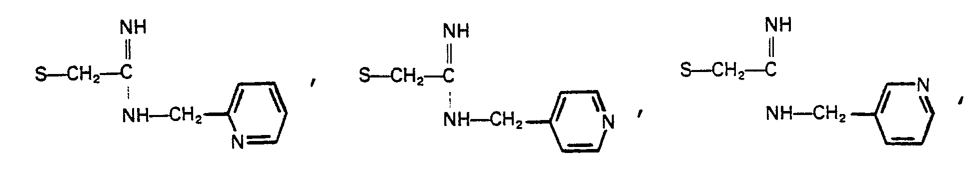 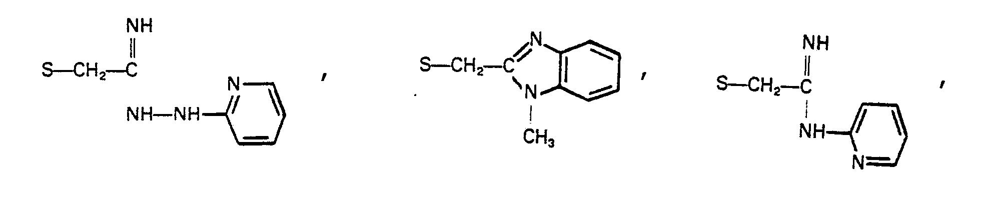 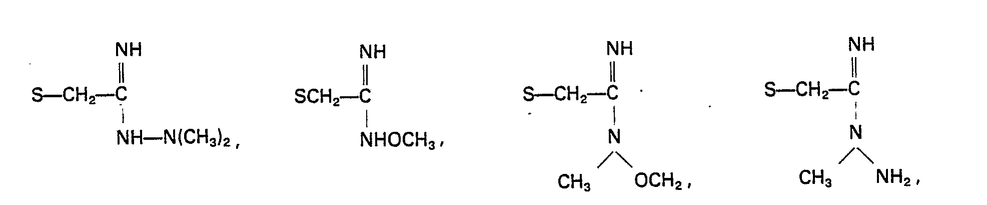 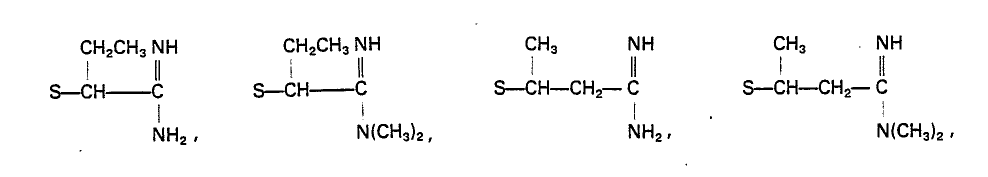 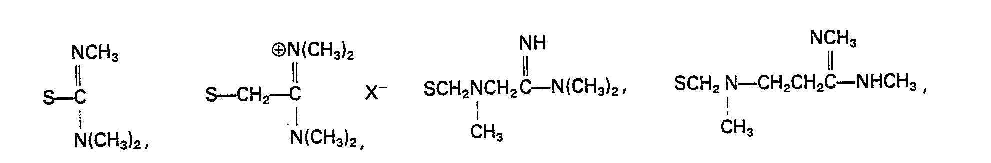 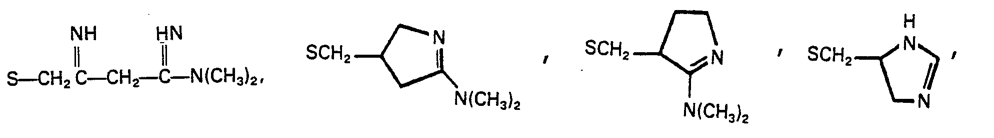 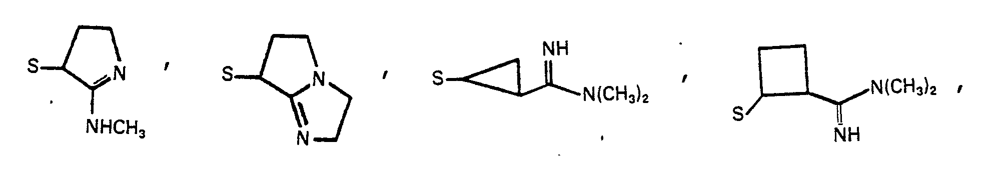 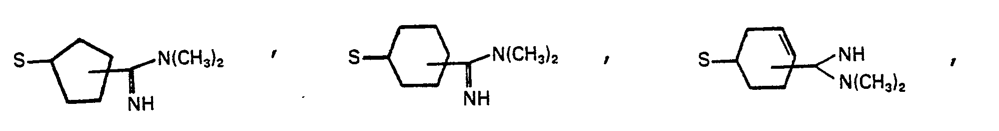 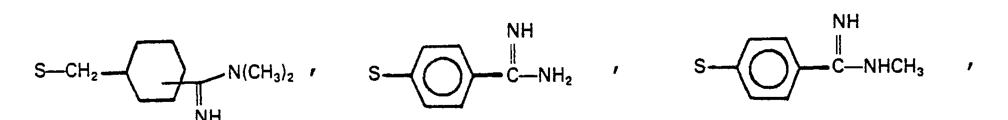 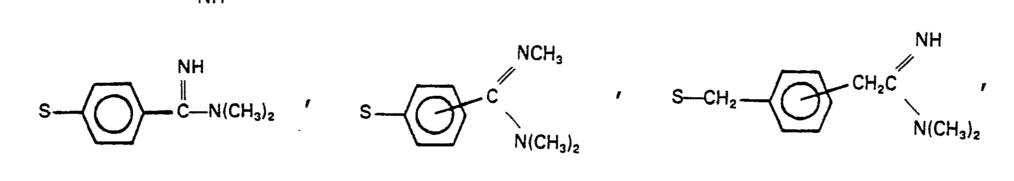 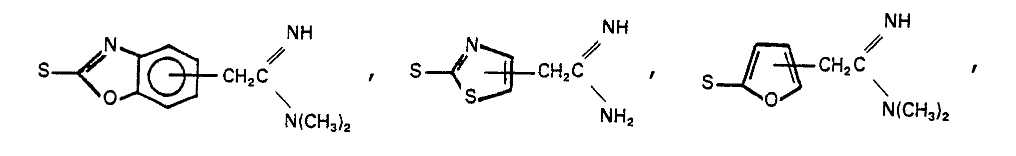 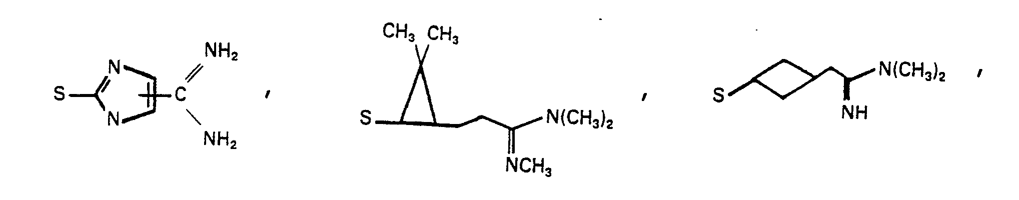 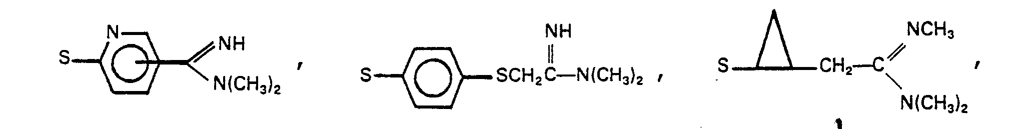 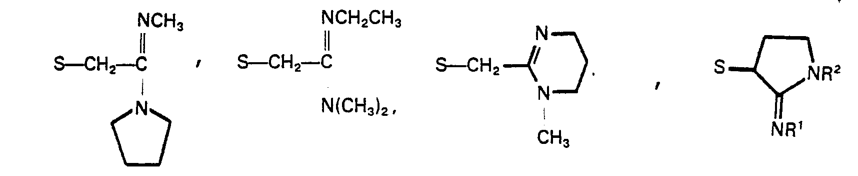 R' = H, CH₃ R² = H, CH₃ 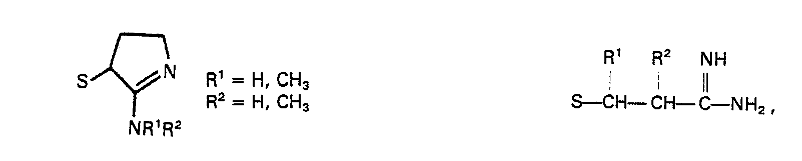 R¹ = CH₃ R² = CH₃, NR₂, OR R = H, CH₃ 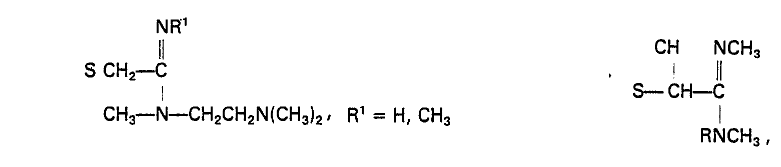 R=H,CH₃ 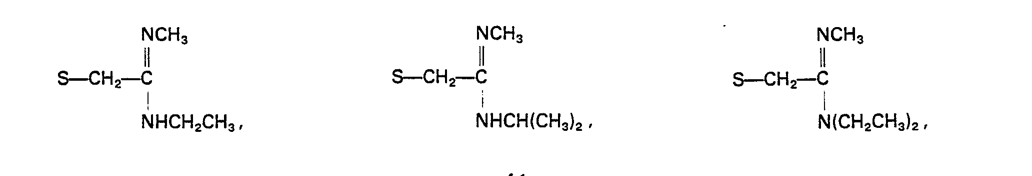 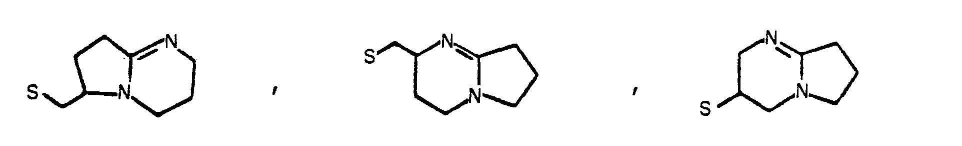 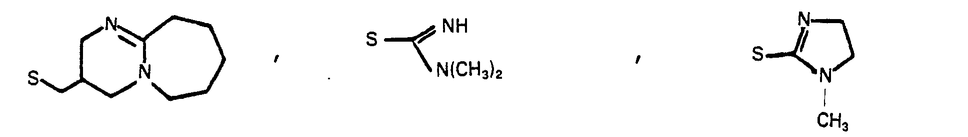 besteht.

3. Eine Verbindung nach Anspruch 1 oder 2, worin R⁷ aus H und CH₃ ausgewählt ist.

4. Eine Verbindung nach einem der Anspruche 1 bis 3, worin R⁶ aus der Gruppe ausgewählt ist, welche besteht aus: substituiertem und unsubstituiertem: Alkyl, Alkenyl und Cycloalkylalkyl, worin der Substituent oder die Substituenten aus Hydroxyl, Alkoxyl mit 1-6 Kohlenstoffatomen, Amino und Carboxy ausgewählt sind.

5. Eine Verbindung nach einem der Ansprüche 1 bis 4, worin R⁶ aus der Gruppe ausgewählt ist, die aus Alkyl, Cycloalkylalkyl, Alkyl, das durch eine oder mehrere Hydroxylgruppen substituiert ist, oder Cycloalkylalkyl, das durch eine oder mehrere Hydroxylgruppen substituiert ist, besteht.

6. Eine Verbindung nach einem der Ansprüche 1 bis 5, worin R⁷ Wasserstoff ist.

7. Eine Verbindung nach einem der Ansprüche 1 bis 6, worin R⁶ ausgewählt ist aus: Wasserstoff,

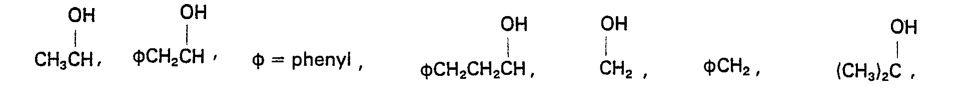

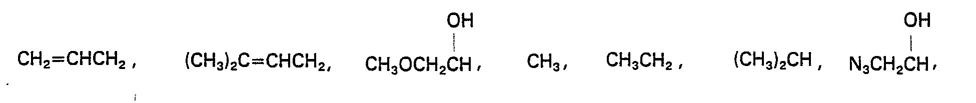

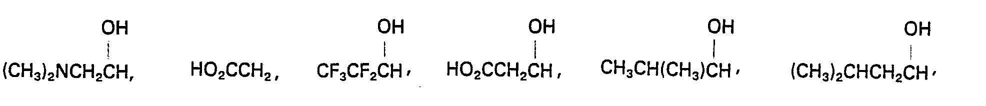

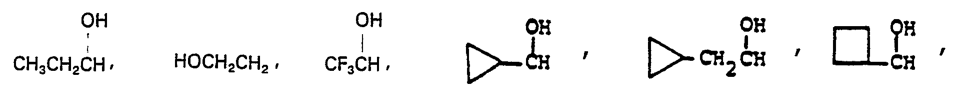

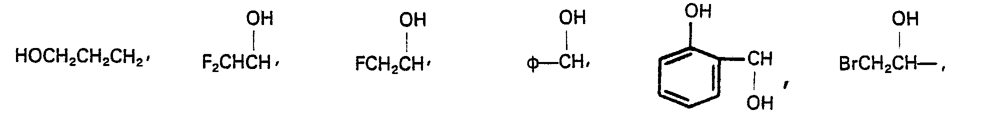

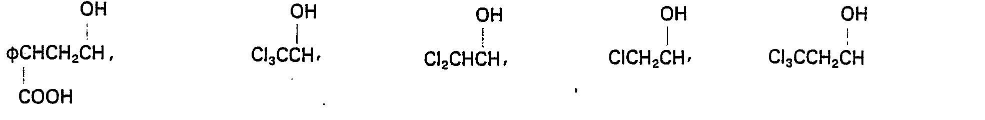

8. Eine Verbindung nach einem der Ansprüche 1 bis 7, worin R⁶ Wasserstoff,

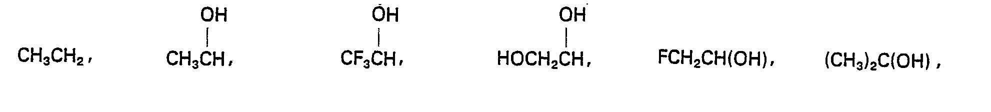

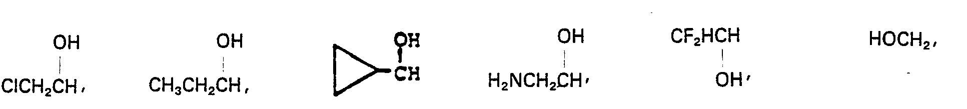

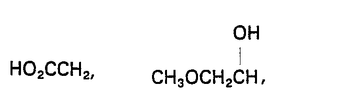

9. Eine Verbindung nach einem der Ansprüche 1 bis 8, worin R⁶ Wasserstoff; substituiertes oder unsubstituiertes: Alkyl, Cycloalkyl, Cycloalkylalkyl oder Phenylalkyl ist; wobei der Substituent oder die Substituenten aus: Hydroxyl, Chlor, Fluor, Brom, Carboxyl, Ureido, Amino, Alkoxy, oder Alkylthio ausgewählt ist bzw. sind.

10. Eine Verbindung nach einem der Ansprüche 1 bis 9, wobei R⁶ ausgewählt ist aus:

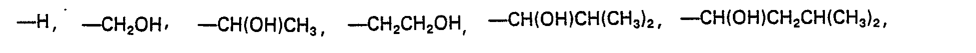

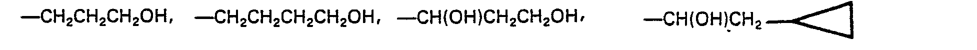

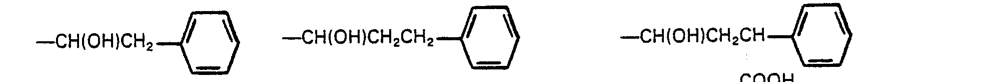

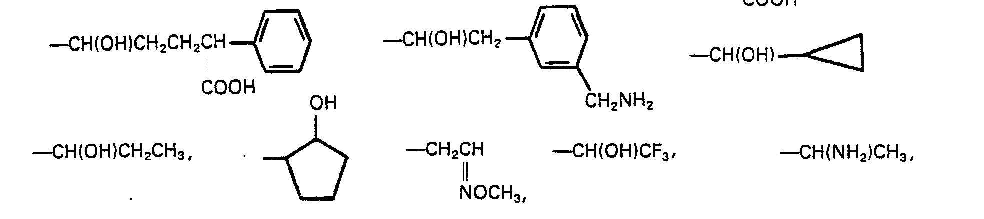

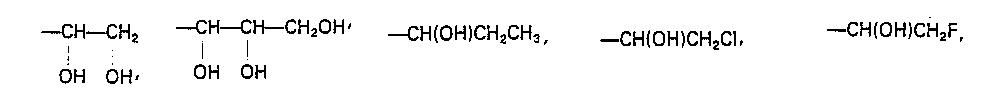

11. Ein Verfahren zur Herstellung einer Verbindung nach Anspruch 1, umfassend die Stufe des Behandelns von: 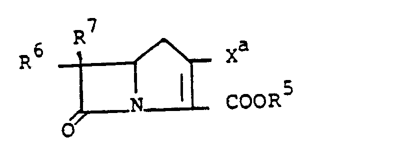 mit HSR⁸; worin X^{a} eine austretende Gruppe ist, und R⁵ eine Schutzgruppe ist.

12. Eine antibiotische Zusammensetzung, enthaltend eine therapeutisch wirksame Menge einer Verbindung nach Anspruch 1 und einen pharmazeutisch wirksamen Träger dafür.

13. Eine Verbindung nach Anspruch 2, worin R⁷ Wasserstoff ist, und R⁶

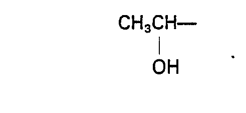

## Patentansprüche (Patentansprüche für folgende(n) Vertragsstaat(en) : AT)

1. Verfahren zur Herstellung einer Verbindung der Strukturformel: 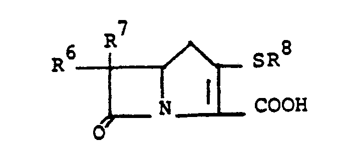 und der pharmazeutisch annehmbaren Salz-, Ester- und Amidderivate davon, wie sie auf dem Gebiet der β-Lactam-antibiotika bekannt sind, worin:
R⁶ und R' unabhängig voneinander aus der Gruppe ausgewählt sind, die aus Wasserstoff; substituiertem und unsubstituiertem: Alkyl, Alkenyl und Alkinyl mit 1-10 Kohlenstoffatomen; Cycloalkyl, Cycloalkylalkyl und Alkylcycloalkyl mit 3-6 Kohlenstoffatom in dem Cycloalkylring und 1-6 Kohlenstoffatomen in dem Alkylrest; Aryl, Aralkyl, Aralkyl und Aralkinyl, worin der Arylrest Phenyl ist und der aliphatische Abschnitt 1-6 Kohlenstoffatome hat, besteht; wobei R⁶ und R⁷ unter Bildung eines Cycloalkyls verbunden sein können, welches zusammen mit dem Kohlenstoffatom, an das sie gebunden sind, 3-6 Kohlenstoffatome aufweist; und wobei der Substituent oder die Substituenten an R⁶ und R⁷ unabhängig voneinander aus Chlor, Fluor, Hydroxy, Brom, Carboxyl, Cyano, Azido, Amino, Mono- und Dialkyl-amino (jedes Alkyl hat 1-6 Kohlenstoffatome), Ureido, Alkylthio mit 1-6 Kohlenstoffatomen und Alkoxyl mit 1-6 Kohlenstoffatomen ausgewählt sind; und R^{B} ein Carbamimidoyl ist, das aus der Gruppe ausgewählt sind; und R⁸ ein Carbamimidoyl ist, das aus der Gruppe ausgewählt ist, welche aus: 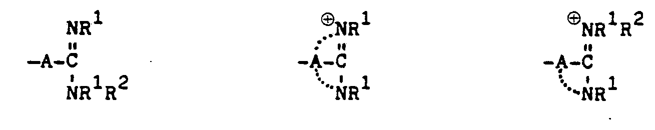 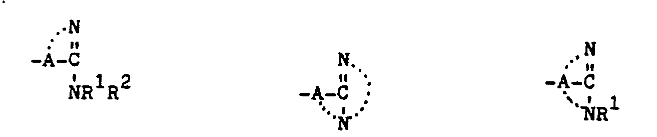 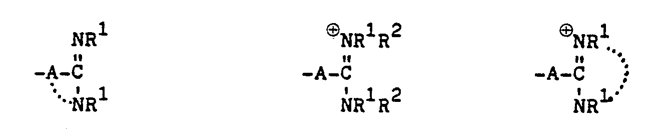 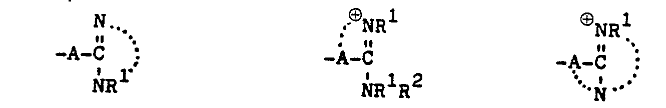 besteht, worin: A eine einfache, direkte Bindung mit der Ausnahme ist, daß A nicht eine einfache, direkte Bindung bei solchen Verbindungen darstellt, worin die Gruppe―C=N(NR¹R²) nicht quaternär ist; oder ein zweiwertiges, cyclisches oder acyclisches Verbindungsglied ist, das aus der Gruppe ausgewählt ist, welche aus Alkylen, Alkenylen und Alkinylen mit 1-10 Kohlenstoffatomen, die durch ein Heteroatom, ausgewählt aus O, S und N, oder durch Phenylen, Cycloalkylen, Cycloalkenylen mit 3-6 Kohlenstoffatomen, Heterocyclylen oder Heteroarylen unterbrochen sein können, wobei solche cyclischen Unterbrechungen 3-6 Ringatome, ausgewählt aus C, 0, S und N, umfassen; Cycloalkylen, Cycloalkenylen mit 3-6 Kohlenstoffatomen, Heterocyclylen oder Heteroarylen, von denen jedes 5-10 Ringatome und ein oder mehrere, aus O, N und S ausgewählte Heteroatome enthält; und Phenylen besteht;
R' und R² unabhängig voneinander aus Wasserstoff und den vorher definierten, jedoch einwertigen, Bedeutungen für die Gruppe A ausgewählt sind; und wobei die punktierten Linien Möglichkeiten für cyclische Strukturen angeben, die durch das Verbinden des angegebenen Stickstoffatoms und der Verbindungsgruppe A und durch das Verbinden der angegebenen Stickstoffatome gebildet werden, mit der Ausnahme von Verbindungen, worin (a) R⁸ 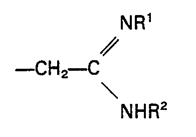 wobei R¹/R² H, H; CH₃, H sind; oder 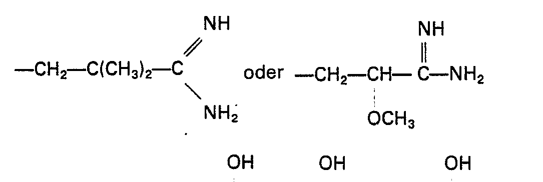 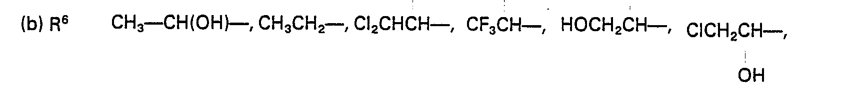 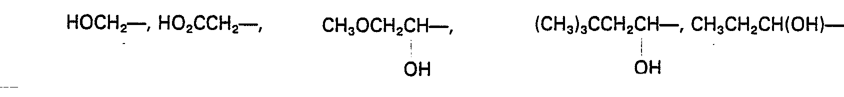 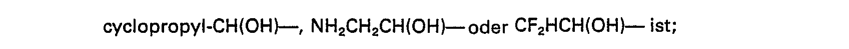 R⁷ H ist und 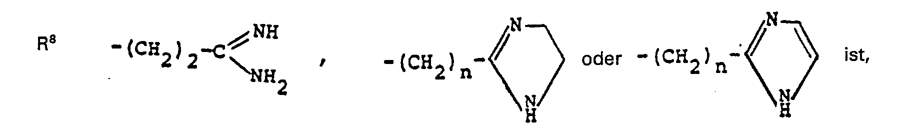 wobei n 1 oder 2 ist; und (c) R⁶ (CH₃)₂CH―CH(OH)― ist, R⁷ H ist und 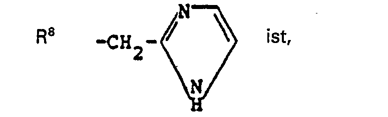 umfassend die Stufe des Behandelns von: 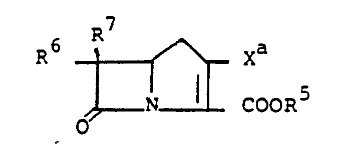 mit HSR⁸; worin X^{a} eine austretende Gruppe ist, und R⁵ eine Schutzgruppe ist.

2. Das Verfahren nach Anspruch 1, wobei ―SR⁸ aus der Gruppe ausgewählt ist, die aus: 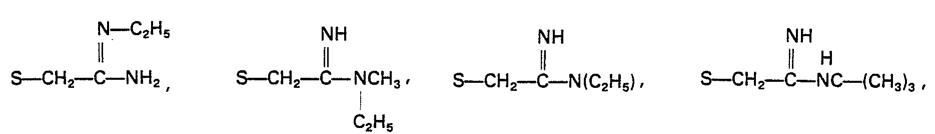 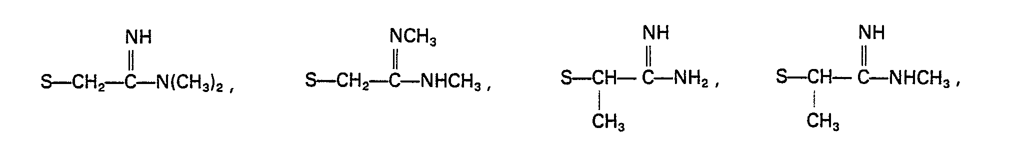 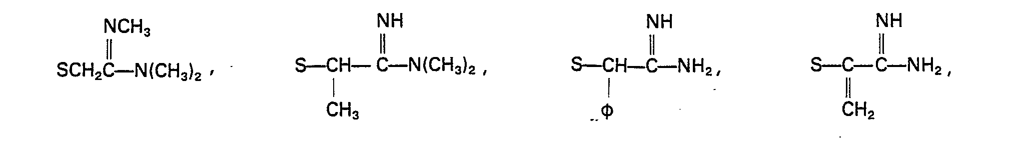 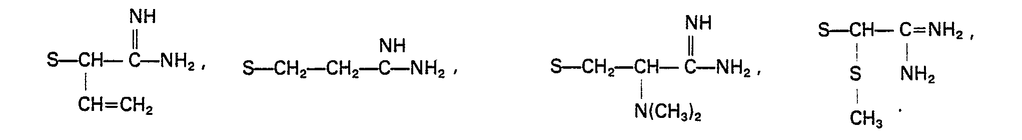 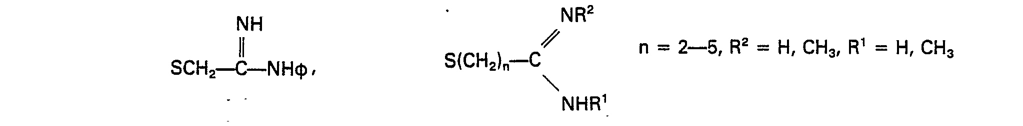 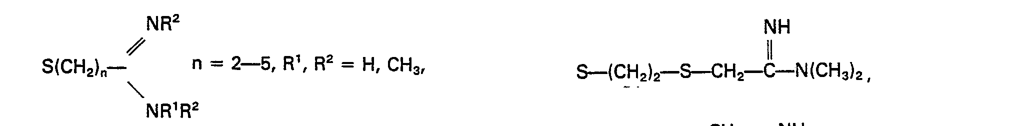 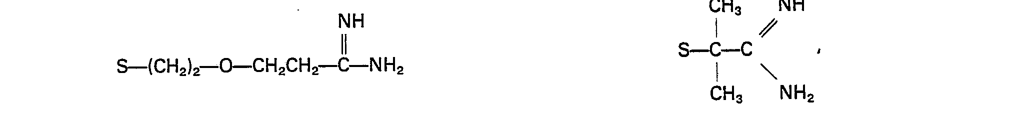 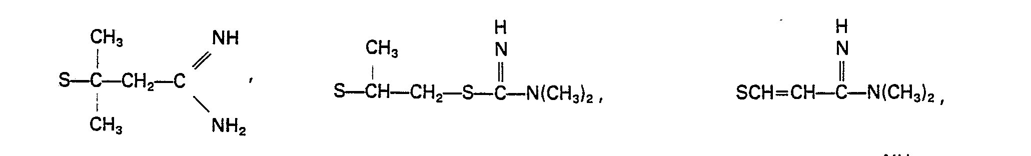 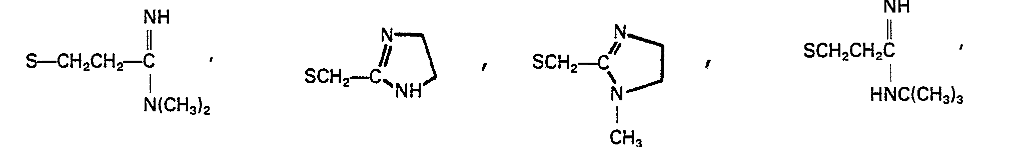 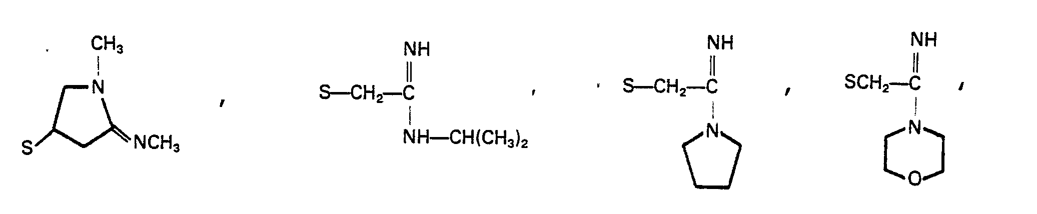 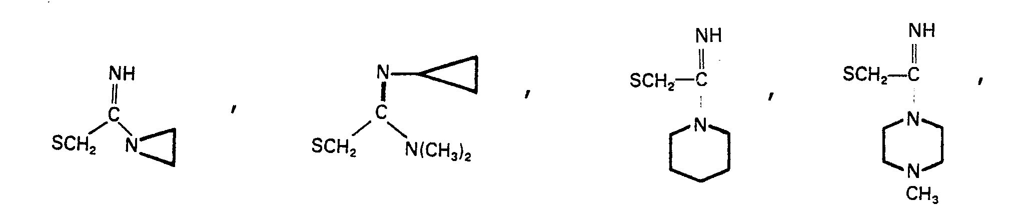 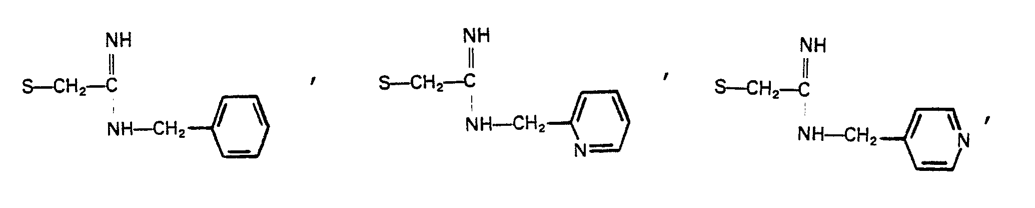 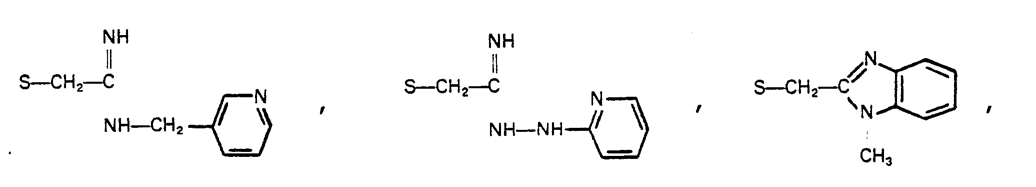 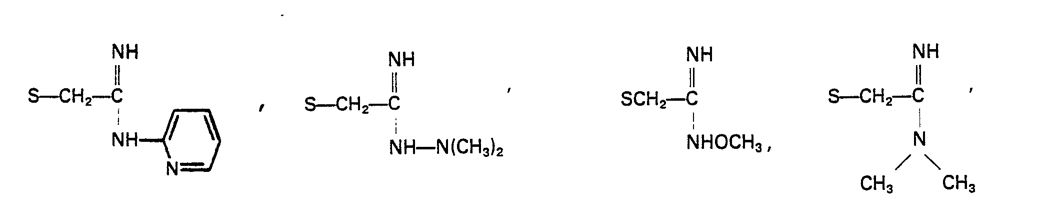 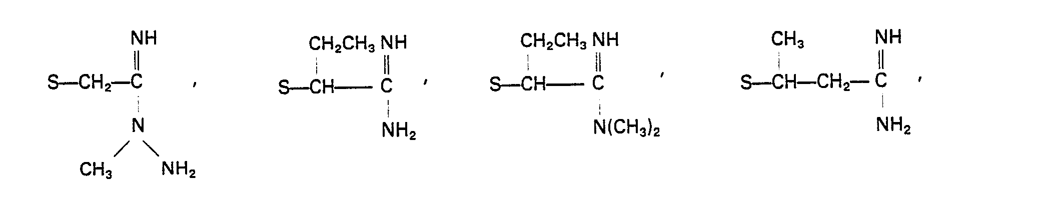 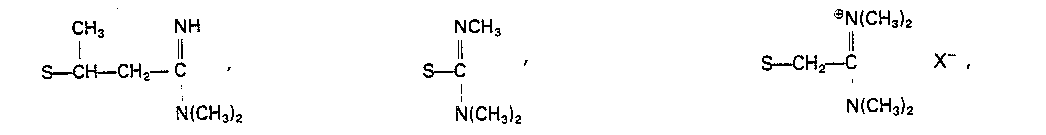 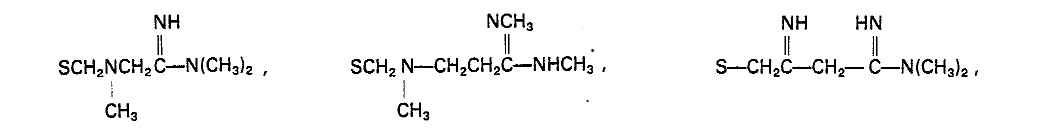 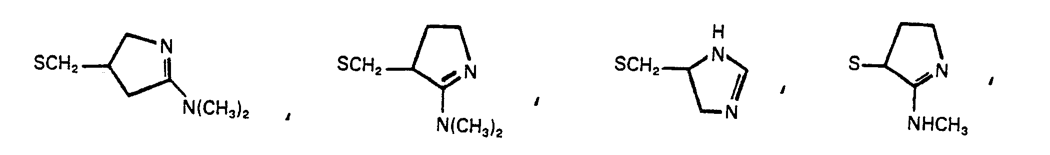 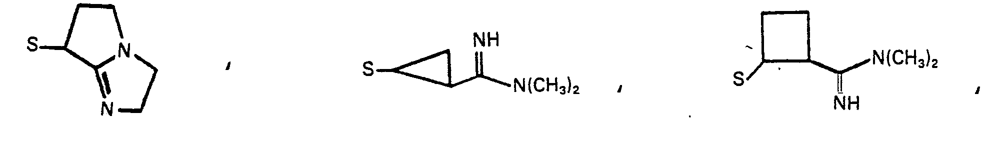 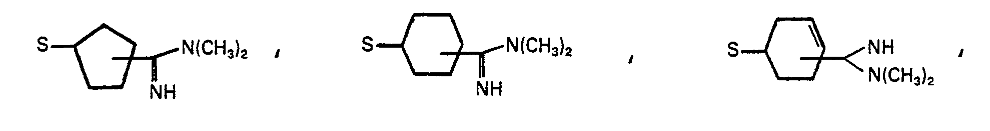 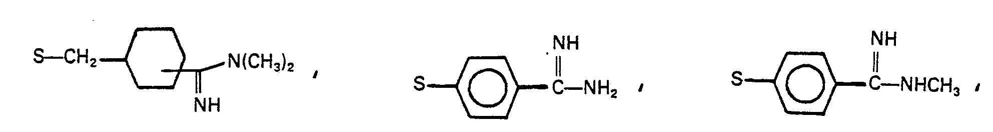 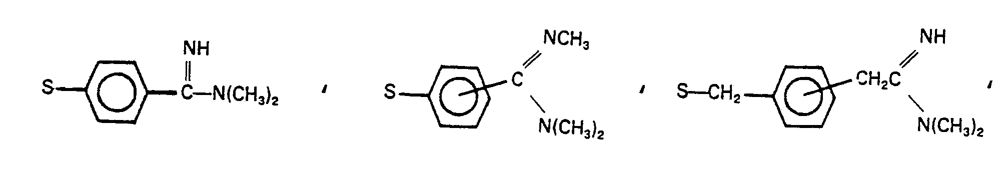 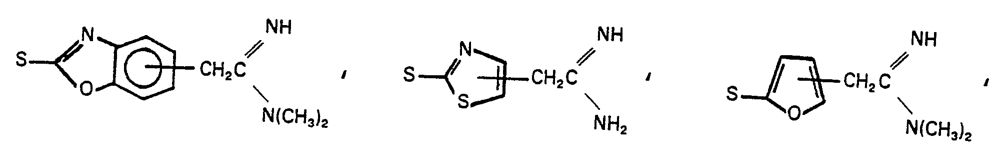 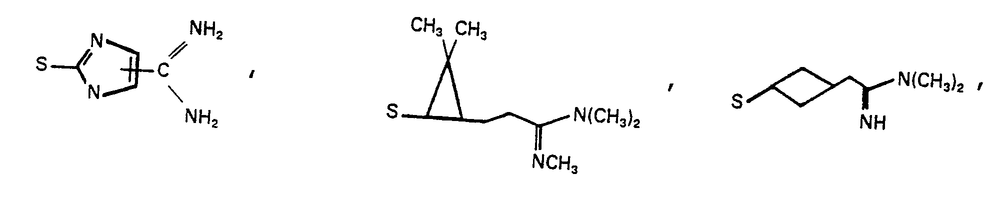 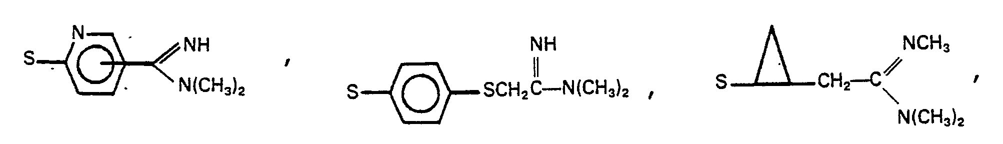 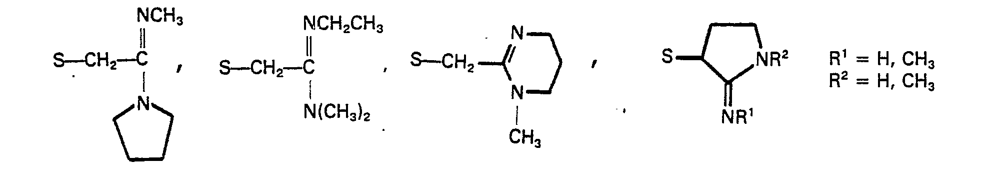 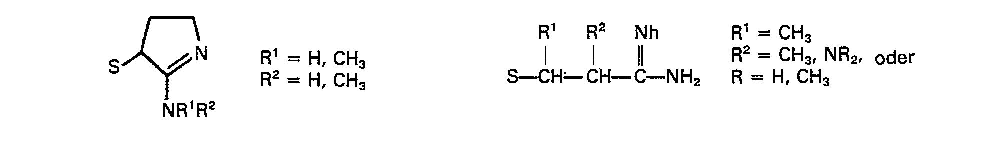 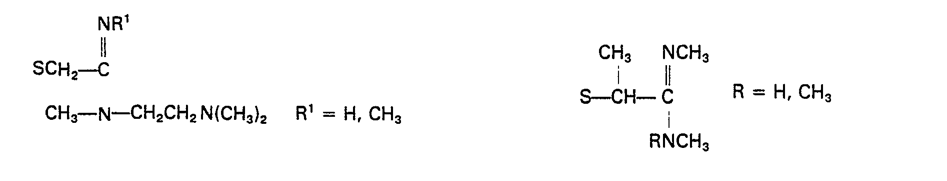 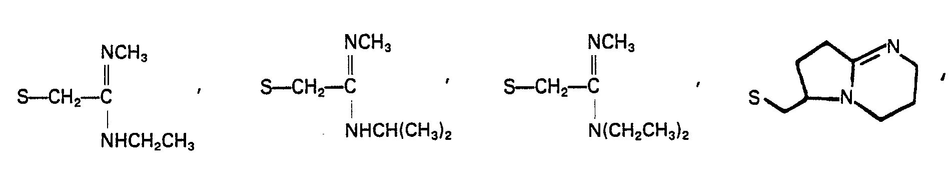 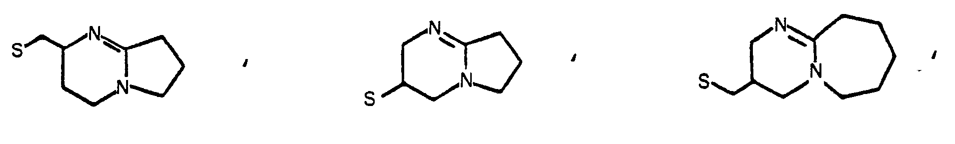 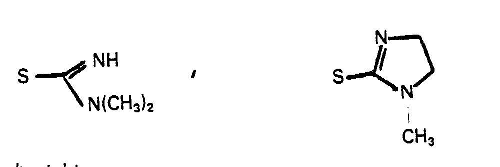 Destent.

3. Das Verfahren nach Anspruch 1 oder 2, worin R⁷ aus H und CH₃ ausgewählt ist.

4. Das Verfahren nach einem der Ansprüche 1 bis 3, worin R⁶ aus der Gruppe ausgewählt ist, welche besteht aus: substituiertem und unsubstituiertem: Alkyl, Alkenyl und Cycloalkylalkyl, worin der Substituent oder die Substituenten aus Hydroxyl, Alkoxyl mit 1-6 Kohlenstoffatomen, Amino und Carboxy ausgewählt sind.

5. Das Verfahren nach einem der Ansprüche 1 bis 4, worin R⁶ aus der Gruppe ausgewählt ist, die aus Alkyl, Cycloalkylalkyl, Alkyl, das durch eine oder mehrere hydroxylgruppen substituert ist, oder Cycloalkylalkyl, das durch eine oder mehrere Hydroxylgruppen substituiert ist, besteht.

6. Das Verfahren nach einem der Ansprüche 1 bis 5, worin R⁷ Wasserstoff ist.

7. Das Verfahren nach einem der Ansprüche 1 bis 6, worin R⁶ ausgewählt ist aus: Wasserstoff,

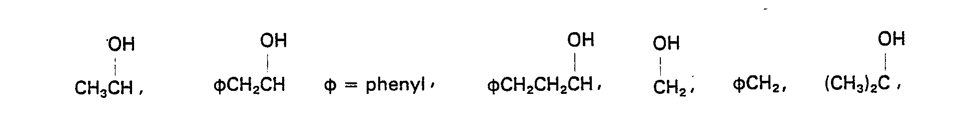

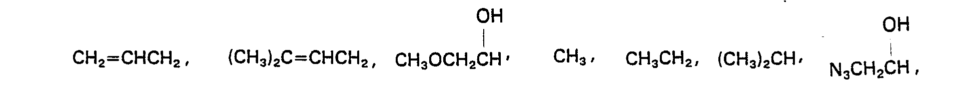

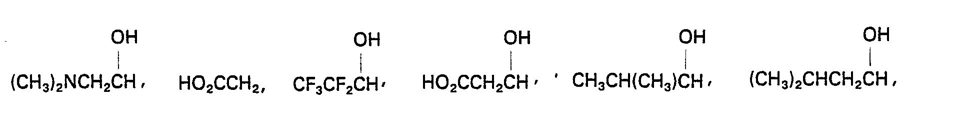

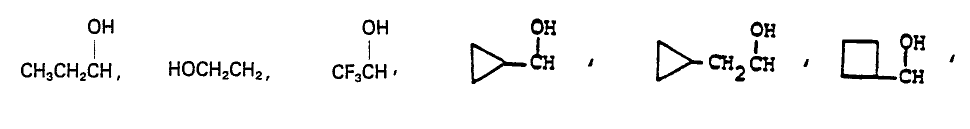

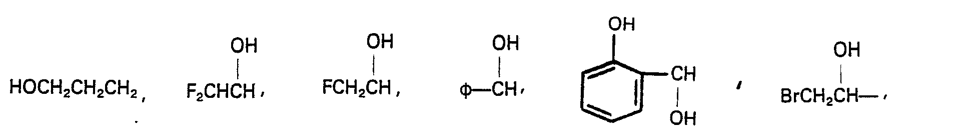

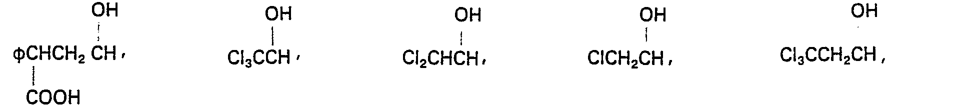

8. Das Verfahren nach einem der Ansprüche 1 bis 7, worin R⁶ Wasserstoff.

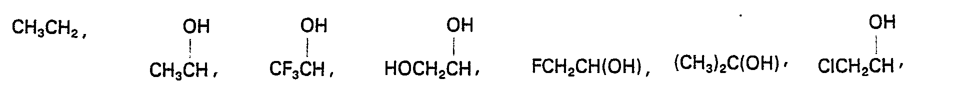

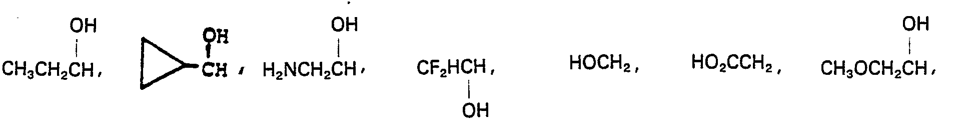

9. Das Verfahren nach einem der Ansprüche 1 bis 8, worin R⁶ Wasserstoff; substituiertes oder unsubstituiertes: Alkyl, Cycloalkyl, Cycloalkylalkyl oder Phenylalkyl ist; wobei der Substituent oder die Substituenten aus: Hydroxyl, Chlor, Fluor, Brom, Carboxyl, Ureido, Amino, Alkoxyl oder Alkylthio ausgewählt ist bzw. sind.

10. Das Verfahren nach einem der Ansprüche 1 bis 9, wobei R⁶ ausgewählt ist aus:

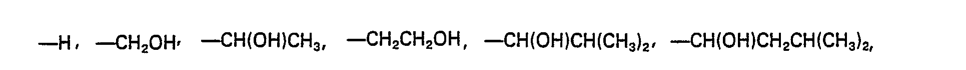

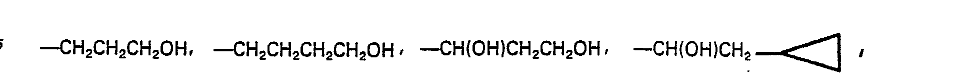

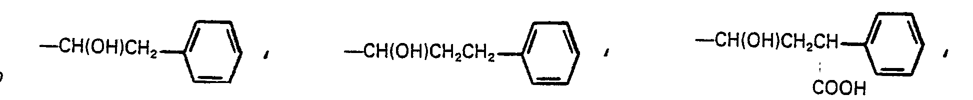

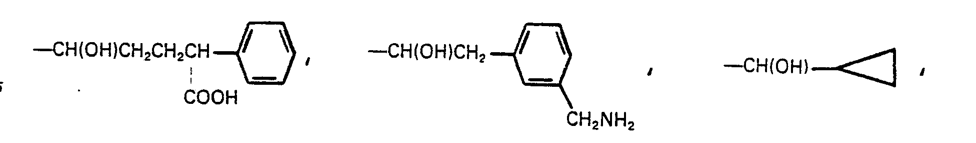

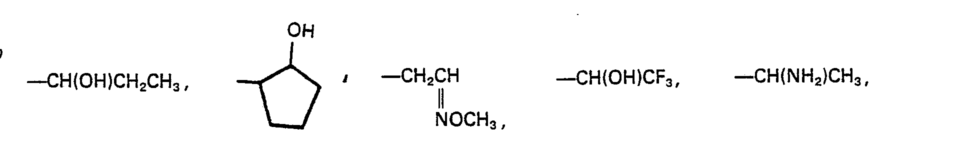

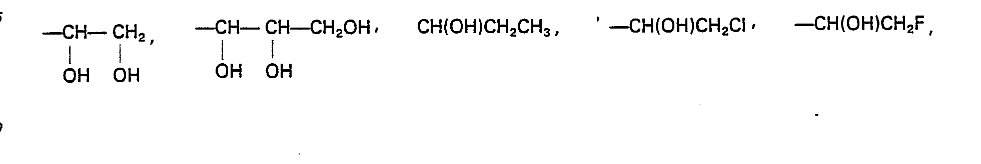

## Revendications (Revendications pour l'(les) Etat(s) contractant(s) suivant(s) : BE CH DE FR GB IT LI LU NL SE)

1. Composé de formule développée: 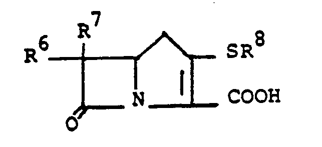 et ses dérivés sels, esters et amides pharmaceutiquement acceptables connus dans la technique des antibiotiques à base de (β-lactame, dans lequel R⁶ et R⁷ sont choisis indépendamment dans le groupe constitué par un hydrogène; les groupes, substitués et non substitués; alkyle, alcényle et alcynyle ayant 1-10 atomes de carbone; cycloalkyle, cycloalkylalkyle et alkylcycloalkyle ayant 3-6 atomes de carbone dans le noyau cycloalkyle et 1―6 atomes de carbone dans le groupement alkyle; aryle, arylalkyle, arylacényle et arylalcynyle dans lequel les groupement aryle est un phényle et la partie aliphatique a 1-6 atomes de carbone; R⁶ et R⁷ peuvent être reliés pour former un groupe cycloalkyle ayant, avec l'atome de carbone auquel ils sont fixés, 3-6 atomes de carbone; dans lequel le ou les substituants de R⁶ et de R⁷ sont choisis indépendamment parmi les groupes chloro, fluoro, hydroxy, bromo, carboxyle, cyano, azido, amino, mono- et dialkylamino (chaque groupe alkyle ayant 1-6 atomes de carbone), uréido, alkylthio, ayant 1-6 atomes de carbone, et alcoxyle ayant 1-6 atomes de carbone; et R est un groupe carbamimidoyle choisi dans le groupe constitué par 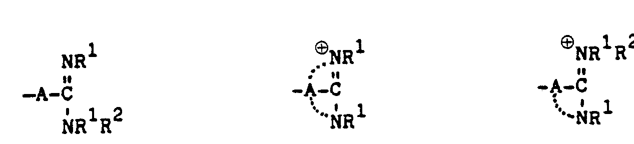 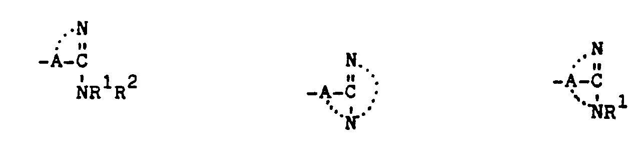 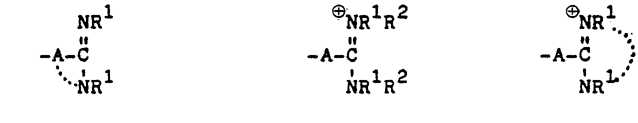 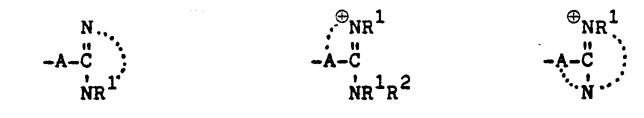 dans lesquels A est un liaison simple directe, sauf que A n'est pas une liaison simple directe avec les composés dans lesquels le groupe -C=N(NR'R²) n'est pas quaternaire, ou est un groupe de jonction divalent, cyclique ou acylique, choisi dans le groupe constitué par un alkylène, un alcénylène et un alcynylène ayant 1-10 atomes de carbone que peut être interrompu par un hétéroatome choisi parmi 0, S ou N, ou par un groupe phénylène, cycloalkylène, cycloalcénylène ayant 3―6 atomes de carbone; hétérocyclylène ou hétéroarylène où ces interruptions cycliques comprennent 3-6 atomes de noyau choisis parmi C, O, S et N; cycloalkylène, cycloalcénylène ayant 3-6 atomes de carbone; hétérocyclylène ou hétéroarylène ayant chacun 5-10 atomes dans le noyau et un ou plusieurs hétéroatomes choisis parmi, 0, N et S; et phénylène; R' et R² sont choisis indépendamment parmi un hydrogène et les définitions indiquées précédemment pour le groupe A, mais monovalentes; et dans lesquels les lignes en pointillés indiquent la place prévue pour des structures cycliques formées par la jonction de l'atome d'azote indiqué et du groupe de jonction A et par la jonction des atomes d'azote indiqués, à l'exception des composés dans lesquels 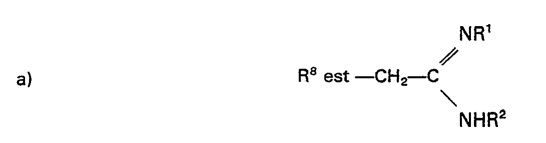 où R¹/R² sont H, H; CH₃, H; ou 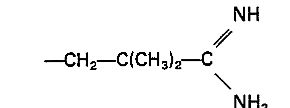 ou 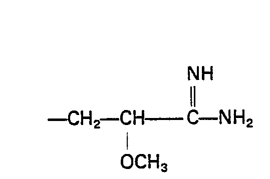 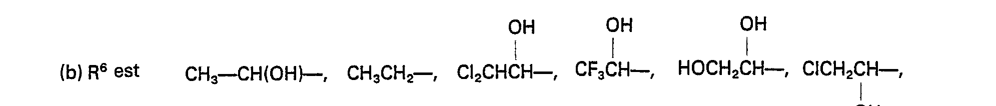 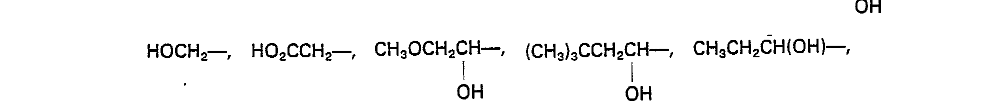 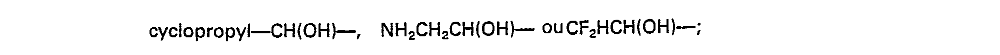 R⁷ est H et 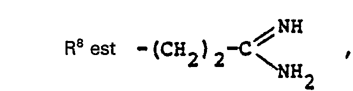 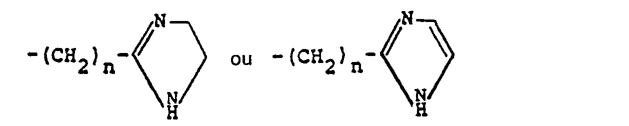 où n est égal à 1 ou 2 et (c) R⁶ est (CH₃)₂CH―CH(OH)― R⁷ est H et

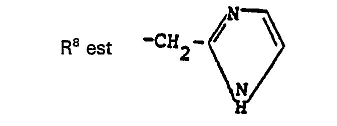

2. Composé selon la revendication 1, dans lequel ―SR⁸ est choisi dans le groupe constitué par:

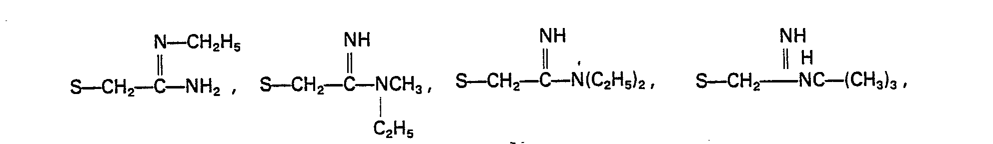

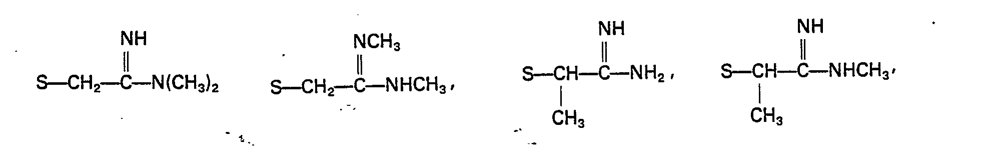

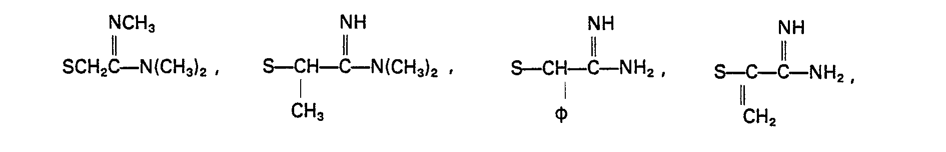

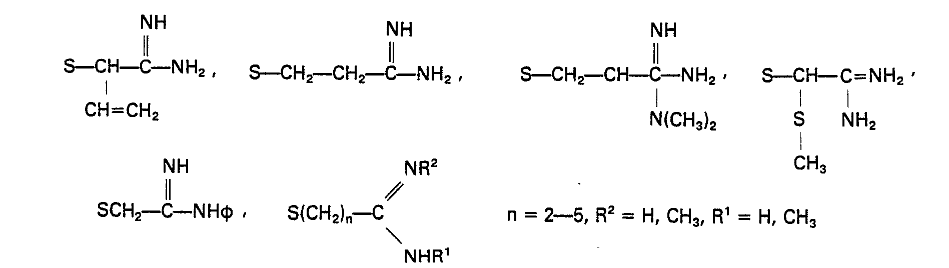

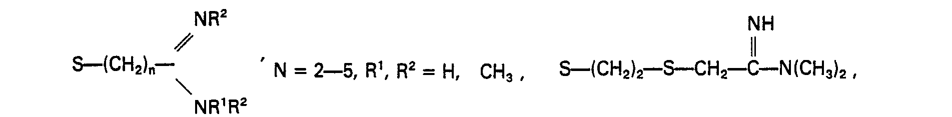

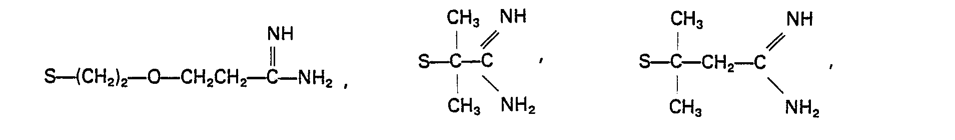

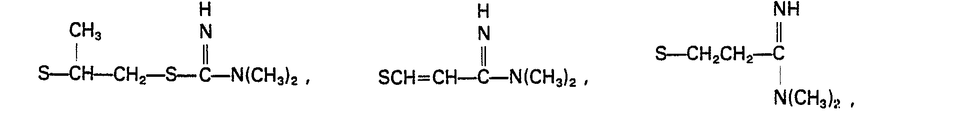

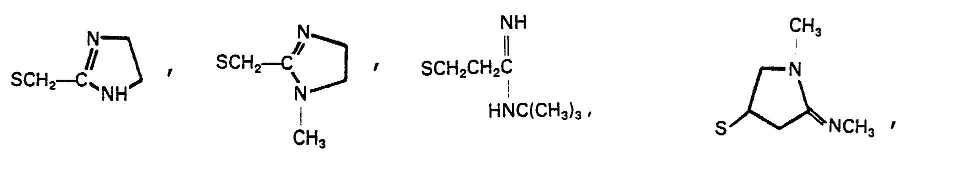

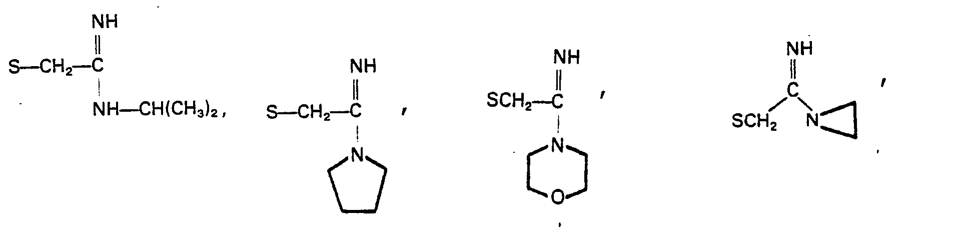

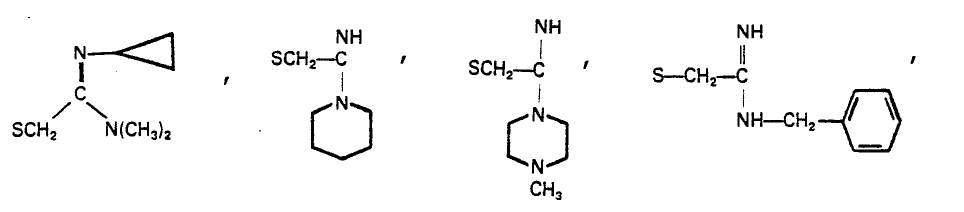

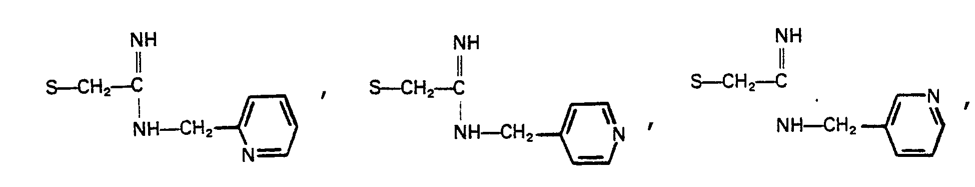

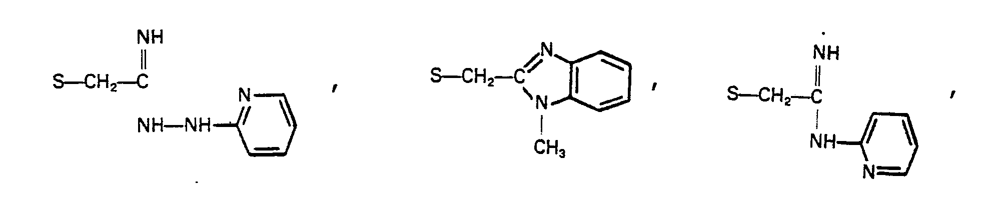

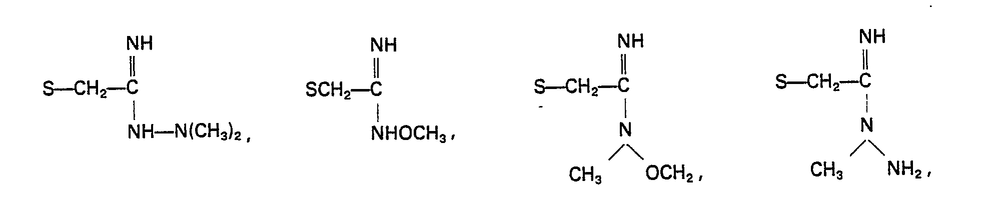

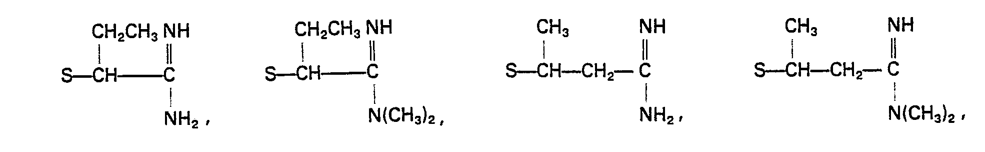

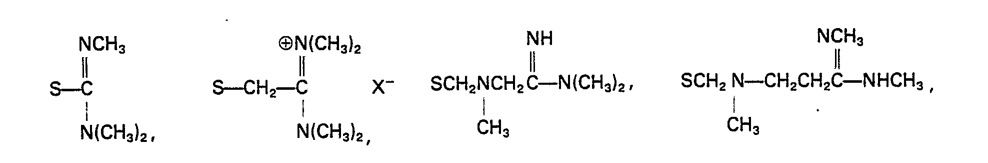

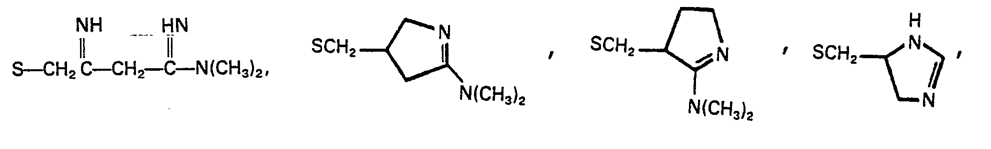

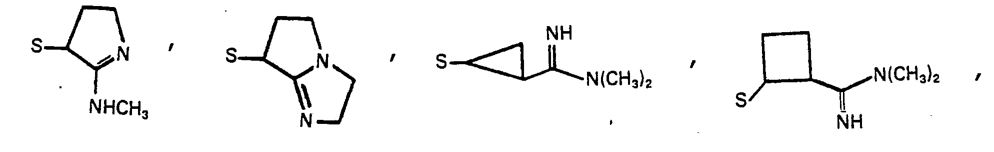

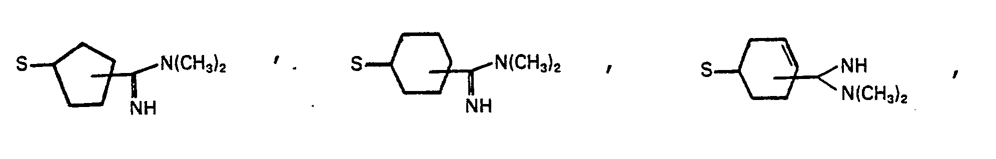

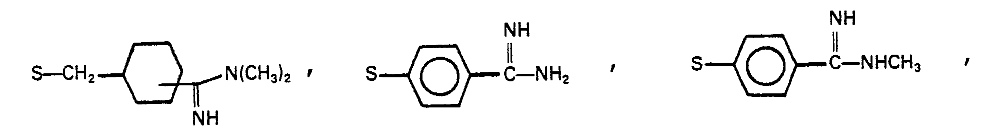

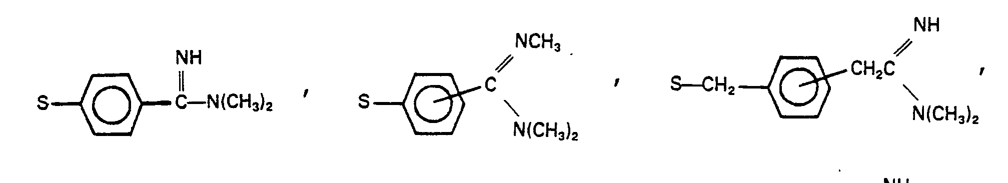

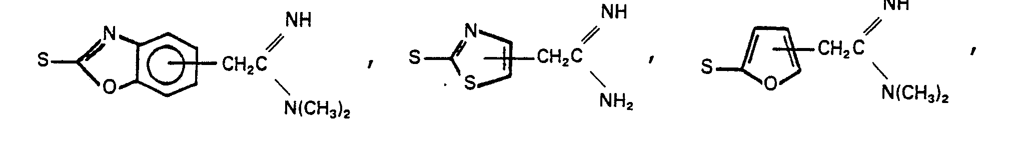

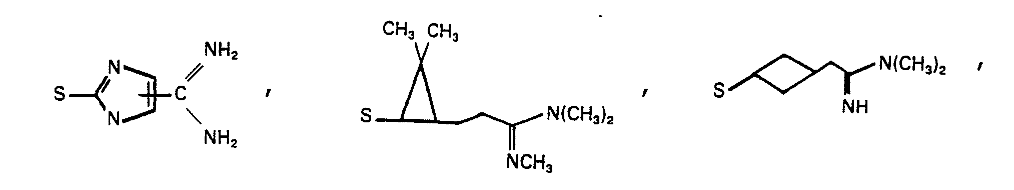

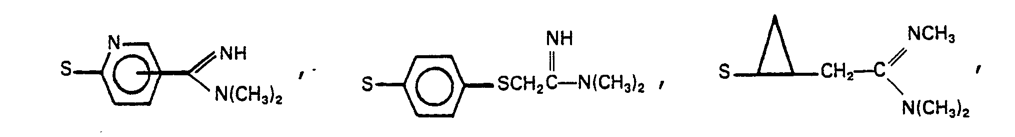

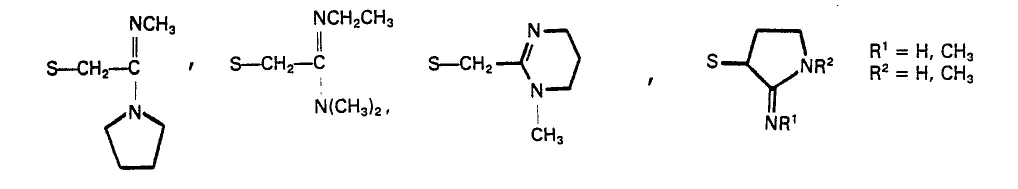

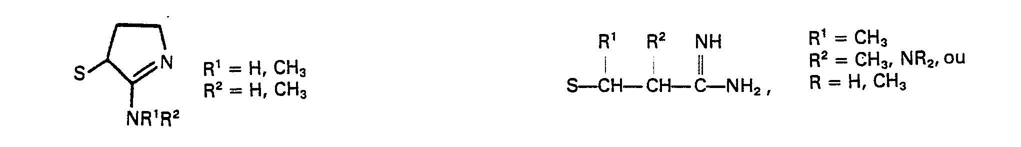

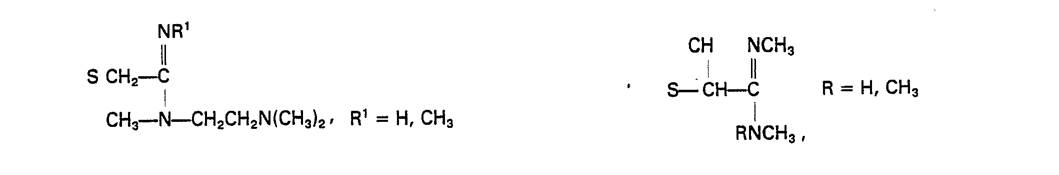

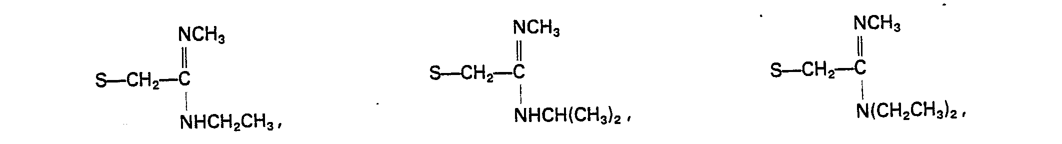

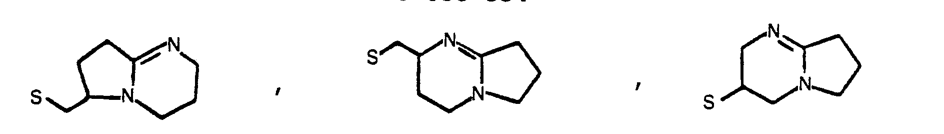

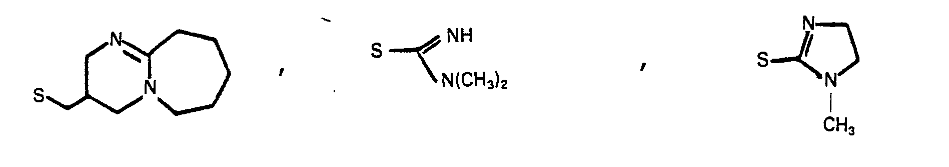

3. Composé selon la revendication 1 ou 2, dans lequel R⁷ est choisi parmi H et CH₃.

4. Composé selon les revendications 1 à 3, dans lequel R⁶ est choisi dans le groupe constitué par les groupes, substitués ou non substitués: alkyle, alcényle et cycloalkylalkyle dans lesquels le ou les substituants sont choisis parmi les groupes hydroxyle, alcoxyle ayant 1 à 6 atomes de carbone, amino et carboxy.

5. Composé selon les revendications 1 à 4, dans lequel R⁶ est choisi dans le groupe constitué par les groupes alkyle, cycloalkylalkyle, alkyle substitué par un ou plusieurs groupes hydroxyle, ou cycloalkylalkyle substitué par un ou plusieurs groupes hydroxyle.

6. Composé selon les revendications 1 à 5, dans lequel R⁷ est un hydrogène.

7. Choisi selon les revendications 1 à 6, dans lequel R⁶ est choisi parmi: un hydrogène,

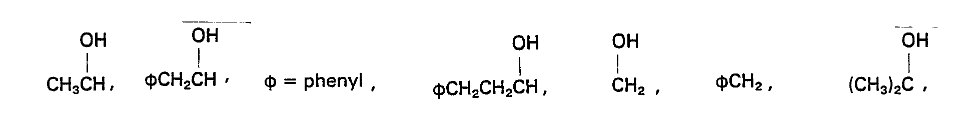

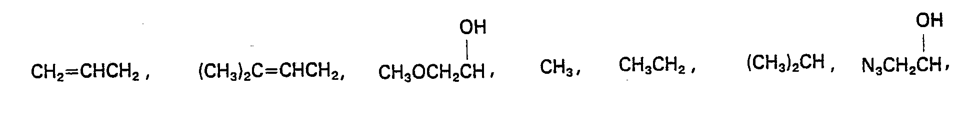

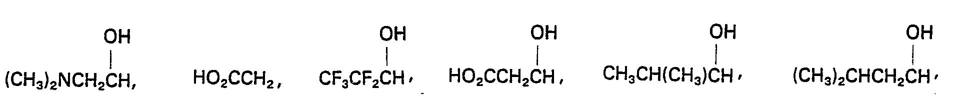

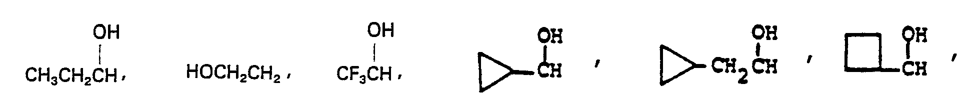

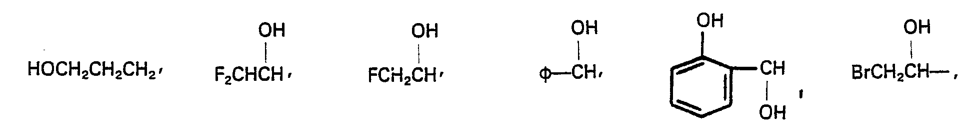

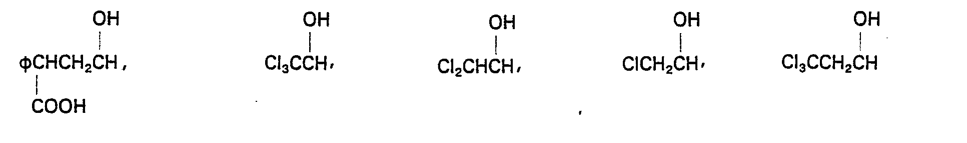

8. Composé selon les revendications 1 à 7, dans lequel R⁶ est un hydrogène,

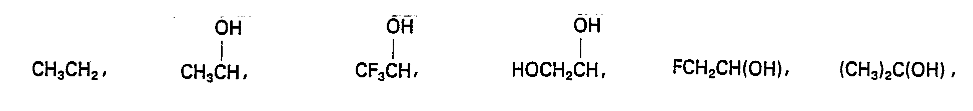

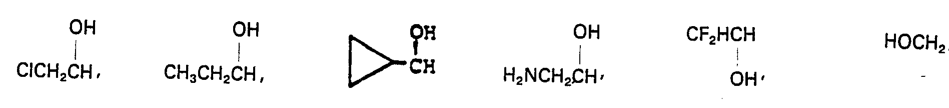

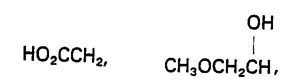

9. Composé selon les revendications 1 à 8, dans lequel R⁶ est un hydrogène; les groupes, substitués ou non substitués: alkyle, cycloalkyle, cycloalkylalkyle, phénylalkyle; dans lequel le ou les substituants sont choisis parmi les groupes: hydroxyle, chloro, fluoro, bromo, carboxyle, uréido, amino, alcoxyle ou alkylthio.

10. Composé selon les revendications 1 à 9, dans lequel R⁶ est choisi parmi:

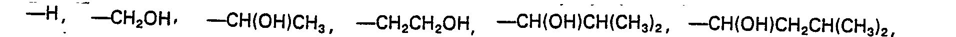

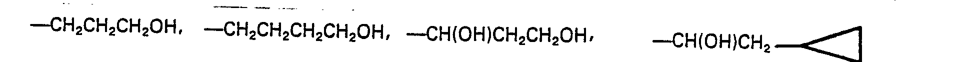

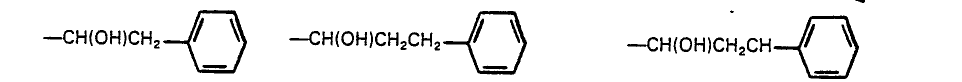

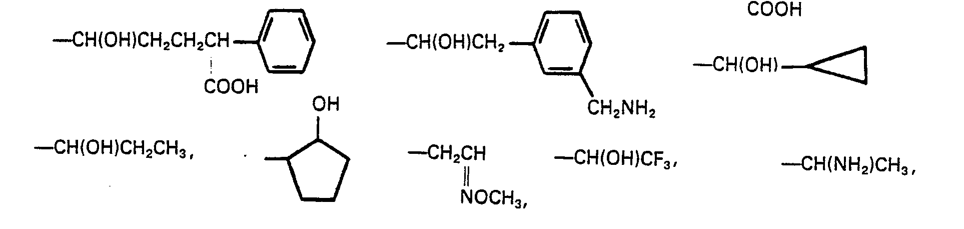

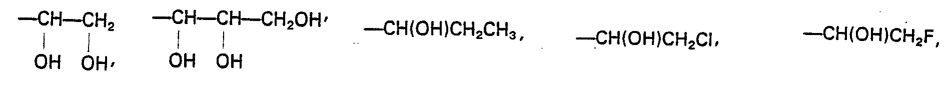

11. Procédé de préparation d'un composé selon la revendication 1, comprenant les étapes qui consistent à traiter: 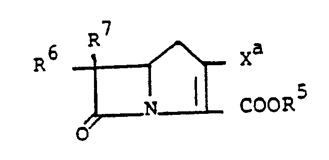 avec HSR⁸; dans lequel X^{a} est un groupe partant et R⁵ est un groupe protecteur.

12. Composition antibiotique comprenant une quantité thérapeutiquement efficace d'une composé selon la revendication 1 et un véhicule pharmaceutiquement efficace pour celui-ci.

13. Composé selon la revendication 2, dans lequel R⁷ est un hydrogène et R⁶ est

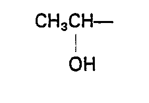

## Revendications (Revendications pour l'(les) Etat(s) contractant(s) suivant(s) : AT)

1. Procédé de préparation d'un composé de formule développée: 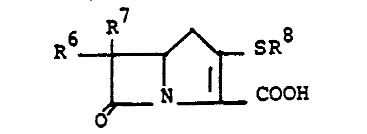 et de ses dérivés sels, esters et amides Pharmaceutiquement acceptables connus dans la technique des antibiotiques à base de β-lactame, dans laquelle R⁶ et R⁷ sont choisis indépendamment dans le groupe constitué par un hydrogène; les groupes, substitués et non substitués: alkyle, alcényle et alcynyle ayant 1-10 atomes de carbone; cycloalkyle, cycloalkylalkyle et alkylcycloalkyle ayant 3-6 atomes de carbone dans le noyau cycloalkyle et 1-6 atomes de carbone dans le groupement alkyle; aryle, arylalkyle; arylalcényle et arylalcynyle dans lequel le groupement aryle est un phényle et la partie aliphatique a 1-6 atomes de carbone; R⁶ et R⁷ peuvent être reliés pour former un groupe cycloalkyle ayant, avec l'atome de carbone auquel ils sont fixés, 3-6 atomes de carbone; dans lequel le ou les substituants de R⁶ et de R⁷ sont choiis indépendamment parmi les groupes chloro, fluoro, hydroxy, bromo, carboxyle, cyano, azido, amino, mono- et dialkylamino (chaque groupe alkyle ayant 1-6 atomes de carbone), uréido, alkylthio ayant 1-6 - atomes de carbone, et alcoxyle ayant 1-6 atomes de carbone; et R^{B} est un groupe carbamimidoyle choisi dans le groupe constitué par 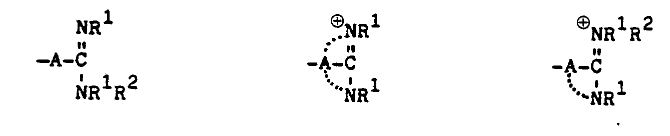 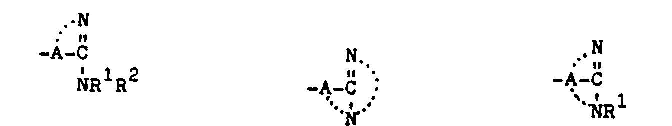 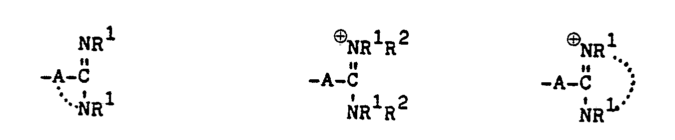 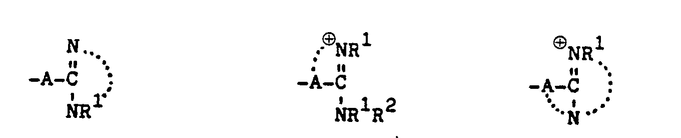 dans lesquels A est une liaison simple directe, sauf que A n'est pas une liaison simple directe avec les composés dans lesquels le groupe ―C=N(NR¹R²) n'est pas quaternaire, ou est un groupe de jonction divalent, cyclique ou acyclique, choisi dans le groupe constitué par un alkylène, un alcénylène et un alcynylène ayant 1-10 atomes de carbone qui peut être interrompu par un hétéroatome choisi parmi 0, S ou N, ou par un groupe phénylène, cycloalkylène, cycloalcénylène ayant 3-6 atomes de carbone; hétérocyclylène ou hétéroarylène où ces interruptions cycliques comprennent 3-6 atomes de noyau choisis parmi C, 0, S et N; cycloalkylène, cycloalcénylène ayant 3-6 atomes de carbone; hétérocyclylène ou hétéroarylène ayant chacun 5-10 atomes dans le noyau et un ou plusieurs hétéroatomes choisis parmi O, N, et S; et phénylène; R' et R² sont choisis indépendamment parmi un hydrogène et les définitions indiquées précédemment pour le groupe A, mais monovalentes; et dans lesquels les lignes en pointillés indiquent la place prévu pour des structures cycliques formées par la jonction de l'atome d'azote indiqué et du groupe de jonction A et par la jonction des atomes d'azote indiqués, à l'exception des composés dans lesauels 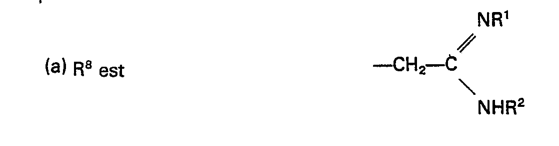 où R¹/R² sont H, H; CH₃, H; ou 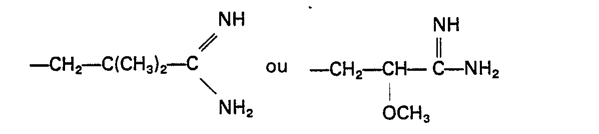 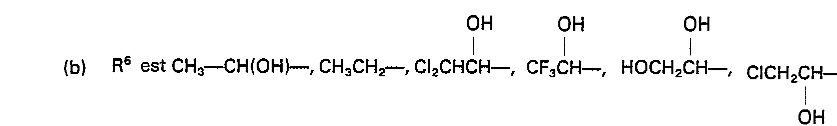 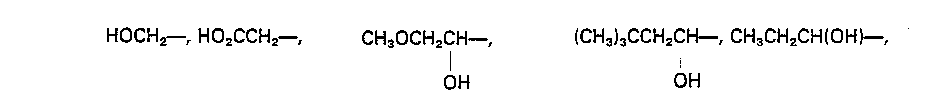 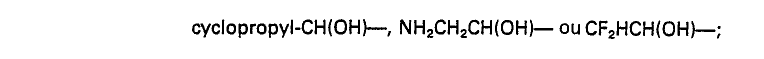 R⁷ est H et 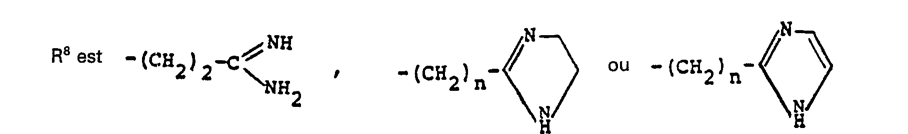 où n est égal à 1 ou 2 et (c) R⁶ est (CH₃)₂CH―CH(OH)― R⁷ est H et 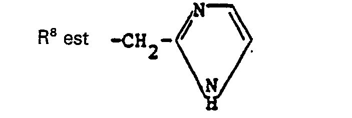 comprenant les étapes qui consistent à traiter 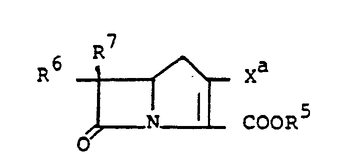 avec HSR⁸; dans laquelle X^{a} est un groupe partant et R⁵ est un groupe protecteur.

2. Procédé selon la revendication 1, dans lequel ―SR⁸ est choisi dans le groupe constitué par: 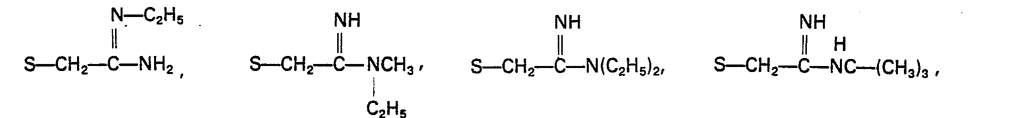 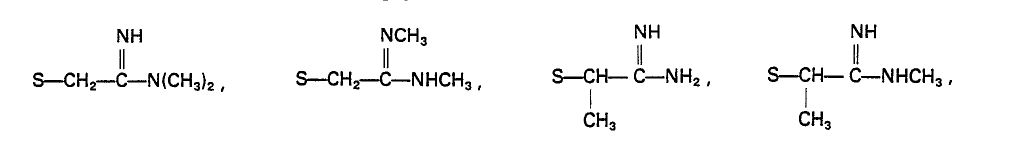 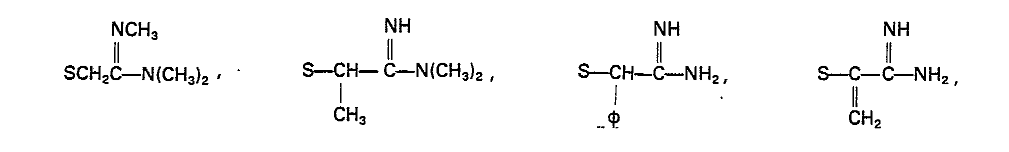 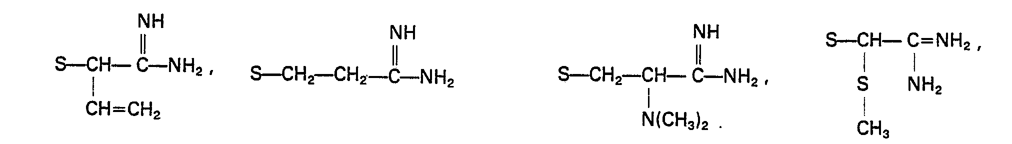 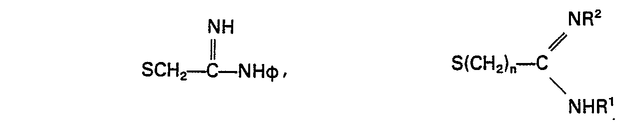 n = 2―5, R² = H, CH₃, R¹ = H, CH₃

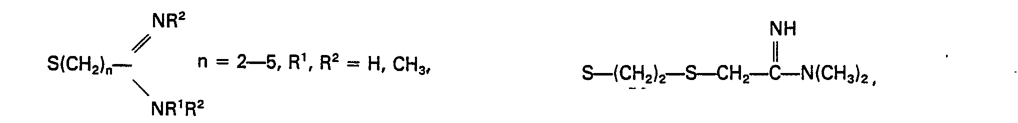

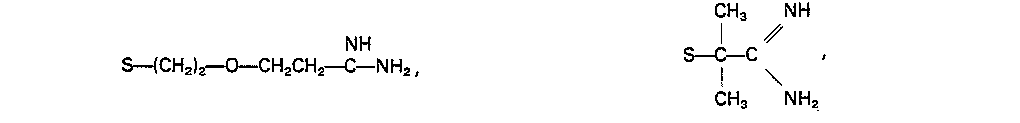

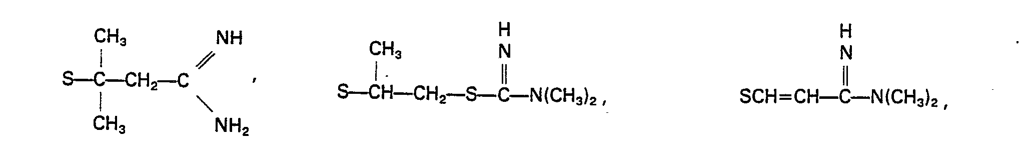

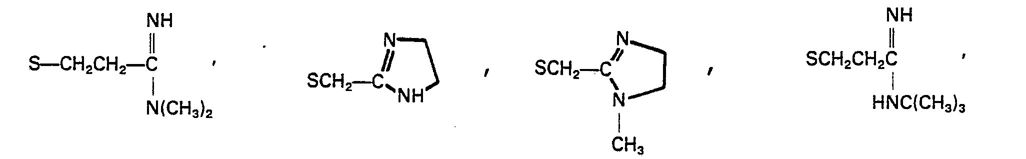

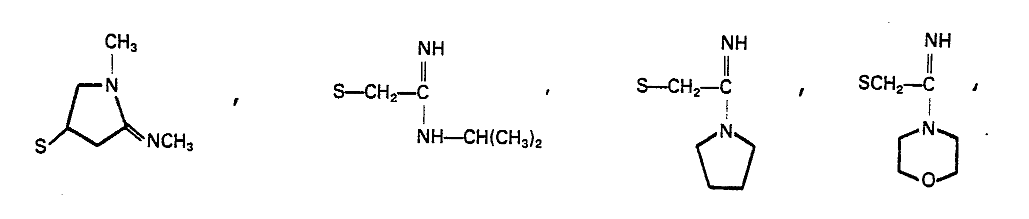

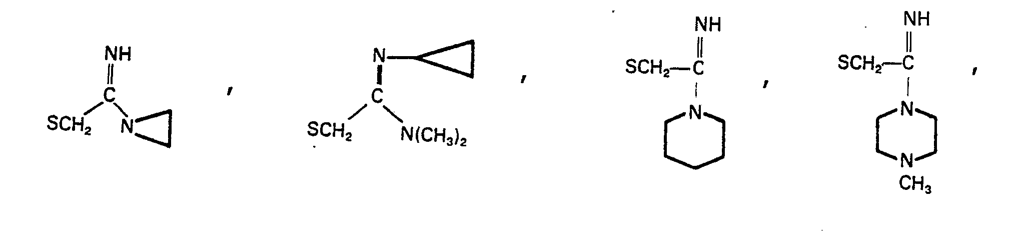

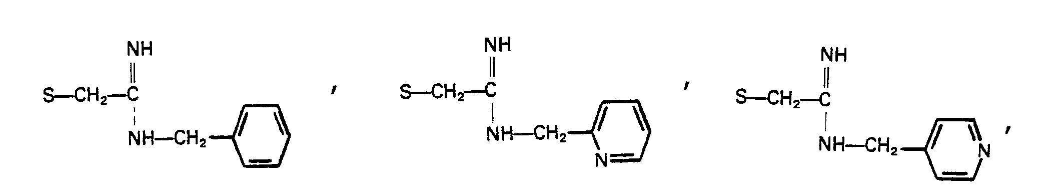

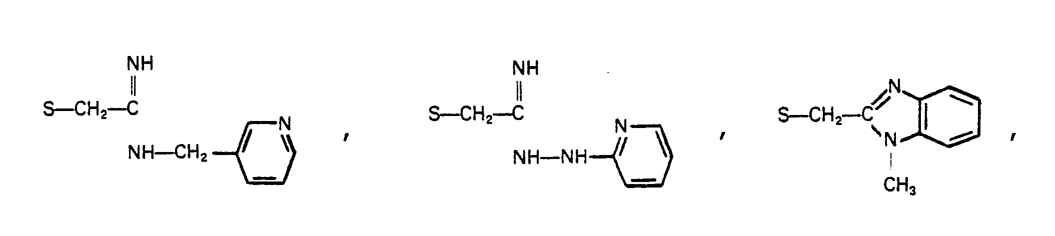

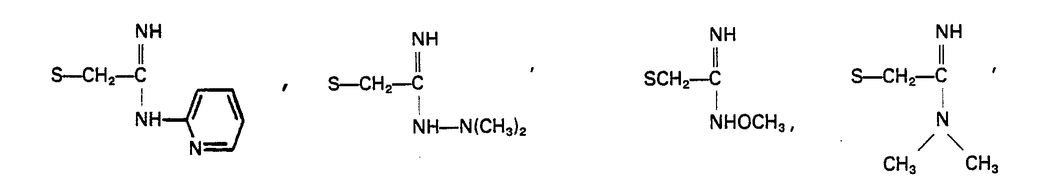

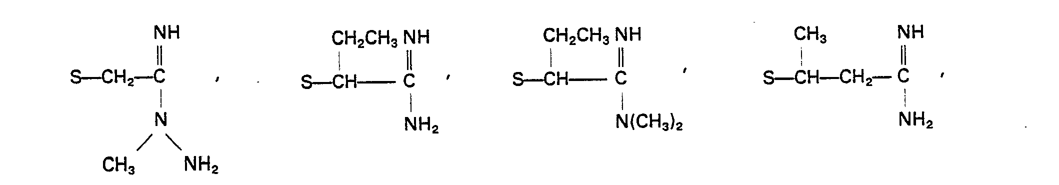

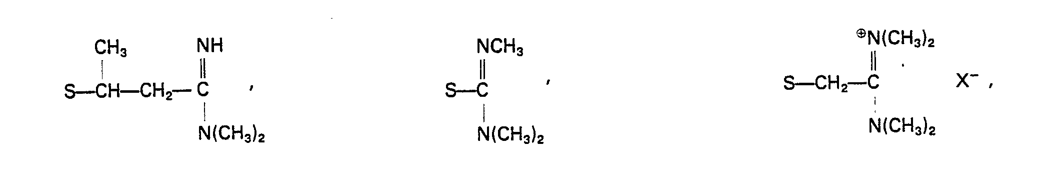

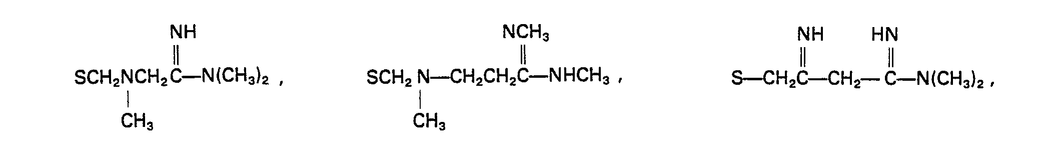

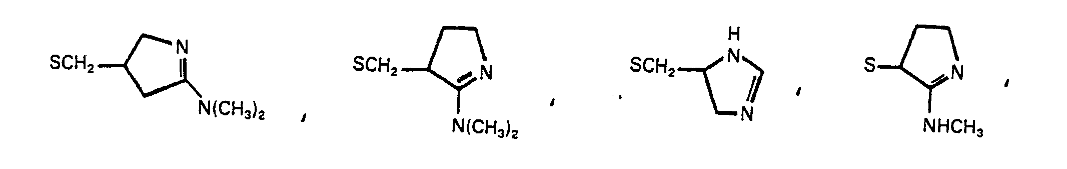

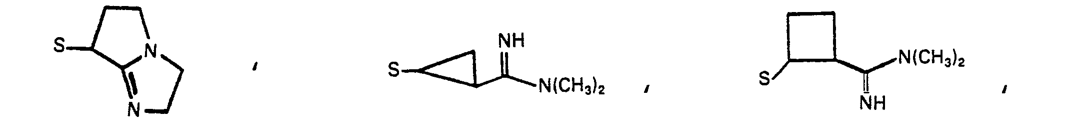

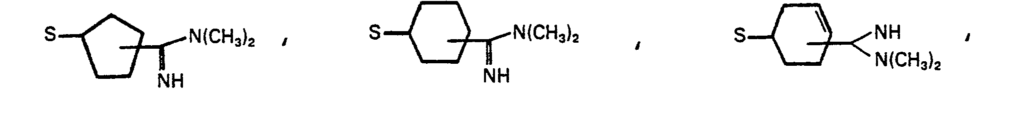

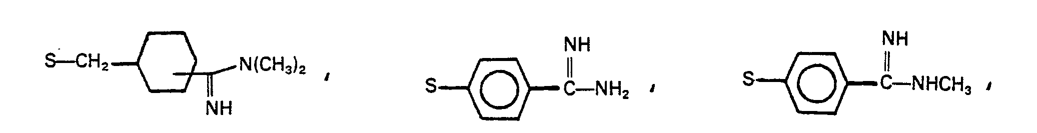

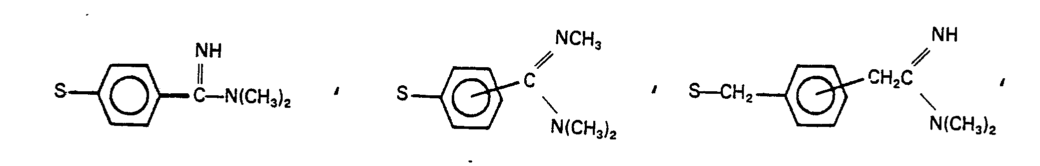

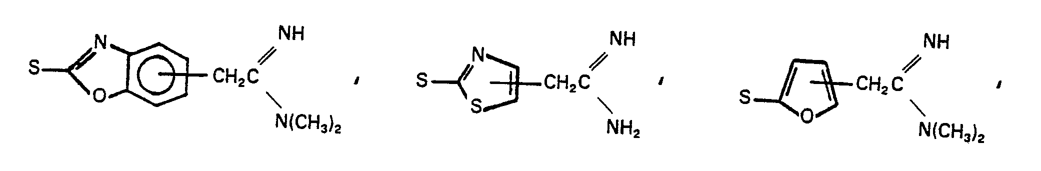

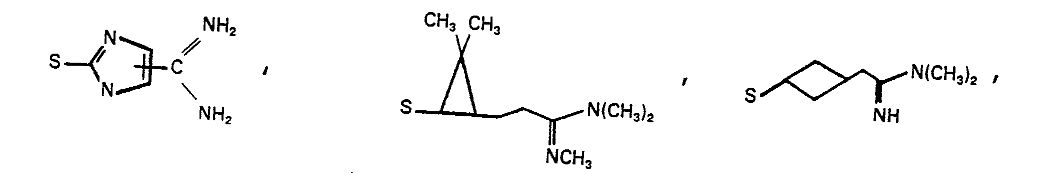

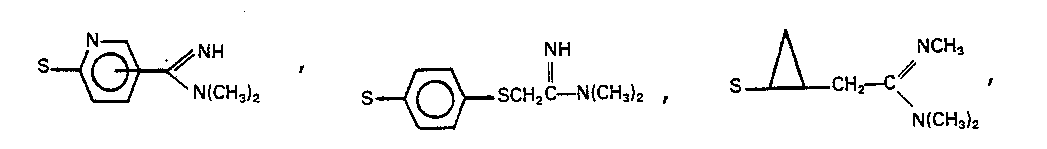

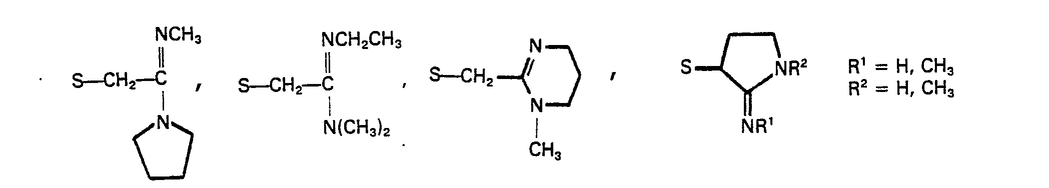

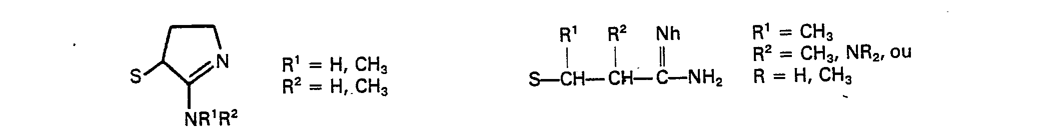

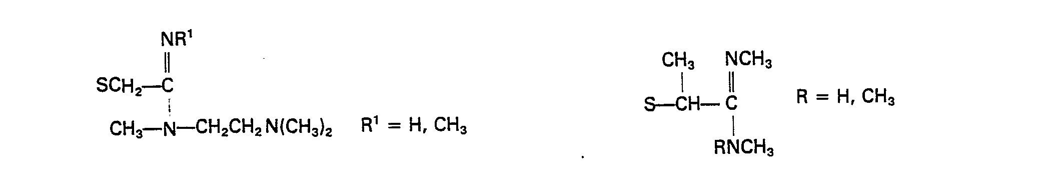

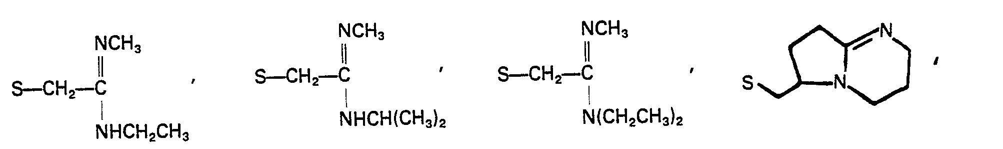

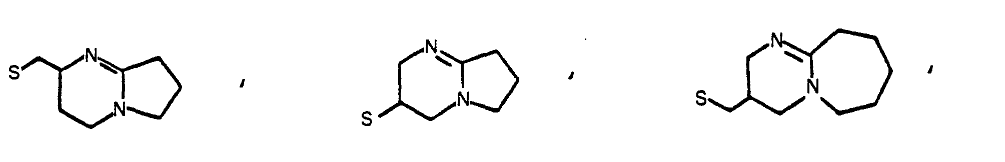

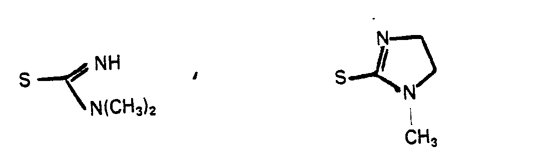

3. Procédé selon la revendication 1 ou 2, dans lequel R⁷ est choisi parmi H et CH₃.

4. Procédé selon les revendications 1 à 3, dans lequel R⁶ est choisi dans le groupe constitué par les groupes, substitués ou non substitués: alkyle, alcényle et cycloalkylalkyle dans lesquels le ou les substituants sont choisis parmi les groupes hydroxyle, alcoxyle ayant 1 à 6 atomes de carbone, amino et carboxy.

5. Procédé selon les revendications 1 à 4, dans lequel R⁶ est choisi dans le groupe constitué par les groupe's alkyle, cycloalkylalkyle, alkyle substitué par un ou plusieurs groupes hydroxyle, ou cycloalkylalkyle substitué par un ou plusieurs groupes hydroxyle.

6. Procédé selon les revendications 1 à 5, dans lequel R⁷ est un hydrogène.

7. Procédé selon les revendications 1 à 6, dans lequel R⁶ est choisi parmi: un hydrogène,

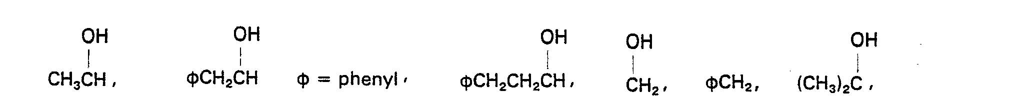

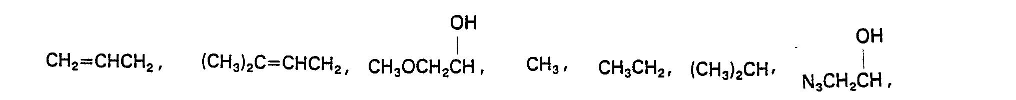

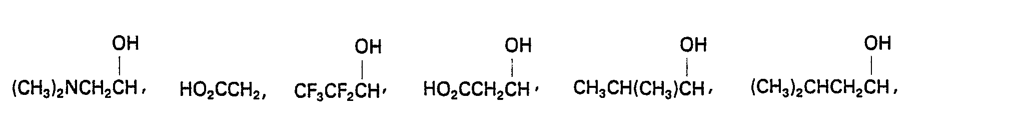

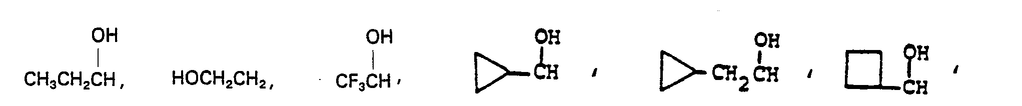

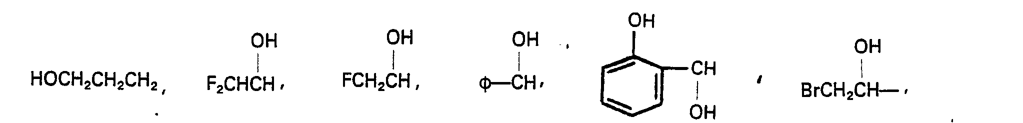

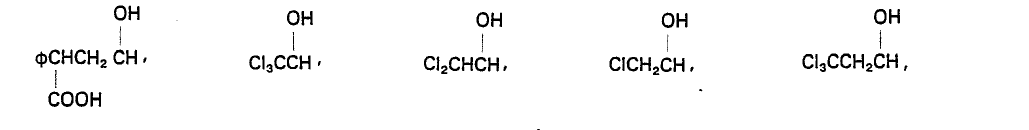

8. Procédé selon les revendications 1 à 7, dans lequel R⁶ est un hydrogène,

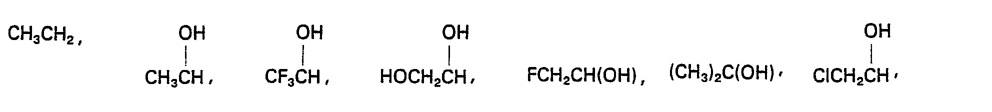

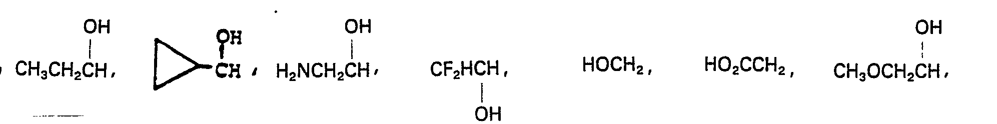

9. Procédé selon les revendications 1 à 8, dans lequel R⁶ est un hydrogène; les groupes, substitués ou non substitués: alkyle, cycloalkyle, cycloalkylalkyle, phénylalkyle; dans lequel le ou les substituants sont choisis parmi les groupes: hydroxyle, chloro, fluoro, bromo, carboxyle, uréido, amino, alcoxyle ou alkylthio.

10. Procédé selon les revendications 1 à 9, dans lequel R⁶ est choisi parmi:
